# EUROPÄISCHE PATENTANMELDUNG

(11) **EP 0 723 961 A1**
(43) Veröffentlichungstag der Anmeldung: **31.07.1996**
(21) Anmeldenummer: 95119942.1
(22) Anmeldetag: 18.12.1995
(51) Int. Cl.: C07D 239/60, C07D 401/12, A01N 43/54

(54) **Fluorierte Pyrimidine als Herbizide**

(30) Priorität: 09.01.1995 DE 19500379
(71) Anmelder: BASF AKTIENGESELLSCHAFT, 67056 Ludwigshafen (DE)
(72) Erfinder: Vogelbacher, Uwe Josef, Dr., D-67071 Ludwigshafen (DE); Rheinheimer, Joachim, Dr., D-67063 Ludwigshafen (DE); Baumann, Ernst, Dr., D-67373 Dudenhofen (DE); Kardorff, Uwe, Dr., D-68159 Mannheim (DE); Misslitz, Ulf, Dr., D-67433 Neustadt (DE); Westphalen, Karl-Otto, Dr., D-67346 Speyer (DE); Walter, Helmut, Dr., D-67283 Obrigheim (DE)

(57) **Zusammenfassung**

Fluorierte Pyrimidine der Formel I 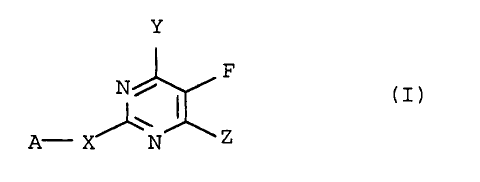
in welcher **A** für einen der folgenden Reste steht: 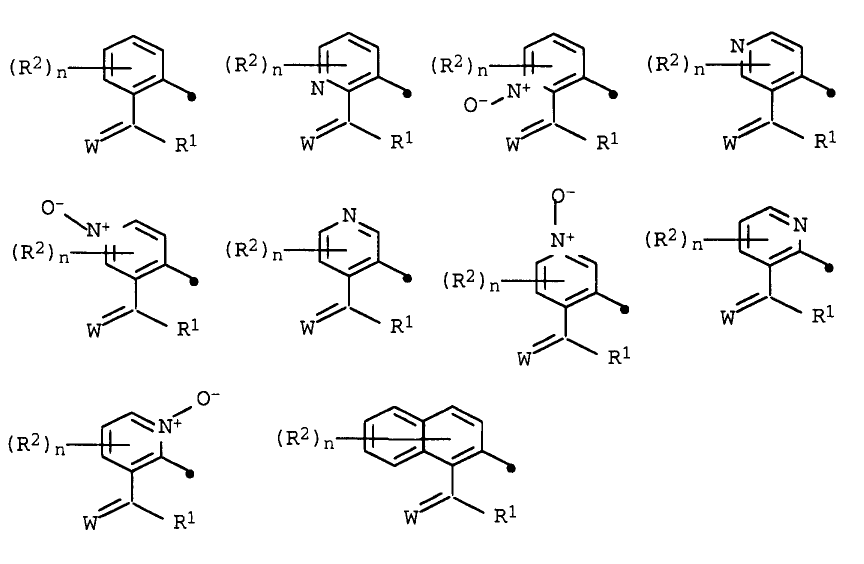
und in der die angegebenen Symbole folgende Bedeutung haben:
- W: =O, =N-R³, =N-O-R³, =N-NR⁴R⁵;
- X: -O-, -S-, und -NR⁶-;
- Y,Z: Halogen, Alkyl, Halogenalkyl, Alkoxy, Halogenalkoxy, Alkylthio, Alkylamino und/oder Dialkylamino;
- R¹, R² und n: die in der Beschreibung angegebene Bedeutung haben, sowie Verfahren und Zwischenprodukte zur Herstellung der Verbindungen I.

## Beschreibung

In den Anmeldungen EP 223406, EP 249707, EP 249708; EP 287072, EP 315889, EP 321846, EP 402751 und WO 94-13065 werden Pyrimidinderivate mit starker herbizider Wirkung beschrieben. Ihre Wirkung und ihre Selektivität sind jedoch nicht immer ausreichend. Aufgabe der vorliegenden Erfindung war es daher, Verbindungen zu finden, die diesen Nachteil überkommen. Diese Aufgabe wurde erreicht durch fluorierte Pyrimidine der Formel I 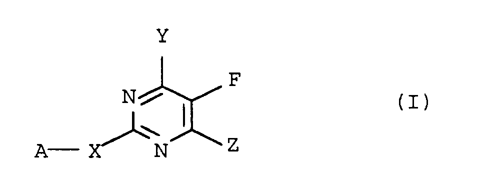
in welchen **A** für einen der folgenden Reste steht: 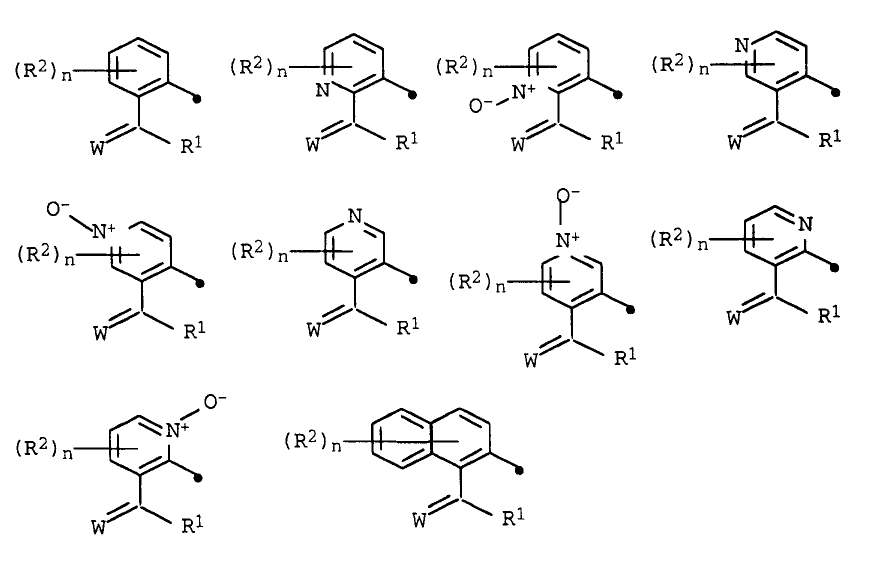
Die angegebenen Symbole haben folgende Bedeutung:
- W: =O, =N-R³, =N-O-R³, =N-NR⁴R⁵;
- X: -O-, -S-, und -NR⁶-;
- Y,Z: Halogen, C₁-C₆-Alkyl, C₁-C₄-Halogenalkyl, C₁-C₄-Alkoxy, C₁-C₄-Halogenalkoxy, C₁-C₄-Alkylthio, C₁-C₄-Alkylamino und/oder Di-C₁-C₄-alkylamino;
- R¹: Wasserstoff;
C₁-C₆-Alkyl, C₁-C₆-Alkylcarbonyl, C₁-C₆-Alkoxycarbonyl;
eine Succinyliminooxygruppe;
ein 5-gliedriger Heteroaromat, enthaltend ein bis drei Stickstoffatome, welcher ein bis vier Halogenatome und/oder ein bis zwei der folgenden Reste tragen kann: C₁-C₆-Alkyl, C₁-C₄-Halogenalkyl, C₁-C₄-Alkoxy, C₁-C₄-Halogenalkoxy und/oder C₁-C₄-Alkylthio;
ein Rest OR⁷;
ein Rest 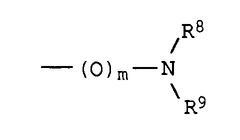
in dem R⁸ und R⁹ gleich oder unterschiedlich sein können;
oder ein Rest 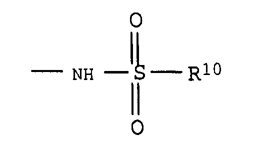
- R²: Wasserstoff;
Halogen;
Amino;
C₁-C₆-Alkyl, C₃-C₆-Alkenyl C₃-C₆-Alkinyl, C₁-C₄-Alkoxy, C₁-C₄-Alkylthio, C₁-C₄-Alkylsulfonyl, C₁-C₄-Alkylamino, Di-C₁-C₄-alkylamino, die einen der folgenden Substituenten tragen können, welche im Falle von Dialkylamino gleich oder unterschiedlich sein können: C₁-C₄-Alkoxy, C₁-C₄-Alkylthio, Di-C₁-C₄-alkylamino, Thienyl, Furyl, einen fünfgliedrigen aromatischen Heterocyclus mit ein bis vier Stickstoffatomen oder ein bis drei Stickstoffatomen sowie zusätzlich einem Schwefel- oder Sauerstoffatom im Ring, wobei die Fünfringheterocyclen ein bis drei Halogenatome und/oder einen bis drei der folgenden Reste tragen können: Nitro, Cyano, C₁-C₆-Alkyl, C₁-C₄-Alkoxy, C₁-C₄-Alkylthio, C₁-C₄-Halogenalkyl; oder Phenyl, das unsubstituiert oder durch ein bis drei Halogenatome und/oder ein bis drei Methylgruppen substituiert ist; oder Phenoxy, das unsubstituiert oder durch ein bis drei Halogenatome und/oder ein bis drei Methylgruppen substituiert ist oder einen sechsgliedrigen aromatischen Heterocyclus mit ein bis drei Stickstoffatomen im Ring, welcher ein bis drei Halogenatome und/oder einen bis drei der folgenden Reste tragen kann: Nitro, Cyano, C₁-C₆-Alkyl, C₁-C₄-Alkoxy, C₁-C₄-Alkylthio, C₁-C₄-Halogenalkyl;
Phenyl, substituiertes Phenyl;
Phenoxy, substituiertes Phenoxy;
Phenylthio, substituiertes Phenylthio, Phenylsulfonyl, substituiertes Phenylsulfonyl;
einen 5-gliedrigen Heteroaromaten mit ein bis vier Stickstoffatomen oder ein bis drei Stickstoffatomen sowie zusätzlich einem Schwefel- oder Sauerstoffatom im Ring, welcher ein bis drei Halogenatome und/oder einen bis drei der folgenden Reste tragen kann: Nitro, Cyano, C₁-C₆-Alkyl, C₁-C₄-Alkoxy, C₁-C₄-Alkylthio, C₁-C₄-Halogenalkyl, C₁-C₄-Halogenalkoxy oder Phenyl, das unsubstituiert oder durch ein bis drei Halogenatome und/oder ein bis drei Methylgruppen substituiert ist;
einen benzokondensierten 5-gliedrigen Heteroaromaten, der ein bis drei Stickstoffatome oder ein Stickstoffatom und zusätzlich ein Sauerstoff- oder Schwefelatom enthalten und einen der folgenden Reste tragen kann: Halogen, Nitro, Cyano, C₁-C₆-Alkyl, C₁-C₄-Alkoxy, C₁-C₄-Alkylthio, C₁-C₄-Halogenalkyl, C₁-C₄-Halogenalkoxy oder Phenyl, das unsubstituiert oder durch ein bis drei Halogenatome und/oder ein bis drei Methylgruppen substituiert ist;
einen Thienyl- oder Furylrest, der ein bis drei Halogenatome und/oder einen bis drei der folgenden Reste tragen kann: Nitro, Cyano, C₁-C₆-Alkyl, C₁-C₄-Alkoxy, C₁-C₄-Alkylthio, C₁-C₄-Halogenalkyl, C₁-C₄-Halogenalkoxy oder Phenyl, das unsubstituiert oder durch ein bis drei Halogenatome und/oder ein bis drei Methylgruppen substituiert ist;
einen Benzthienyl- oder Benzofurylrest, der ein bis fünf Halogenatome und/oder einen bis drei der folgenden Reste tragen kann: Nitro, Cyano, C₁-C₆-Alkyl, C₁-C₄-Alkoxy, C₁-C₄-Alkylthio, C₁-C₄-Halogenalkyl, C₁-C₄-Halogenalkoxy oder Phenyl, das unsubstituiert oder durch ein bis drei Halogenatome und/oder ein bis drei Methylgruppen substituiert ist;
einen 6-gliedrigen Heteroaromaten mit ein bis vier Stickstoffatomen im Ring, welcher ein bis drei Halogenatome und/oder einen bis drei der folgenden Reste tragen kann: Nitro, Cyano, C₁-C₆-Alkyl, C₁-C₄-Alkoxy, C₁-C₄-Alkylthio, C₁-C₄-Halogenalkyl, C₁-C₄-Halogenalkoxy oder Phenyl, das unsubstituiert oder durch ein bis drei Halogenatome und/oder ein bis drei Methylgruppen substituiert ist;
einen Naphthyl-, Chinolinyl-, Isochinolinyl- oder Chinazolinylrest welcher jeweils bis zu drei Halogenatome und/oder bis zu drei der folgenden Reste tragen kann: Nitro, Cyano, C₁-C₆-Alkyl, C₁-C₄-Alkoxy, C₁-C₄-Alkylthio, C₁-C₄-Halogenalkyl, C₁-C₄-Halogenalkoxy oder Phenyl, das unsubstituiert oder durch ein bis drei Halogenatome und/oder ein bis drei Methylgruppen substituiert ist;
einen über ein Sauerstoffatom gebundenen 5-gliedrigen Heteroaromaten mit ein bis vier Stickstoffatomen oder ein bis drei Stickstoffatomen sowie zusätzlich einem Schwefel- oder Sauerstoffatom im Ring, welcher ein bis drei Halogenatome und/oder einen bis drei der folgenden Reste tragen kann: Nitro, Cyano, C₁-C₆-Alkyl, C₁-C₄-Alkoxy, C₁-C₄-Alkylthio, C₁-C₄-Halogenalkyl, C₁-C₄-Halogenalkoxy oder Phenyl, das unsubstituiert oder durch ein bis drei Halogenatome und/oder ein bis drei Methylgruppen substituiert ist;
einen über ein Sauerstoffatom gebundenen benzokondensierten 5-gliedrigen Heteroaromaten, der ein bis drei Stickstoffatome oder ein Stickstoffatom und zusätzlich ein Sauerstoff- oder Schwefelatom enthalten und einen der folgenden Reste tragen kann: Halogen, Nitro, Cyano, C₁-C₆-Alkyl, C₁-C₄-Alkoxy, C₁-C₄-Alkylthio, C₁-C₄-Halogenalkyl, C₁-C₄-Halogenalkoxy oder Phenyl, das unsubstituiert oder durch ein bis drei Halogenatome und/oder ein bis drei Methylgruppen substituiert ist;
einen über ein Sauerstoffatom gebundenen Thienyl- oder Furylrest, der ein bis drei Halogenatome und/oder einen bis drei der folgenden Reste tragen kann: Nitro, Cyano, C₁-C₆-Alkyl, C₁-C₄-Alkoxy, C₁-C₄-Alkylthio, C₁-C₄-Halogenalkyl, C₁-C₄-Halogenalkoxy oder Phenyl, das unsubstituiert oder durch ein bis drei Halogenatome und/oder ein bis drei Methylgruppen substituiert ist;
einen über ein Sauerstoffatom gebundenen Benzthienyl- oder Benzofurylrest, der ein bis fünf Halogenatome und/oder einen bis drei der folgenden Reste tragen kann: Nitro, Cyano, C₁-C₆-Alkyl, C₁-C₄-Alkoxy, C₁-C₄-Alkylthio, C₁-C₄-Halogenalkyl, C₁-C₄-Halogenalkoxy oder Phenyl, das unsubstituiert oder durch ein bis drei Halogenatome und/oder ein bis drei Methylgruppen substituiert ist;
einen über ein Sauerstoffatom gebundenen 6-gliedrigen Heteroaromaten mit ein bis vier Stickstoffatomen im Ring, welcher ein bis drei Halogenatome und/oder einen bis drei der folgenden Reste tragen kann: Nitro, Cyano, C₁-C₆-Alkyl, C₁-C₄-Alkoxy, C₁-C₄-Alkylthio, C₁-C₄-Halogenalkyl, C₁-C₄-Halogenalkoxy oder Phenyl, das unsubstituiert oder durch ein bis drei Halogenatome und/oder ein bis drei Methylgruppen substituiert ist;
einen über ein Sauerstoffatom gebundenen Naphthyl-, Chinolinyl-, Isochinolinyl- oder Chinazolinylrest welcher jeweils bis zu drei Halogenatome und/oder bis zu drei der folgenden Reste tragen kann: Nitro, Cyano, C₁-C₆-Alkyl, C₁-C₄-Alkoxy, C₁-C₄-Alkylthio, C₁-C₄-Halogenalkyl, C₁-C₄-Halogenalkoxy oder Phenyl, das unsubstituiert oder durch ein bis drei Halogenatome und/oder ein bis drei Methylgruppen substituiert ist;
einen Rest 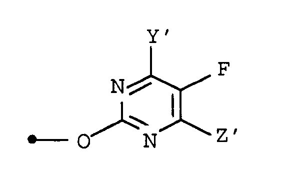 in dem die Symbole Z', X¹', X²' und X³' die oben für Z, X¹, X² und X³angegebene Bedeutung haben und gleich oder unterschiedlich zu ihnen sein können;
eine Gruppierung 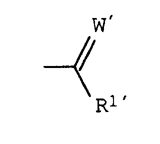 in der W' und R¹' die gleiche Bedeutung wie W bzw. R¹ haben, und gleich oder verschieden von diesen Einheiten sein können;
- R³: Wasserstoff, Phenyl, substituiertes Phenyl oder C₁-C₆-Alkyl, C₃-C₆-Alkenyl, C₃-C₆-Alkinyl, die ein bis drei der folgenden Substituenten tragen können: Halogen, C₁-C₆-Alkyl, C₁-C₄-Alkoxy, C₁-C₆-Alkylcarbonyl, C₁-C₆-Alkoxycarbonyl, Carboxy, C₁-C₄-Alkylthio, Phenyl, substituiertes Phenyl;
- R⁴: Wasserstoff, C₁-C₆-Alkyl, Phenyl;
- R⁵: Wasserstoff, Phenyl, substituiertes Phenyl oder C₁-C₆-Alkyl, das ein bis drei der folgenden Substituenten tragen kann: Halogen, C₁-C₄-Alkoxy, C₁-C₆-Alkylcarbonyl, C₁-C₆-Alkoxycarbonyl, Carboxy, C₁-C₄-Alkylthio, Phenyl, substituiertes Phenyl;
- R⁶: Wasserstoff, C₁-C₆-Alkyl, Formyl, Carbamoyl, C₁-C₆-Alkoxycarbonyl;
- R⁷: Wasserstoff, ein Alkalimetallkation, das Äquivalent eines Erdalkalimetallkations oder ein organisches Ammoniumion;
eine C₃-C₁₂-Cycloalkylgruppe, welche ein bis drei C₁-C₄-Alkylreste tragen kann;
eine C₁-C₆-Alkylgruppe welche ein bis fünf Halogenatome und/oder einen oder zwei der folgenden Reste tragen kann: C₁-C₄-Alkoxy, C₁-C₄-Alkylthio, Cyano, C₁-C₆-Alkylcarbonyl, C₁-C₆-Alkoxycarbonyl, C₃-C₁₂-Cycloalkyl, ein Rest-O-N=CR¹¹R¹², in dem R¹¹ und R¹² gleich oder verschieden sein können, Phenyl, Phenoxy, oder Phenylcarbonyl, wobei die aromatischen Reste ihrerseits ein bis fünf Halogenatome und/oder ein bis drei der folgenden Reste tragen können: C₁-C₆-Alkyl, C₁-C₄-Halogenalkyl, C₁-C₄-Alkoxy, C₁-C₄-Halogenalkoxy und/oder C₁-C₄-Alkylthio;
eine C₁-C₆-Alkylgruppe, welche ein bis fünf Halogenatome tragen kann und einen der folgenden Reste trägt: ein 5-gliedriger Heteroaromat, enthaltend ein bis drei Stickstoffatome, welcher ein bis vier Halogenatome und/oder ein bis zwei der folgenden Reste tragen kann: C₁-C₆-Alkyl, C₁-C₄-Halogenalkyl, C₁-C₄-Alkoxy, C₁-C₄-Halogenalkoxy und/oder C₁-C₄-Alkylthio;
eine Alkylgruppe, welche in der 2-Position einen der folgenden Reste trägt: C₁-C₁₂-Alkoxyimino, C₃-C₁₂-Alkenyloxyimino, C₃-C₆-Halogenalkenyloxyimino oder Benzyloxyimino;
eine C₃-C₆-Alkenyl- oder eine C₃-C₆-Alkinylgruppe, wobei diese Gruppen ihrerseits ein bis fünf Halogenatome tragen können;
unsubstituiertes oder ein- bis dreifach durch Nitro, C₁-C₆-Alkyl oder C₁-C₆-Alkoxy substituiertes oder ein- bis fünffach durch Halogen substituiertes Phenyl;
ein Rest -N=CR¹³R¹⁴, in dem R¹³ und R¹⁴ gleich oder verschieden sein können;
ein über ein Stickstoffatom gebundener 5-gliedriger aromatischer Heterocyclus mit ein bis vier Stickstoffatomen im Ring oder ein über ein Stickstoffatom gebundener benzokondensierter 5-gliedriger aromatischer Heterocyclus mit ein bis drei Stickstoffatomen im Ring, die von Halogen, C₁-C₆-Alkyl, C₁-C₄-Halogenalkyl substituiert sein können;
- R⁸,R⁹: Wasserstoff;
C₁-C₆-Alkyl, C₃-C₆-Alkenyl, C₃-C₆-Alkinyl, wobei diese Reste jeweils ein bis fünf Halogenatome und/oder ein bis zwei der folgenden Gruppen tragen können: C₁-C₄-Alkoxy, C₁-C₄-Alkylthio, Cyano, C₁-C₆-Alkylcarbonyl, C₁-C₆-Alkoxycarbonyl, Di-C₁-C₄-alkylamino, cyclo-C₃-C₁₂-Alkyl;
cyclo-C₃-C₁₂-Alkyl das ein bis drei C₁-C₄-Alkylsubstituenten tragen kann;
Phenyl oder substituiertes Phenyl;
gemeinsam eine zu einem Ring geschlossene C₃-C₁₂-Alkylenkette oder gemeinsam eine zu einem Ring geschlossene C₃-C₁₂-Alkylenkette mit einem Heteroatom, das Sauerstoff, Schwefel oder Stickstoff sein kann, die jeweils ein bis drei C₁-C₄-Alkylsubstituenten tragen können;
oder eine Gruppe 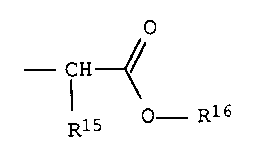
- R¹⁰: C₁-C₆-Alkyl oder Phenyl, die ein bis vier der folgenden Substituenten tragen können: Halogen, Nitro, Cyano, C₁-C₆-Alkyl;
- R¹¹,R¹²: C₁-C₆-Alkyl, welches eine C₁-C₄-Alkoxy- oder C₁-C₄-Alkylthiogruppe tragen kann, gemeinsam eine C₃-C₁₂-Alkylenkette;
- R¹³,R¹⁴: C₁-C₆-Alkyl, welches einen Phenylrest, eine C₁-C₄-Alkoxy- und/oder eine C₁-C₄-Alkylthiogruppe tragen kann, C₃-C₁₂-Cycloalkyl, Phenyl, gemeinsam eine C₃-C₁₂-Alkylenkette, welche ein bis fünf C₁-C₆-Alkylgruppen tragen kann und die durch eine C₃-C₁₂-Alkylenkette überbrückt sein kann;
- R¹⁵: Wasserstoff oder C₁-C₆-Alkyl, das durch Hydroxy-, Amino-, Hydrogensulfid-, C₁-C₄-Alkylthio-, Carboxy-, Carbamoyl-, Guanidinyl-, Phenyl-, Hydroxyphenyl-, Imidazolyl- oder Indolyl-Radikale substituiert sein kann oder zusammen mit R⁹ über eine C₃-C₁₂-Alkylenkette zu einem Ring verbunden ist;
- R¹⁶: C₁-C₆-Alkyl, C₃-C₆-Alkenyl oder C₃-C₆-Alkinyl;
- m: 0 oder 1;
- n: 0, 1, 2 oder 3;
Substituiertes Phenyl, substituiertes Phenoxy, substituiertes Phenylthio, substituiertes Phenylsulfonyl bedeutet jeweils, daß der Phenylring ein bis fünf Halogenatome und/oder einen bis drei der folgenden Reste tragen kann: Nitro, Cyano, C₁-C₆-Alkyl, C₁-C₄-Halogenalkyl, C₁-C₆-Alkoxy, C₁-C₄-Halogenalkoxy, C₁-C₄-Alkylthio, C₁-C₄-Alkylamino, Di-C₁-C₄-alkylamino, C₁-C₆-Alkylcarbonyl, C₁-C₆-Alkoxycarbonyl, Phenyl, mit ein bis drei Halogenatomen oder mit ein bis drei Methylgruppen substuiertes Phenyl, Phenoxy, mit ein bis drei Halogenatomen oder mit ein bis drei Methylgruppen substituiertes Phenoxy oder an zwei benachbarten Atomen eine Einheit -O-(CH₂)ₒ-O-, wobei o die Werte 1 oder 2 annehmen kann.

Bevorzugt sind fluorierte Pyrimidine der Formel I, in welcher A für einen der folgenden Reste steht: 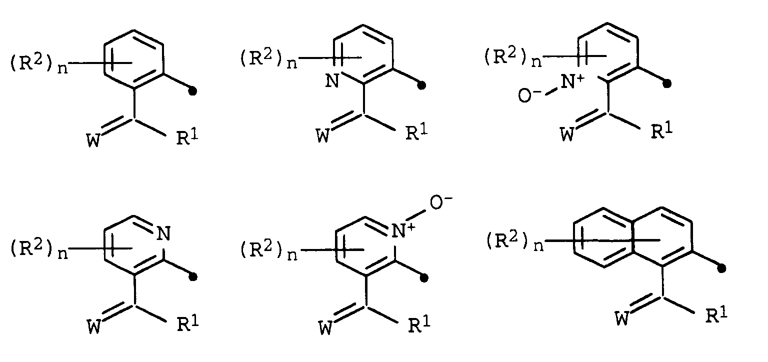
und in der die angegebenen Symbole folgende Bedeutung haben:
- W: =O;
- X: -O- und -S-;
- Y,Z: Alkyl, Halogenalkyl, Alkoxy, Halogenalkoxy, Alkylthio, Dialkylamino;
- R¹: Wasserstoff;
ein 5-gliedriger Heteroaromat, enthaltend ein bis drei Stickstoffatome,
ein Rest OR⁷;
ein Rest 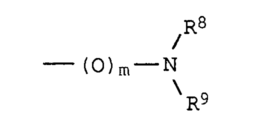 in dem R⁸ und R⁹ gleich oder unterschiedlich sein können;
- R²: Wasserstoff;
Halogen;
Alkyl, Alkenyl, Alkinyl, Alkoxy, Alkylamino, Dialkylamino, die einen der folgenden Substituenten tragen können, welche im Falle von Dialkylamino gleich oder unterschiedlich sein können: Alkoxy, Alkylthio, Dialkylamino, Thienyl, Furyl, einen fünfgliedrigen aromatischen Heterocyclus mit ein bis vier Stickstoffatomen oder ein bis drei Stickstoffatomen sowie zusätzlich einem Schwefel- oder Sauerstoffatom im Ring, wobei die Fünfringheterocyclen ein bis drei der folgenden Reste tragen können: Alkyl, Alkoxy, Alkylthio, Halogenalkyl;
Phenyl, substituiertes Phenyl;
Phenoxy, substituiertes Phenoxy;
Phenylthio, substituiertes Phenylthio, Phenylsulfonyl, substituiertes Phenylsulfonyl;
einen 5-gliedrigen Heteroaromaten aus der Gruppe Pyrrol, Pyrazol, Imidazol, 1,2,3-Triazol, 1,2,4-Triazol, Tetrazol, Oxazol, Isoxazol, Thiazol, Isothiazol, 1,3,4-Oxadiazol, Furazan, 1,3,4-Thiadiazol, 1,2,3-Thiadiazol, welcher ein bis drei Halogenatome und/oder einen bis drei der folgenden Reste tragen kann: Alkyl, Alkoxy, Alkylthio, Halogenalkyl, Halogenalkoxy;
einen Thienyl- oder Furylrest, der ein bis drei Halogenatome und/oder einen bis drei der folgenden Reste tragen kann: Alkyl, Alkoxy, Alkylthio, Halogenalkyl, Halogenalkoxy;
einen Benzthienyl- oder Benzofurylrest, der einen bis drei der folgenden Reste tragen kann: Alkyl, Alkoxy, Alkylthio, Halogenalkyl, Halogenalkoxy;
einen 6-gliedrigen Heteroaromaten aus der Gruppe Pyridin, Pyridazin, Pyrimidin, Pyrazin, 1,2,3-Triazin, 1,2,4-Triazin, 1,3,5-Triazin, welcher ein bis drei Halogenatome und/oder einen bis drei der folgenden Reste tragen kann: Alkyl, Alkoxy, Alkylthio, Halogenalkyl, Halogenalkoxy;
einen Naphthyl-, Chinolinyl-, Isochinolinyl- oder Chinazolinylrest welcher jeweils bis zu drei Halogenatome und/oder bis zu drei der folgenden Reste tragen kann: Alkyl, Alkoxy, Halogenalkyl, Halogenalkoxy;
einen über ein Sauerstoffatom gebundenen Thienyl- oder Furylrest, der ein bis drei Halogenatome und/oder einen bis drei der folgenden Reste tragen kann: Alkyl, Alkoxy, Alkylthio, Halogenalkyl, Halogenalkoxy;
einen über ein Sauerstoffatom gebundenen Benzthienyl- oder Benzofurylrest, der einen bis drei der folgenden Reste tragen kann: Alkyl, Alkoxy, Alkylthio, Halogenalkyl, Halogenalkoxy;
einen über ein Sauerstoffatom gebundenen 6-gliedrigen Heteroaromaten aus der Gruppe Pyrimidin, 1,3,5-Triazin, welcher ein bis drei Halogenatome und/oder einen bis drei der folgenden Reste tragen kann: Alkyl, Alkoxy, Alkylthio, Halogenalkyl, Halogenalkoxy;
einen über ein Sauerstoffatom gebundenen Naphthyl-, Chinolinyl-, Isochinolinyl- oder Chinazolinylrest welcher bis zu drei der folgenden Reste tragen kann: Alkyl, Alkoxy, Halogenalkyl, Halogenalkoxy;
einen Rest 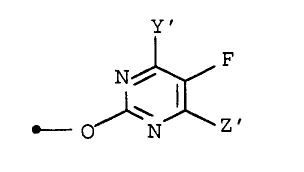 in dem Y' und Z', die oben für X und Z angegebene Bedeutung haben und gleich oder unterschiedlich zu ihnen sein können;
eine Gruppierung 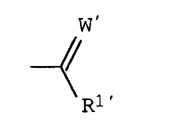 in der W' und R¹' die gleiche Bedeutung wie W bzw. R¹ haben, und gleich oder verschieden von diesen Einheiten sein können;
- R⁷: Wasserstoff, ein Alkalimetallkation, oder das Äquivalent eines Erdalkalimetallkations;
eine Alkylgruppe welche ein bis fünf Halogenatome und/oder einen oder zwei der folgenden Reste tragen kann: Alkoxy, Alkylthio, Cyano, Alkylcarbonyl, Alkoxycarbonyl, Cycloalkyl;
eine Alkylgruppe, welche ein bis fünf Halogenatome tragen kann und einen der folgenden Reste trägt: ein 5-gliedriger Heteroaromat, enthaltend ein bis drei Stickstoffatome;
eine Alkylgruppe, welche in der 2-Position einen der folgenden Reste trägt: Alkoxyimino oder Alkenyloxyimino;
eine Alkenyl- oder eine Alkinylgruppe, wobei diese Gruppen ihrerseits ein bis fünf Halogenatome tragen können;
unsubstituiertes oder ein- bis dreifach durch Nitro, Alkyl substituiertes oder ein- bis fünffach durch Halogen substituiertes Phenyl;
ein Rest -N=CR¹³R¹⁴, in dem R¹³ und R¹⁴ gleich oder verschieden sein können;
- R⁸,R⁹: Wasserstoff;
Alkyl, Alkenyl, Alkinyl;
cyclo-Alkyl;
Phenyl oder substituiertes Phenyl;
gemeinsam eine zu einem Ring geschlossene Alkylenkette oder gemeinsam eine zu einem Ring geschlossene Alkylenkette mit einem Heteroatom, das Sauerstoff, Schwefel oder Stickstoff sein kann;
- R¹³,R¹⁴: Alkyl, welches einen Phenylrest, tragen kann, Cycloalkyl, Phenyl oder gemeinsam eine Alkylenkette;
- m: 0 oder 1;
- n: 0, 1, 2 oder 3;
wobei substituiertes Phenyl, substituiertes Phenoxy, substituiertes Phenylthio, substituiertes Phenylsulfonyl bedeutet, daß der Phenylring ein bis fünf Halogenatome und/oder einen bis drei der folgenden Reste tragen kann: Nitro, Cyano, Alkyl, Halogenalkyl, Alkoxy, Halogenalkoxy, Alkylthio, Dialkylamino, Alkylcarbonyl oder an zwei benachbarten Atomen eine Einheit -O-(CH₂)ₒ-O-, wobei o die Werte 1 oder 2 annehmen kann.

Besonders bevorzugt sind fluorierte Pyrimidin der Formel I, in welcher A für einen der folgenden Reste steht: 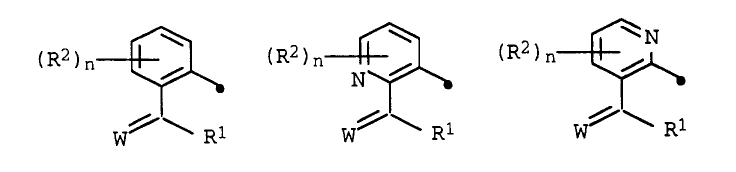
und in der die angegebenen Symbole folgende Bedeutung haben:
- W: =O;
- X: -O- und -S-;
- Y,Z: Alkoxy;
- R¹: ein Rest OR⁷;
- R²: Wasserstoff;
Halogen;
Alkyl, Alkoxy, Dialkylamino;
Phenyl, substituiertes Phenyl;
einen 5-gliedrigen Heteroaromaten aus der Gruppe Pyrazol, Thiazol, welcher einen der folgenden Reste tragen kann: Halogen, Alkoxy;
einen Thienylrest, der ein Halogenatom und/oder folgenden Rest tragen kann: Alkoxy;
einen 6-gliedrigen Heteroaromaten aus der Gruppe Pyridin, Pyrimidin, 1,3,5-Triazin, welcher einen der folgenden Reste tragen kann: Halogen, Alkoxy;
einen über ein Sauerstoffatom gebundenen Thienyl- oder Furylrest, der ein Halogenatom und/oder folgenden Rest tragen kann: Alkoxy;
einen über ein Sauerstoffatom gebundenen 6-gliedrigen Heteroaromaten aus der Gruppe Pyrimidin, 1,3,5-Triazin, welcher einen der folgenden Reste tragen kann: Halogen, Alkoxy;
einen Rest 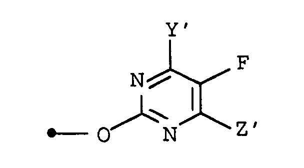 in dem Y' und Z', die oben für X und Z angegebene Bedeutung haben und gleich oder unterschiedlich zu ihnen sein können;
eine Gruppierung 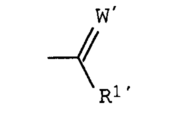 in der W' für eine Alkoxyiminogruppe und R1' für Wasserstoff oder eine Alkylgruppe stehen;
- R⁷: Wasserstoff oder ein Alkalimetallkation;
eine Alkylgruppe, welche einen oder zwei der folgenden Reste tragen kann: Alkoxy, Alkylthio;
eine Alkenyl- oder eine Alkinylgruppe;
unsubstituiertes oder einfach durch Nitro, oder einfach durch Halogen substituiertes Phenyl;
ein Rest -N=CR¹³R¹⁴, in dem R¹³ und R¹⁴ gleich oder verschieden sein können;
- n: 1;
wobei substituiertes Phenyl, substituiertes Phenoxy, substituiertes Phenylthio, substituiertes Phenylsulfonyl bedeutet, daß der Phenylring ein bis zwei der folgenden Reste tragen kann: Alkoxy, Alkylthio, an zwei benachbarten Atomen eine Einheit -O-(CH₂)ₒ-O-, wobei o die Werte 1 oder 2 annehmen kann.

Die in der Beschreibung genannten Substituenten haben bevorzugt folgende Bedeutung:
C₁-C₆-Alkyl: Methyl, Ethyl, 1-Propyl, 2-Propyl, 1-Butyl, 2-Butyl, 2-Methyl-propyl, 1,1-Dimethyl-ethyl, 1-Pentyl, 2-Pentyl, 3-Pentyl, 2-Methyl-butyl 3-Methyl-butyl, 2-Methyl-2-butyl, 3-Methyl-2-butyl, 1,1-Dimethyl-propyl, 1,2-Dimethyl-propyl, 2,2-Dimethyl-propyl, 1-Hexyl, 2-Hexyl, 3-Hexyl, 2-Methyl-pentyl, 3-Methyl-pentyl, 4-Methyl-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-butyl, 2,3-Dimethyl-butyl, 3,3-Dimethyl-butyl, 3,3-Dimethyl-2-butyl, 2-Ethyl-butyl, 1,1,2-Trimethyl-propyl, 1,2,2-Trimethyl-propyl, insbesondere Methyl, Ethyl, Propyl, 2-Propyl, Butyl, 2-Butyl, 1,1-Dimethylethyl, Pentyl, 2,2-Dimethyl-propyl, Hexyl;
C₁-C₄-Halogenalkyl: Chlormethyl, Difluormethyl, Dichlormethyl, Trifluormethyl, Trichlormethyl, Chlordifluormethyl, 1-Fluorethyl, 2-Fluorethyl, 2,2-Difluorethyl, 1,1,2,2-Tetrafluorethyl, 2,2,2-Trifluorethyl, 2-Chlor-1,1,2-trifluorethyl und Pentafluorethyl, Decafluorbutyl, 1,1-Bis-trifluormethyl-2,2,2-trifluorethyl, bevorzugt Difluormethyl, Trifluormethyl, Trichlormethyl und Chlordifluormethyl;
C₃-C₆-Alkenyl: Propenyl, 1-Butenyl, 2-Butenyl, 2-Methyl-propenyl, Pentenyl, 2-Pentenyl, 2-Methyl-butenyl, 3-Methyl-butenyl, 2-Methyl-2-butenyl, Hexenyl, 2-Hexenyl, 3-Hexenyl, 2-Methyl-pentenyl, 3-Methyl-pentenyl, 4-Methyl-pentenyl, 2-Methyl-2-pentenyl, 3-Methyl-2-pentenyl, 4-Methyl-2-pentenyl, 2,3-Dimethyl-butenyl, 2-Ethyl-butenyl, 3,3-Dimethyl-butenyl, 2,3-Dimethyl-2-butenyl;
C₃-C₆-Alkinyl: Propinyl, Butinyl, 2-Butinyl, Pentinyl, 2-Pentinyl, 3-Methylbutinyl, Hexinyl, 2-Hexinyl,3-Hexinyl, 3-Methyl-pentinyl, 4-Methyl-pentinyl, 4-Methyl-2-pentinyl;
C₃-C₁₂-Cycloalkyl: Cyclopropyl, Cyclobutyl, Cyclopentyl, Cyclohexyl, Cycloheptyl, Cyclooctyl, Cyclononyl, Cyclodecyl, Cycloundecyl und Cyclododecyl, besonders bevorzugt Cyclopropyl, Cyclopentyl und Cyclohexyl;
C₁-C₄-Alkoxy: Methoxy, Ethoxy, Propoxy, 1-Methylethoxy, Butoxy, 2-Butoxy, 1-Methylpropoxy, 2-Methylpropoxy, 1,1-Dimethylethoxy, insbesondere Methoxy, Ethoxy, 1-Methylethoxy;
C₁-C₆-Alkoxy: C₁-C₄-Alkoxy sowie Pentoxy, 2-Pentoxy, 3-Pentoxy, 1-Methylbutoxy, 2-Methylbutoxy, 3-Methylbutoxy, 2-Methyl-2-butoxy, 3-Methyl-2-butoxy, 1,1-Dimethyl-propoxy, 1,2-Dimethyl-propoxy, 2,2-Dimethyl-propoxy, 1-Hexoxy, 2-Hexoxy, 3-Hexoxy, 2-Methyl-pentoxy, 3-Methyl-pentoxy, 4-Methyl-pentoxy, 2-Methyl-2-pentoxy, 3-Methyl-2-pentoxy, 4-Methyl-2-pentoxy, 2-Methyl-3-pentoxy, 3-Methyl-3-pentoxy, 2,2-Dimethyl-butoxy, 2,3-Dimethyl-butoxy, 3,3-Dimethyl-butoxy, 2,3-Dimethyl-2-butoxy, 3,3-Dimethyl-2-butoxy, insbesondere Methoxy, Ethoxy, 1-Methylethoxy;
C₁-C₄-Halogenalkoxy: Difluormethoxy, Trifluormethoxy, Chlordifluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, 1,1,2,3,3,3-Hexafluor-propoxy, Heptafluor-propoxy, Decafluorbutoxy, 1,1-Bis-trifluormethyl-2,2,2-trifluorethoxy, bevorzugt Difluormethoxy, Trifluormethoxy und Chlordifluormethoxy;
C₁-C₄-Alkylthio: Methylthio, Ethylthio, Propylthio, 1-Methylethylthio, Butylthio, 2-Butoxy, 1-Methylpropylthio, 2-Methylpropylthio, 1,1-Dimethylethylthio, insbesondere Methylthio, Ethylthio, 1-Methylethylthio;
C₁-C₄-Alkylamino: Methylamino, Ethylamino, Propylamino, 1-Methylethylamino, Butylamino, 2-Butylamino, 1-Methylpropylamino, 2-Methylpropylamino, 1,1-Dimethylethylamino, insbesondere Methylamino, Ethylamino, 1-Methylethylamino;
Di-C₁-C₄-Alkylamino: Dimethylamino, N-Methyl-N-ethylamino, Diethylamino, N-Methyl-N-propylamino, N-Ethyl-N-propylamino, Dipropylamino, Diisopropylamino, N-Isopropyl-N-methylamino, N-Ethyl-N-isopropylamino, N-Isopropyl-N-propylamino, Dibutylamino, Di-2-methylpropylamino, Di-1-methylpropylamino, N-Butyl-N-methylamino sowie Isomere, N-Butyl-N-ethylamino sowie Isomere, N-Butyl-N-propylamino sowie Isomere;
C₁-C₆-Alkylcarbonyl: Acetyl, Propionyl, 1-Propylcarbonyl, 2-Propylcarbonyl, 1-Butylcarbonyl, 2-Butylcarbonyl, 2-Methylpropylcarbonyl, 1,1-Dimethyl-ethylcarbonyl, 1-Pentylcarbonyl, 2-Pentylcarbonyl, 3-Pentylcarbonyl, 2-Methyl-butylcarbonyl, 3-Methyl-butylcarbonyl, 2-Methyl-2-butylcarbonyl, 3-Methyl-2-butylcarbonyl, 1,1-Dimethyl-propylcarbonyl, 1,2-Dimethyl-propylcarbonyl, 2,2-Dimethyl-propylcarbonyl, 1-Hexylcarbonyl, 2-Hexylcarbonyl, 3-Hexylcarbonyl, 2-Methyl-pentylcarbonyl, 3-Methyl-pentylcarbonyl, 4-Methyl-pentylcarbonyl, 2-Methyl-2-pentylcarbonyl, 3-Methyl-2-pentylcarbonyl, 4-Methyl-2-pentylcarbonyl, 2-Methyl-3-pentylcarbonyl, 3-Methyl-3-pentylcarbonyl, 2,2-Dimethyl-butylcarbonyl, 2,3-Dimethyl-butylcarbonyl, 3,3-Dimethyl-butylcarbonyl, 3,3-Dimethyl-2-butylcarbonyl, 2-Ethyl-butylcarbonyl, 1,1,2-Trimethyl-propylcarbonyl, 1,2,2-Trimethyl-propylcarbonyl, insbesondere Acetyl, Propionyl, Propylcarbonyl, 2-Propylcarbonyl, Butylcarbonyl, 2-Butylcarbonyl, 1,1-Dimethyl-propionyl, Pentylcarbonyl, 2,2-Dimethyl-propylcarbonyl, Hexylcarbonyl;
C₁-C₆-Alkoxycarbonyl: Acetoxycarbonyl, Propoxycarbonyl, 1-Propoxycarbonyl, 2-Propoxycarbonyl, 1-Butoxycarbonyl, 2-Butoxycarbonyl, 2-Methyl-propoxycarbonyl, 1,1-Dimethyl-ethoxycarbonyl, 1-Pentoxycarbonyl, 2-Pentoxycarbonyl, 3-Pentoxycarbonyl, 2-Methyl-butoxycarbonyl, 3-Methyl-butoxycarbonyl, 2-Methyl-2-butoxycarbonyl, 3-Methyl-2-butoxycarbonyl, 1,1-Dimethyl-propoxycarbonyl, 1,2-Dimethyl-propoxycarbonyl, 2,2-Dimethyl-propoxycarbonyl, 1-Hexoxycarbonyl, 2-Hexoxycarbonyl, 3-Hexoxycarbonyl, 2-Methyl-pentoxycarbonyl, 3-Methyl-pentoxycarbonyl, 4-Methyl-pentoxycarbonyl, 2-Methyl-2-pentoxycarbonyl, 3-Methyl-2-pentoxycarbonyl, 4-Methyl-2-pentoxycarbonyl, 2-Methyl-3-pentoxycarbonyl, 3-Methyl-3-pentoxycarbonyl, 2,2-Dimethyl-butoxycarbonyl, 2,3-Dimethyl-butoxycarbonyl, 3,3-Dimethyl-butoxycarbonyl, 3,3-Dimethyl-2-butoxycarbonyl, 2-Ethyl-butoxycarbonyl, 1,1,2-Trimethyl-propoxycarbonyl, 1,2,2-Trimethyl-propoxycarbonyl, insbesondere Acetoxycarbonyl, Propoxycarbononyl, 2-Propoxycarbonyl, Butoxycarbonyl, 2-Butoxycarbonyl, 1,1-Dimethyl-propionyl, Pentoxycarbonyl, 2,2-Dimethyl-propoxycarbonyl, Hexoxycarbonyl;
C₁-C₁₂-Alkoxyimino: Methoxyimino, Ethoxyimino, Propoxyimino, 2-Propoxyimino, Butoxyimino, 1,1-Dimethylethoxyimino, Pentoxyimino, Hexoxyimino, Heptyloxyimino, Octyloxyimino, Nonyloxyimino, Decyloxyimino, Undecyloxyimino, Dodecyloxyimino, insbesondere Methoxyimino und Ethoxyimino;
C₃-C₁₂-Alkenyloxyimino: Propenyloxyimino, 2-Propenyloxyimino, Butenyloxyimino, Pentenyloxyimino, Mexenyloxyimino, Heptenyloxylmino, Octenyloxyimino, Nonenyloxyimino, Decenyloxyimino, Undecenyloxyimino, Dodecenyloxyimino, insbesondere 2-Propenyloxyimino;
C₃-C₆-Halogenalkenyloxyimino: 2,3,3-Trifluor-2-propenyloxyimino, 2,3,3-Trichlor-2-propenyloxyimino, Pentafluor-2-propenyloxyimino, Pentachlor-2-propenyloxyimino;
C₃-C₁₂-Alkylenkette: Propylen, Butylen, Pentylen, Hexylen, Heptylen, Octylen, Nonylen, Decylen, Undecylen, Dodecylen, insbesondere Butylen, Pentylen;
Als 5-gliedrige Heteroaromaten seien vor allem folgende Heterocyclen genannt: 2-Thienyl, 3-Thienyl, 2-Furyl, 3-Furyl, 1-Pyrrolyl, 2-Pyrrolyl, 3-Pyrrolyl, 1-Pyrazolyl, 3-Pyrazolyl, 4-Pyrazolyl, 5-Pyrazolyl, 1-Imidazolyl, 2-imidazolyl, 4-Imidazolyl, 5-Imidazolyl, 2-Oxazolyl, 4-Oxazolyl, 5-Oxazolyl, 2-Thiazolyl, 4-Thiazolyl, 5-Thiazolyl, 3-Isoxazolyl, 4-Isoxazolyl, 5-Isoxazolyl, 3-Isothiazolyl, 4-Isothiazolyl, 5-Isothiazolyl, 1,2,3-Triazol-1-yl, 1,2,3-Triazol-4-yl, 1,2,3-Triazol-5-yl, (1H)1,2,4-Triazol-1-yl, (1H)1,2,4-Triazol-3-yl, (1H)1,2,4-Triazol-5-yl, (4H)1,2,4-Triazol-2(5)-yl, (4H)1,2,4-Triazol-4-yl, 1-Tetrazolyl, 5-Tetrazolyl, 2-Oxazolyl, 4-Oxazolyl, 5-Oxazolyl, 3-Isoxazolyl, 4-Isoxazolyl, 5-Isoxazolyl, 2-Thiazolyl, 4-Thiazolyl, 5-Thiazolyl, 3-Isothiazolyl, 4-Isothiazolyl, 5-Isothiazolyl, 1,2,3-Oxadiazol-4-yl, 1,2,3-Oxadiazol-5-yl, 1,2,4-Oxadiazol-3-yl, 1,2,4-Oxadiazol-5-yl, 3(4)-Furazanyl, 1,3,4-Oxadiazol-2(5)-yl, 1,2,3-Thiadiazol-4-yl, 1,2,3-Thiadiazol-5-yl, 1,2,5-Thiadiazol-3-yl, 1,3,4-Thiadiazol-2-yl, 1,2,4-Thiadiazol-3-yl, 1,2,4-Thiadiazol-5-yl.

Als 6-gliedrige Heteroaromaten seien vor allem folgende Heterocyclen genannt: 2-Pyridyl, 3-Pyridyl, 4-Pyridyl, 2-Pyrimidinyl, 4(6)-Pyrimidinyl, 5-Pyrimidinyl, 3(6)-Pyrazinyl, 4(5)-Pyrazinyl, 1,3,5-Triazin-2-yl, 1,2,4-Triazin-3-yl, 1,2,4-Triazin-5-yl, 1,2,4-Triazin-6-yl, 1,2,4,5-Tetrazin-3(6)-yl.

Benzokondensierte 5- oder 6-Ring-Aromaten sind vor allem: Benzofuranyl, Benzothienyl, Indolyl, Benzoxazolyl, Benzisoxazolyl, Benzthiazolyl, Benzisothiazolyl, Benzpyrazolyl, Indazolyl, 1,2,3-Benzothiadiazolyl, 2,1,3-Benzothiadiazolyl, Benzotriazolyl, Benzofuroxanyl, Chinolinyl, Isochinolinyl, Cinnolinyl, Chinazolinyl, Chinoxalinyl, Phthalazinyl oder Bananazinyl.

Die erfindungsgemäßen Verbindungen der Formel I erhält man dadurch, daß man ein Derivat der Formel

A-XH

in welcher die Symbole A und X die oben angegebene Bedeutung haben, in Gegenwart einer Base mit einem Pyrimidin der Formel II 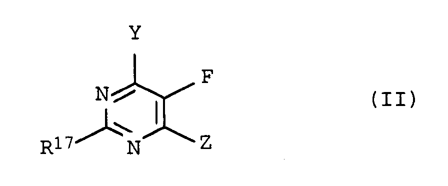
umsetzt, in welcher die Symbole Y und Z die oben angegebene Bedeutung haben und in der R¹⁷ für eine nucleofuge Abgangsgruppe wie Halogen, bevorzugt Chlor und Brom, Alkylsulfonyl, bevorzugt Methylsulfonyl, Phenylsulfonyl oder substituiertes Phenylsulfonyl, bevorzugt 4-Methylphenylsulfonyl, oder eine andere äquivalente Abgangsgruppe steht. Als Base können Alkali- oder Erdalkalimetallhydride wie NaH oder CaH₂, Alkalimetallhydroxyde wie NaOH oder KOH, Alkalimetallalkoholate wie Kalium-tert.-butylat, Alkalimetallcarbonate wie Natriumcarbonat oder Kaliumcarbonat, Alkalimetallamide wie Natriumamid, Lithiumdiisopropylamid oder Lithium-tetramethylpiperidid oder tertiäre Amine Verwendung finden. Bei Einsatz einer anorganischen Base kann man einen Phasentransferkatalysator zusetzen, wenn dies den Umsatz fördert. Als Lösungsmittel dienen vor allem protische oder aprotische polare Lösungsmittel wie beispielsweise niedere Alkohole, tetraalkylsubstituierte cyclische oder acyclische Harnstoffe, Dimethylformamid oder Dimethylsulfoxid.

Verbindungen der Formel I können auch dadurch hergestellt werden, daß man von den entsprechenden Carbonsäuren, d.h. Verbindungen der Formel I, in denen W Sauerstoff und R¹ Hydroxyl bedeutet, ausgeht und diese zunächst auf übliche Weise in eine aktivierte Form wie ein Halogenid oder Imidazolid überführt und dieses dann mit einem entsprechenden Nucleophil umsetzt. Diese beiden Schritte lassen sich beispielsweise auch dadurch vereinfachen, daß man die Carbonsäure in Gegenwart eines wasserabspaltenden Mittels wie eines Carbodiimids auf das Nucleophil einwirken läßt.

Verbindungen der Formel II lassen sich ausgehend von 2-Fluormalonsäureestern, 2-Fluorcyanessigestern, 2-Fluormalodinitril oder 1,3-Dicarbonylverbindungen der Formeln III und IV 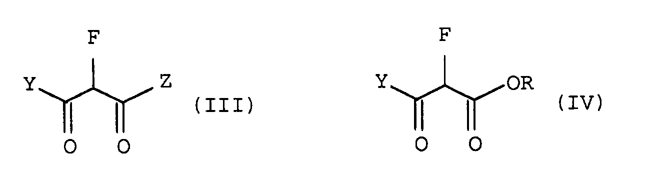
nach literaturbekannten Methoden herstellen (z.B. A.R. Katritzky und C.W. Rees, Comprehensive Heterocyclic Chemistry, Pergamon Press 1984; EP 582288).

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

Unter Berücksichtigung der Vielseitigkeit der Applikationsmethode können die Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spp. altissima, Beta vulagris spp. rapa, Brassica napus var. napus, Brassica napus var. napobrassica, Brassica rapa var. silvestris, Camellia sinensis, Carthamus tinctorius, Carya illinolnensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N.rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylestre, Ricinus communis, Saccharum officinarum, Secale cereale, Solanum tuberosum, Sorghurn bicolor (s. vulgare), Theobroma cacao, Trifolium pratense, Triticum aestivum, Triticum durum, Vicia faba, Vitis vinifera, Zea mays,
Darüber hinaus können die Verbindungen I auch in Kulturen, die durch Züchtung einschließlich gentechnischer Methoden gegen die Wirkung von Herbiziden tolerant sind, verwandt werden.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Methanol, Ethanol, Propanol, Butanol, Cyclohexanol, Cyclohexanon oder stark polare Lösungsmittel, wie N-Methylpyrrolidon oder Wasser in Betracht.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Die Formulierungen enthalten im allgemeinen 0,001 bis 98 Gew. %, vorzugsweise 0,01 bis 95 Gew. %, Wirkstoff. Die Wirkstoffe werden dabei in einer Reinheit von 90% bis 100%, vorzugsweise 95% bis 100% (nach NMR-Sektrum) eingesetzt.

Die erfindungsgemäßen Verbindungen I können beispielsweise wie folgt formuliert werden:
I 20 Gewichtsteile der Verbindung Nr. I.001 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew. % des Wirkstoffs enthält.
II 20 Gewichtsteile der Verbindung Nr. I.124 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew. % des Wirkstoffs enthält.
III 20 Gewichtsteile des Wirkstoffs Nr. I.059 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew. % des Wirkstoffs enthält.
IV 20 Gewichtsteile des Wirkstoffs Nr. I.001 werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew. % des Wirkstoffs enthält.
V 3 Gewichtsteile des Wirkstoffs Nr. I.124 werden mit 97 Gewichtsteilen feinteiligem Kaolin vermischt. Man erhält auf diese Weise ein Stäubemittel, das 3 Gew. % des Wirkstoffs enthält.
VI 20 Gewichtsteile des Wirkstoffs Nr. I.059 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondesates und 68 Gewichtsteilen eines paraffinischen Mineralöls innig vermischt. Man erhält eine stabile ölige Dispersion.
VII 1 Gewichtsteil der Verbindung I.001 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII 1 Gewichtsteil der Verbindung I.124 wird in einer Mischung gelöst, die aus 80 Gewichtsteilen Cyclohexanon und 20 Gewichtsteilen Emulphor EL besteht. Man erhält ein stabiles Emulsionskonzentrat.

Zur Verbreiterung des Wirkungsspektrums und zur Erzielung synergistischer Effekte können die fluorierten Pyrmidine I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamte, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Verbindungen der Formel I können weiterhin die verschiedenen Entwicklungsstadien einer Pflanze beeinflussen und deshalb als Wachstumsregulatoren eingesetzt werden. Die Wirkungsvielfalt der Pflanzenwachstumsregulatoren ist vor allem abhängig
a) von der Pflanzenart und -sorte,
b) von dem Zeitpunkt der Applikation, bezogen auf das Entwicklungsstadium der Pflanze, und von der Jahreszeit,
c) von dem Applikationsort und -verfahren (z.B. Samenbeize, Bodenbehandlung, Blattapplikation oder Stamminjektion bei Bäumen),
d) von klimatischen Faktoren (z.B. Temperatur, Niederschlagsmenge, außerdem auch Tageslänge und Lichtintensität),
e) von der Bodenbeschaffenheit (einschließlich Düngung),
f) von der Formulierung bzw. Anwendungsform des Wirkstoffs und
g) von den angewendeten Konzentrationen der aktiven Substanz.

Aus der Reihe der verschiedenartigen Anwendungsmöglichkeiten der erfindungsgemäßen Pflanzenwachstumsregulatoren der Formel I im Pflanzenanbau, in der Landwirtschaft und im Gartenbau, werden einige nachstehend erwähnt:
A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs aus; außerdem ist eine dunklere Blattfärbung zu beobachten.
   Als vorteilhaft für die Praxis erweist sich eine verminderte Intensität des Wachstums von Gräsern an Straßenrändern, Hecken, Kanalböschungen und auf Rasenflächen wie Park-Sport- und Obstanlagen, Zierrasen und Flugplätzen, so daß der Arbeits- und kostenaufwendige Rasenschnitt reduziert werden kann.
   Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.
   Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.
   Bei Obst- und anderen Bäumen lassen sich mit den Wachstumregulatoren Schnittkosten einsparen. Außerdem kann die Alternanz von Obstbäumen durch Wachstumsregulatoren gebrochen werden.
   Durch Anwendung von Wachstumsregulatoren kann auch die seitliche Verzweigung der Pflanzen vermehrt oder gehemmt werden. Daran besteht Interesse, wenn z. B. bei Tabakpflanzen die Ausbildung von Seitentrieben (Geiztrieben) zugunsten des Blattwachstums gehemmt werden soll.
   Mit Wachstumsregulatoren laßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generative Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.
B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchte zu erhöhen, den Proteingehalt in Getreide oder Soja zu steigern oder Gummibäume zum vermehrten Latexfluß zu stimulieren.
   Dabei können die Verbindungen der Formel I Ertragssteigerungen durch Eingriffe in den pflanzlichen Stoffwechsel bzw. durch Förderung oder Hemmung des vegetativen und/oder des generativen Wachstums verursachen.
C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.
   Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wie beispielsweise Baumwolle wesentlich.
D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.
   - die Öffnungsweite der Stomata reduziert wird
   - eine dickere Epidermis und Cuticula ausgebildet werden,
   - die Durchwurzelung des Bodens verbessert wird und
   - das Mikroklima im Pflanzenbestand durch einen kompakteren Wuchs günstig beeinflußt wird.

   Besonders gut eignen sich die Verbindungen I zur Halmverkürzung von Kulturpflanzen wie Gerste, Raps und Weizen.
   Die erfindungsgemäß zu verwendenden Wachstumsregulatoren der Formel I können den Kulturpflanzen sowohl vom Samen her (als Saatgutbeizmittel) als auch über den Boden, d.h. durch die Wurzel sowie - besonders bevorzugt - durch Spritzung über das Blatt, zugeführt werden. Die Herstellung der Mittel erfolgt dabei analog zu der von Herbiziden (s.o.).
   Die Aufwandmenge an Wirkstoff ist infolge der hohen Pflanzenverträglichkeit nicht kritisch. Die optimale Aufwandmenge variiert je nach Bekämpfungsziel, Jahreszeit, Zielpflanzen und Wachstumsstadien.

Die Aufwandmengen an Wirkstoff betragen je nach Bekämpfungsziel, Jahreszeit, Zielpflanzen und Wachstumsstadium 0.001 bis 3.0, vorzugsweise 0.01 bis 2.0 kg/ha aktive Substanz (a. S.)

### Anwendungsbeispiele

Die herbizide und wachstumsregulatorische Wirkung der fluorierten Pyrimidine der Formel I ließ sich durch Gewächshausversuche zeigen:
Als Kulturgefäße dienten Plastikblumentöpfe mit lehmigem Sand mit etwa 3,0% Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung werden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen werden dafür entweder direkt gesät und in den gleichen Gefäßen aufgezogen oder sie werden erst als Keimpflanzen getrennt angezogen und einige Tage vor der Behandlung in die Versuchsgefäße verpflanzt. Die Aufwandmenge für die Nachauflaufbehandlung betragt 0.0625 bzw. 0.0313 kg/ha a. S.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Alopecurus myosuroides | Ackerfuchsschwanz | blackgrass |
| Sinapis alba | Weißer Senf | white mustard |
| Solanum nigrum | Schwarzer Nachtschatten | black nightshade |

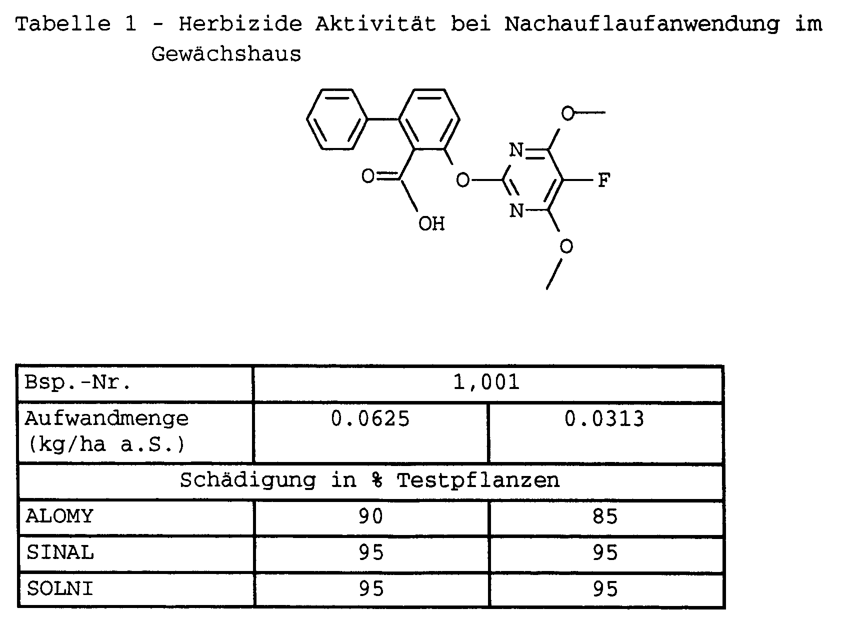

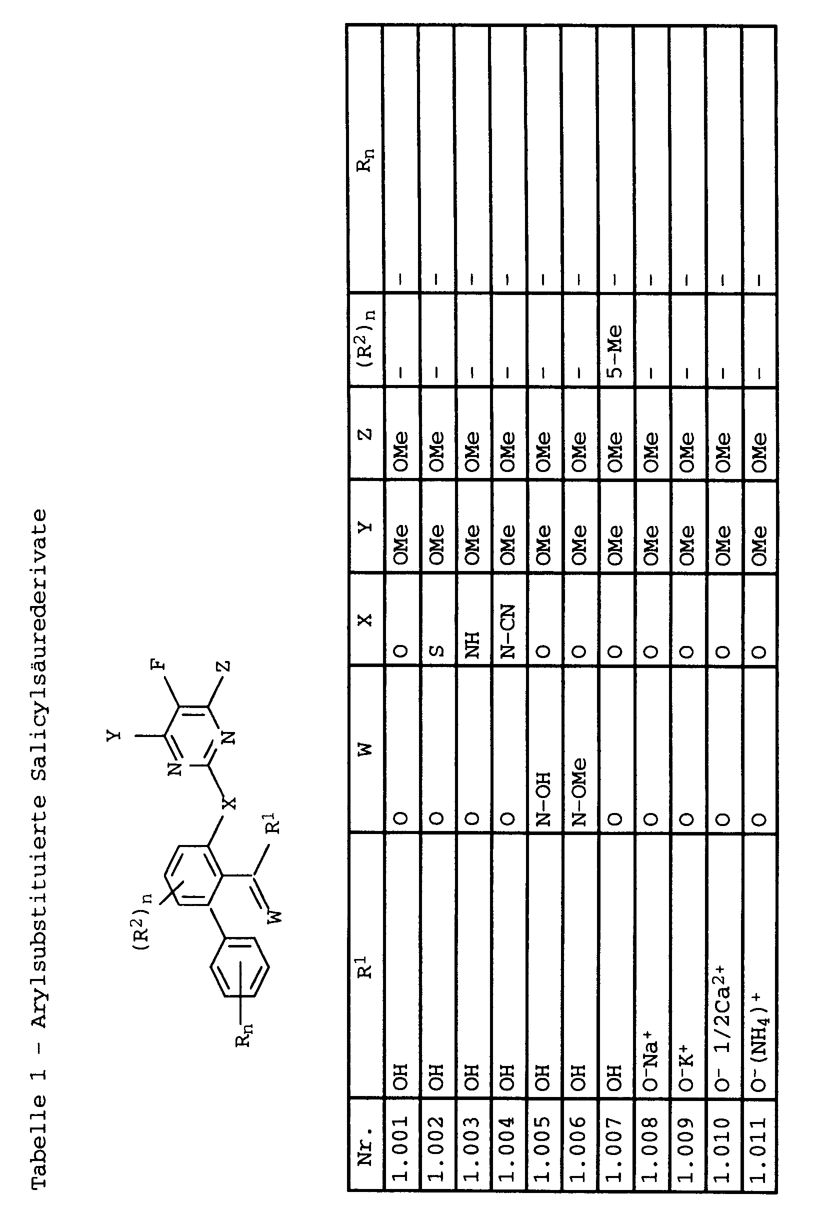

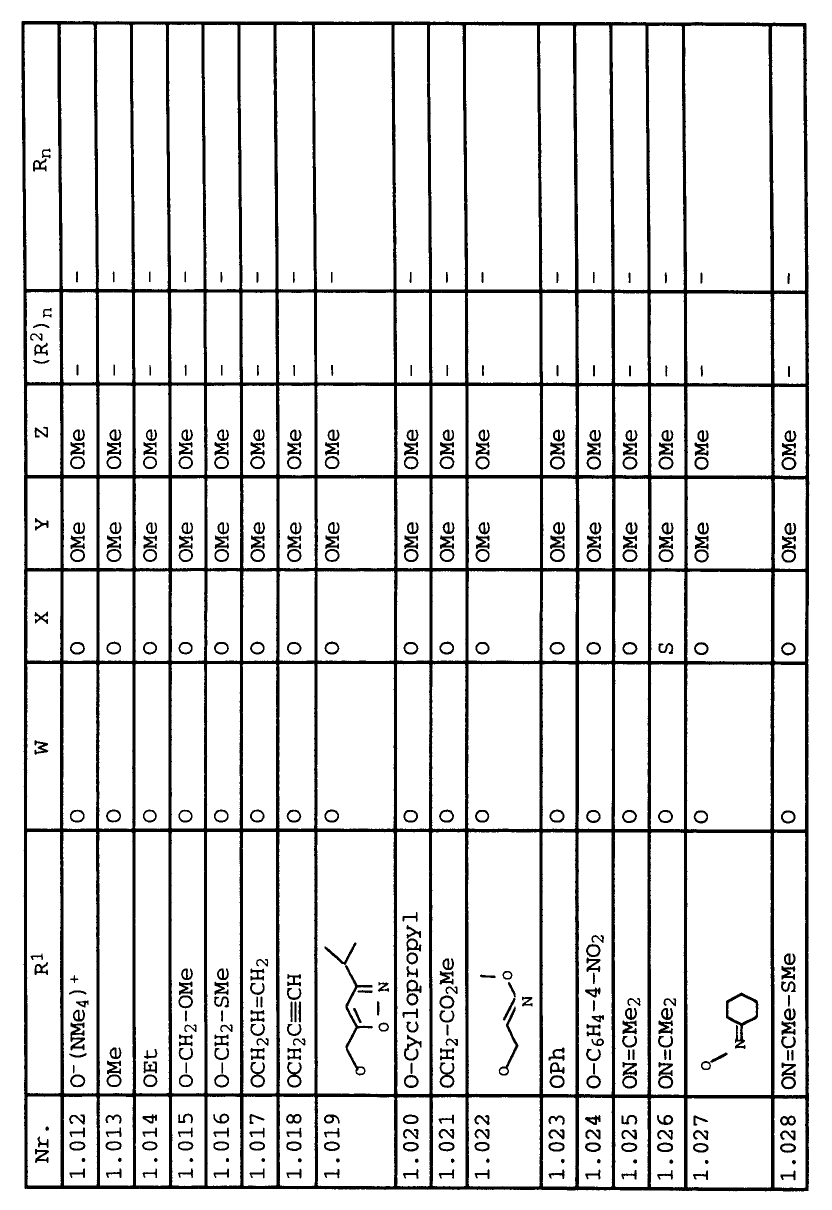

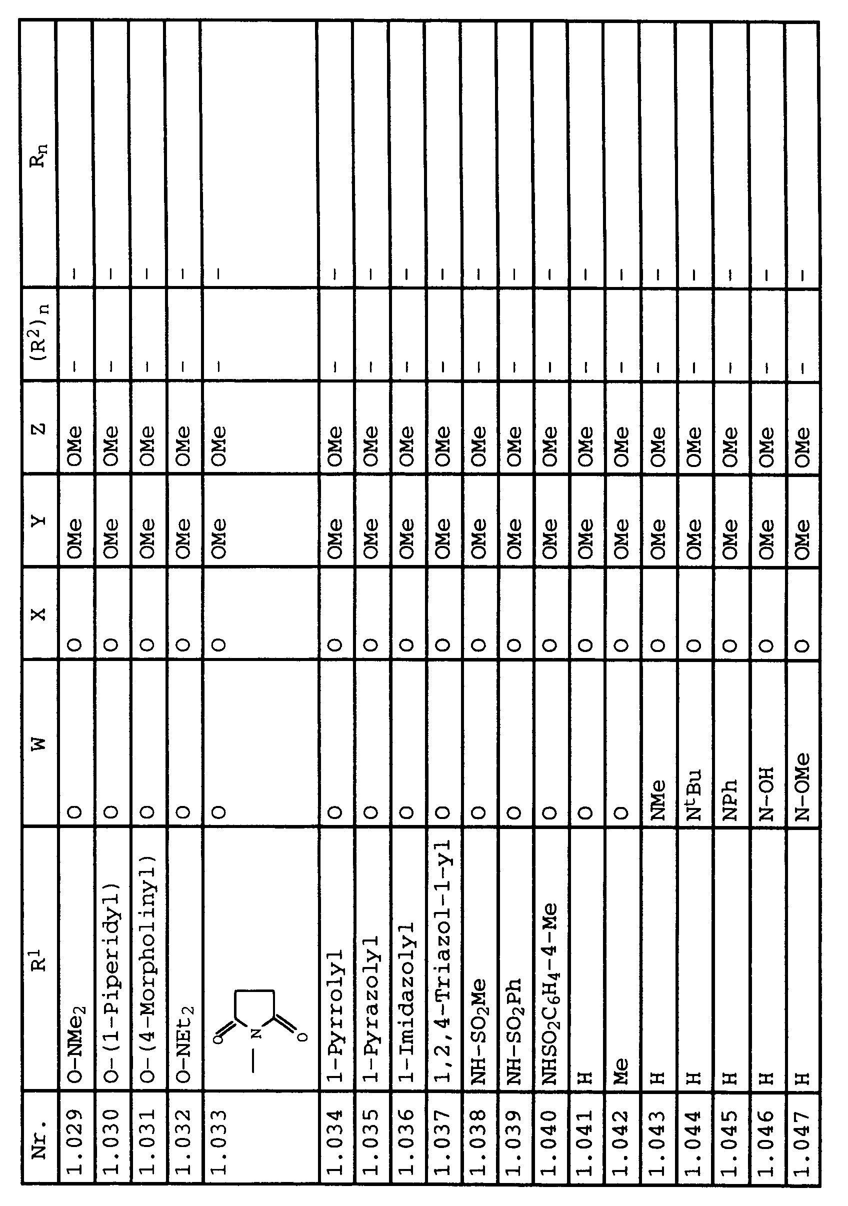

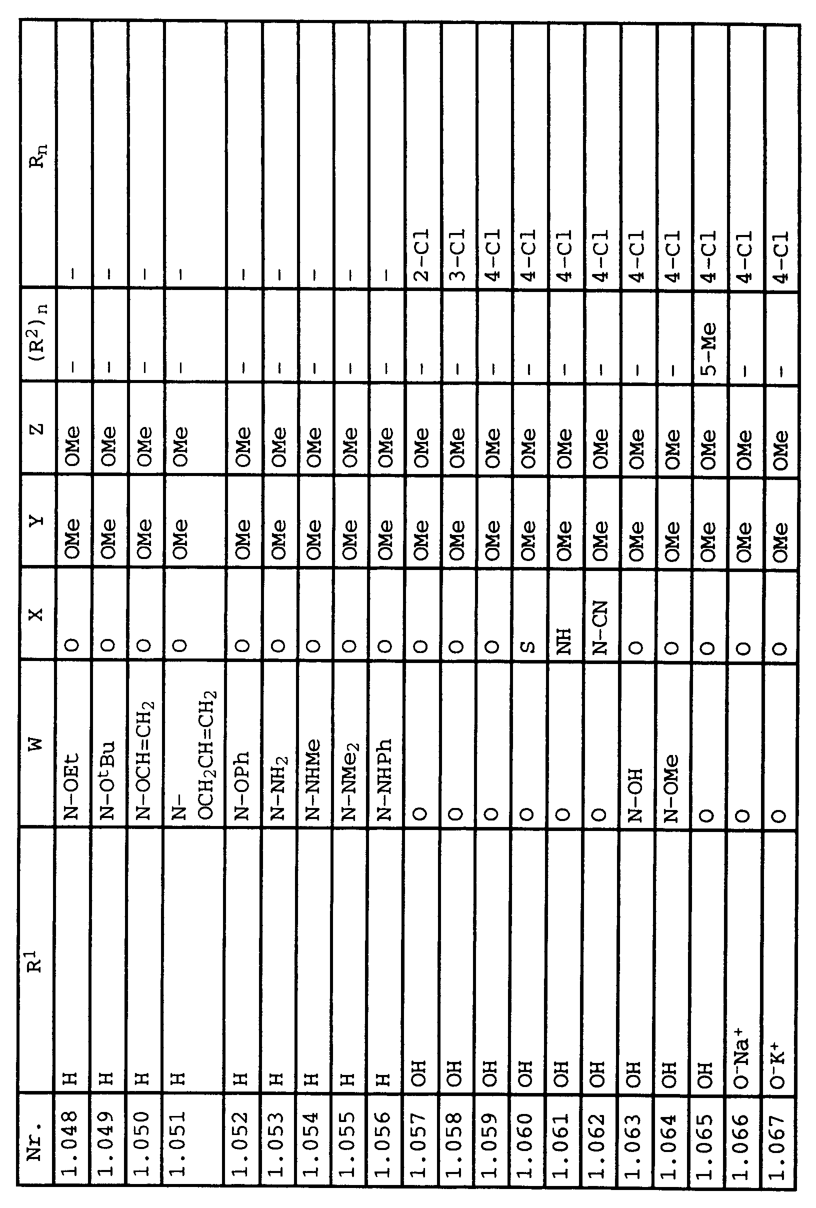

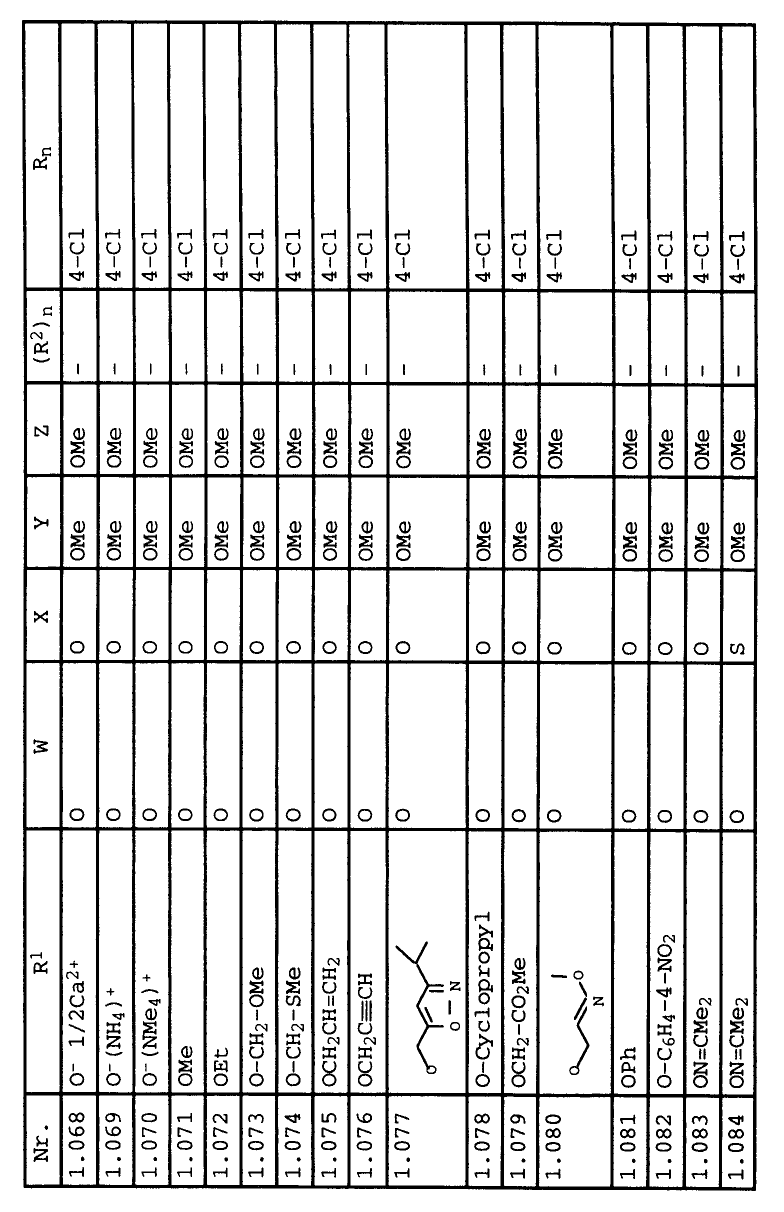

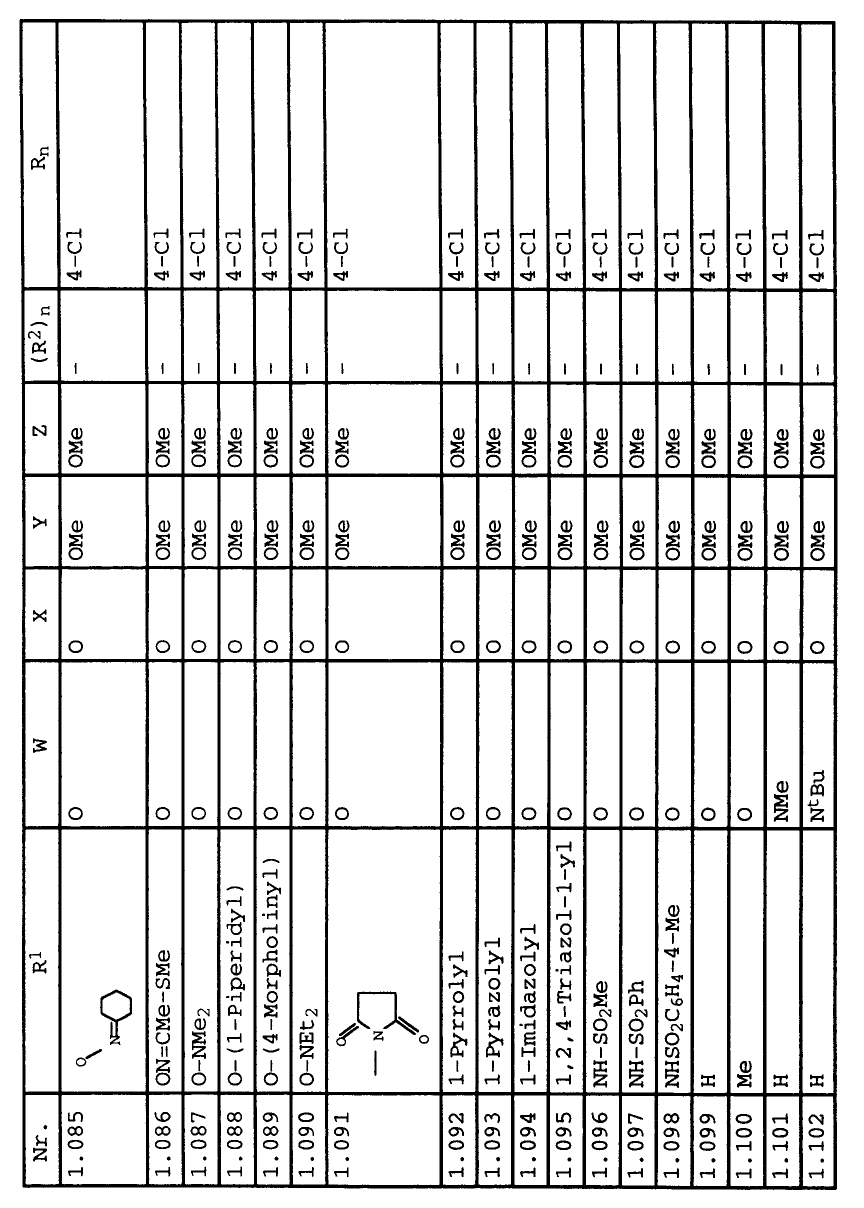

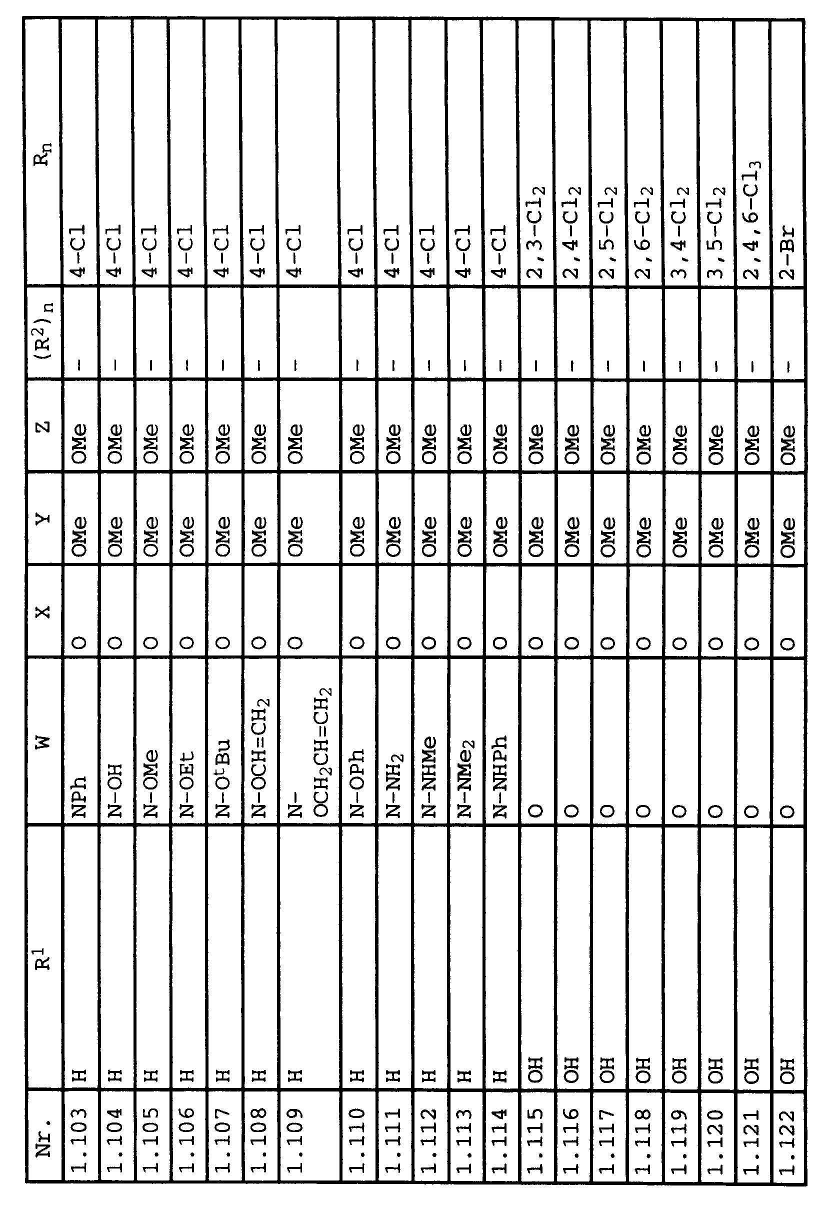

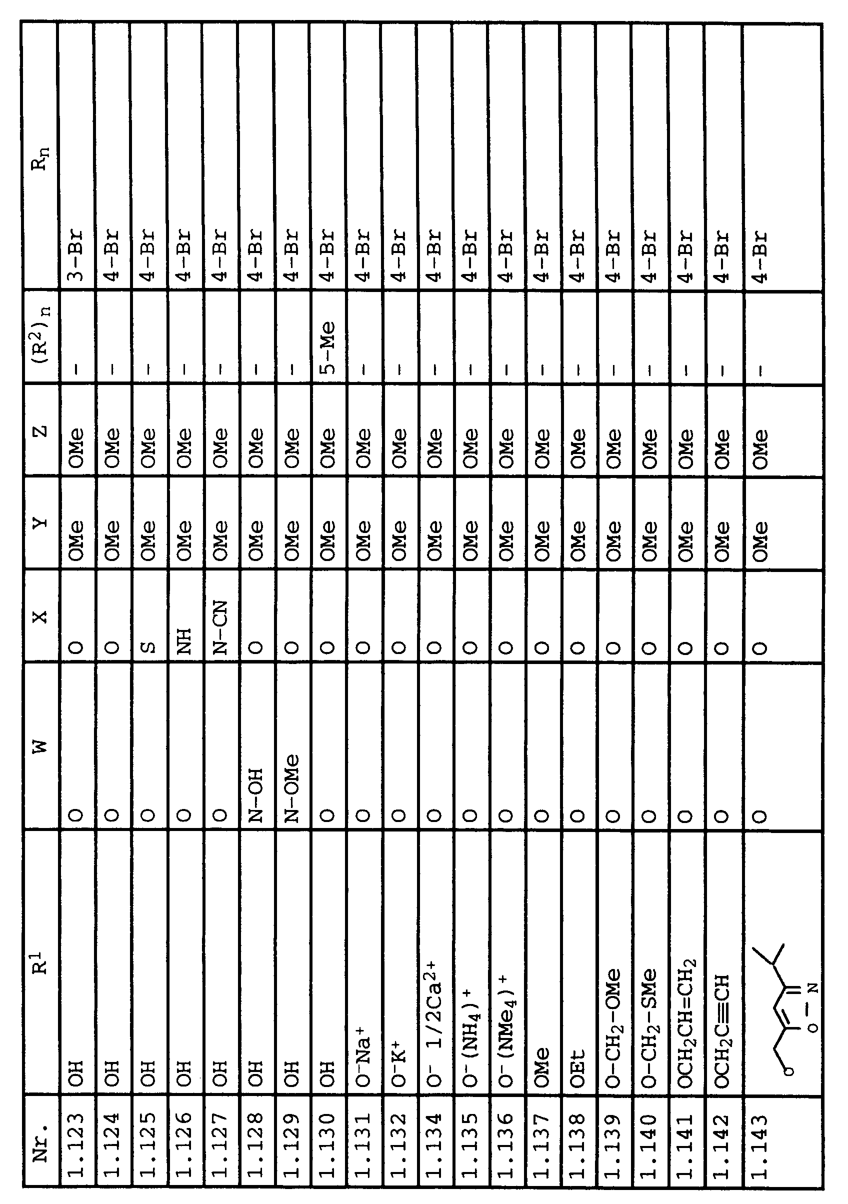

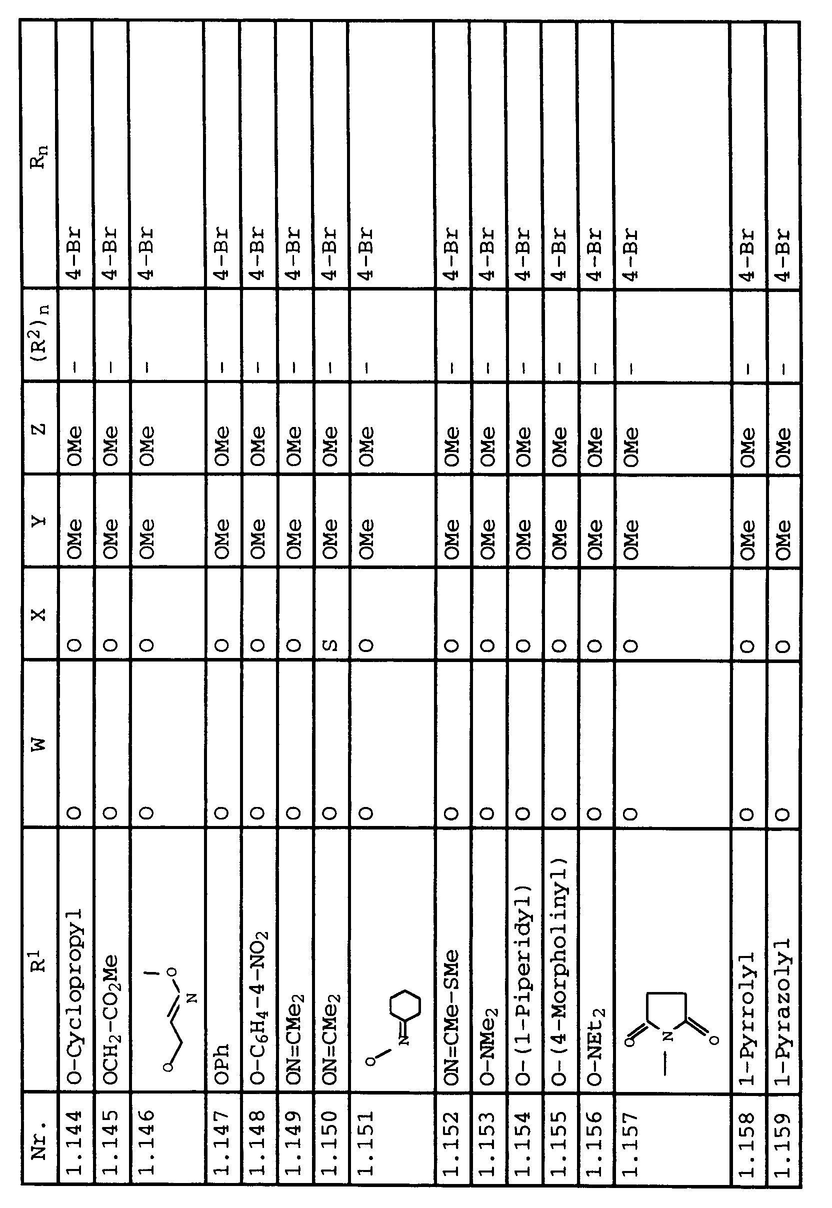

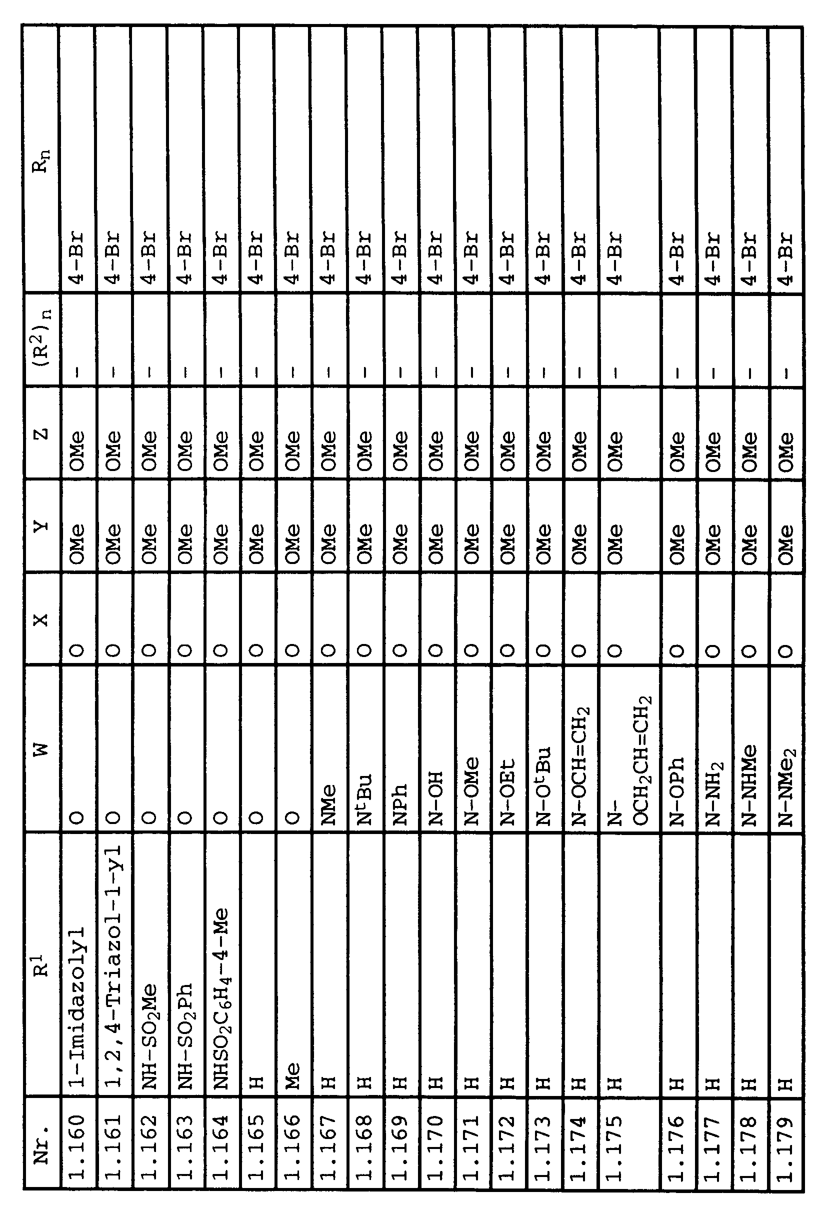

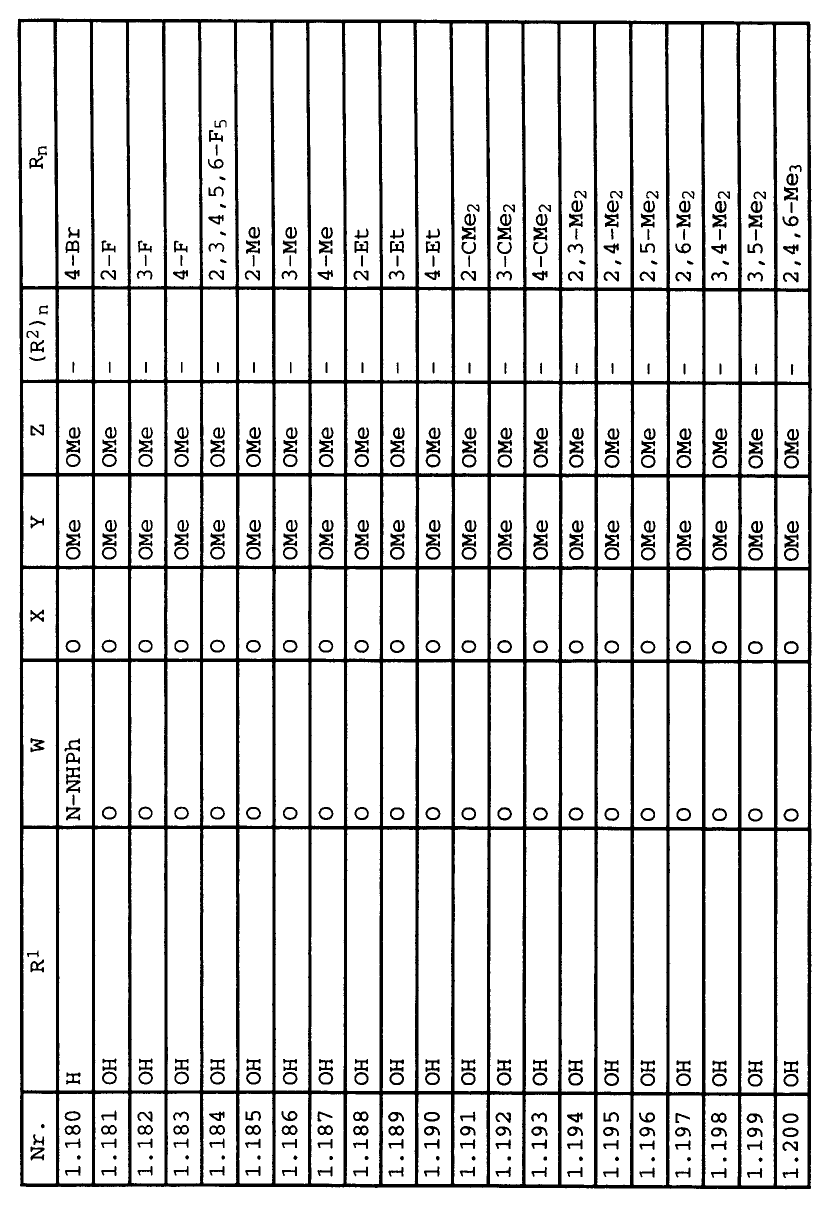

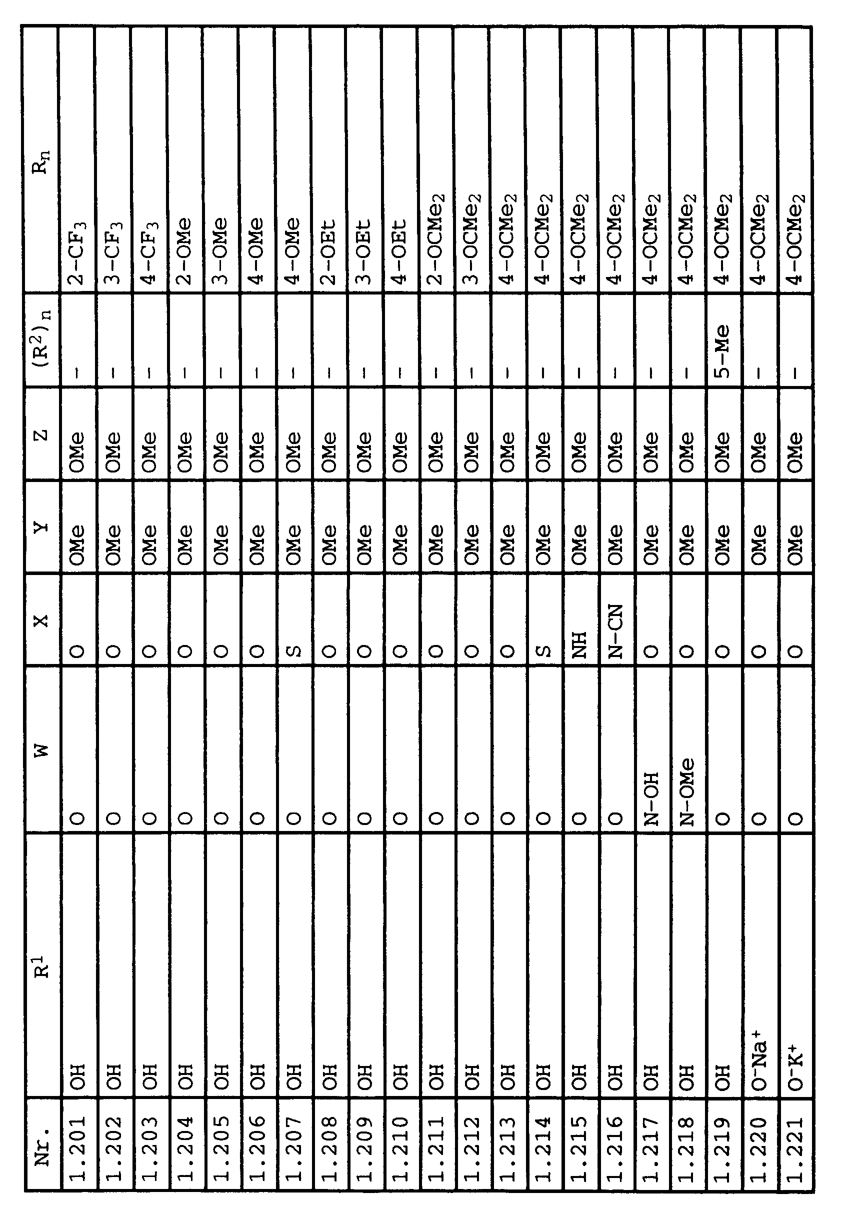

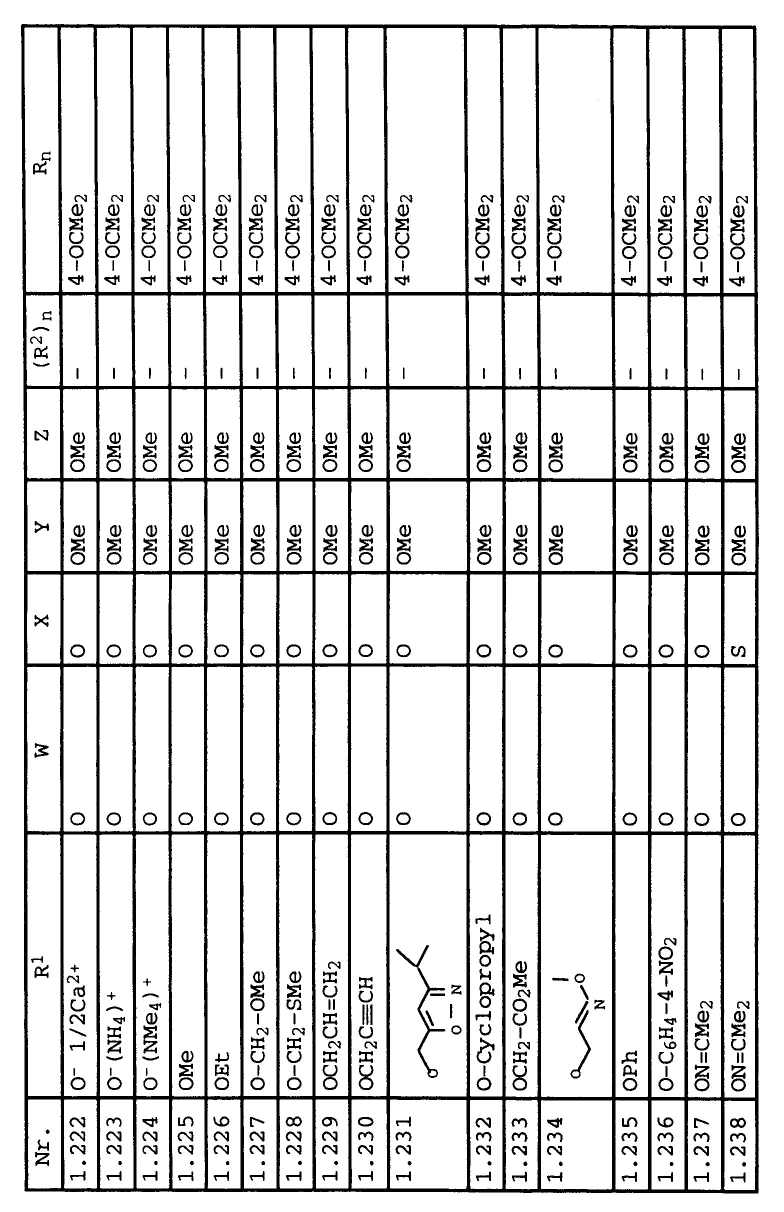

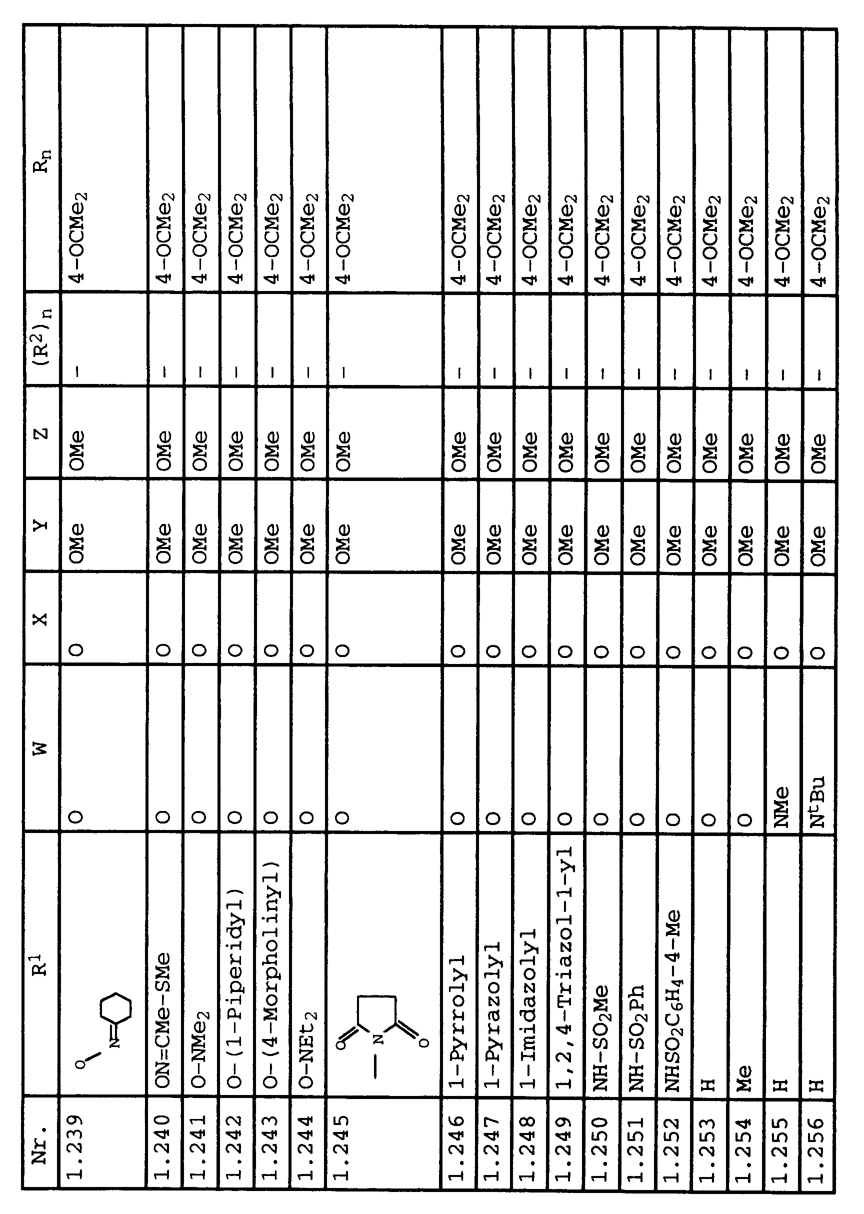

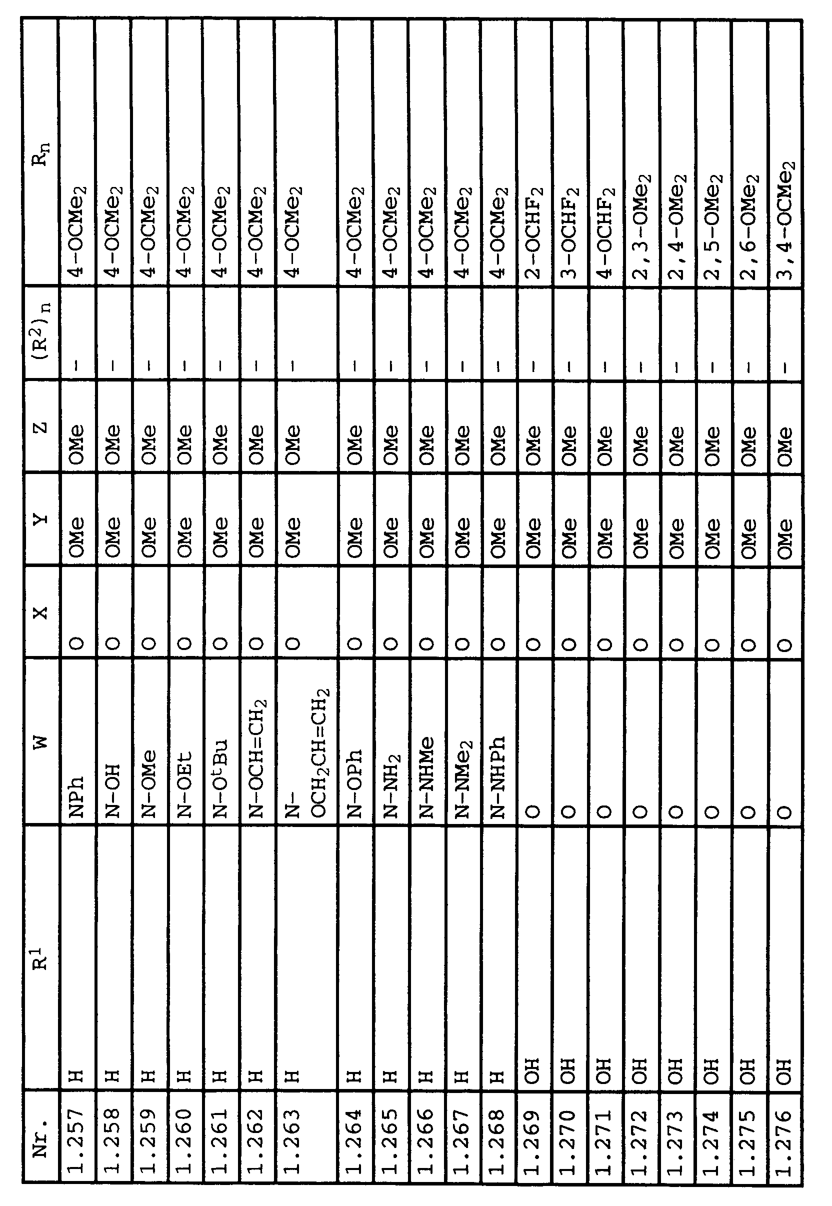

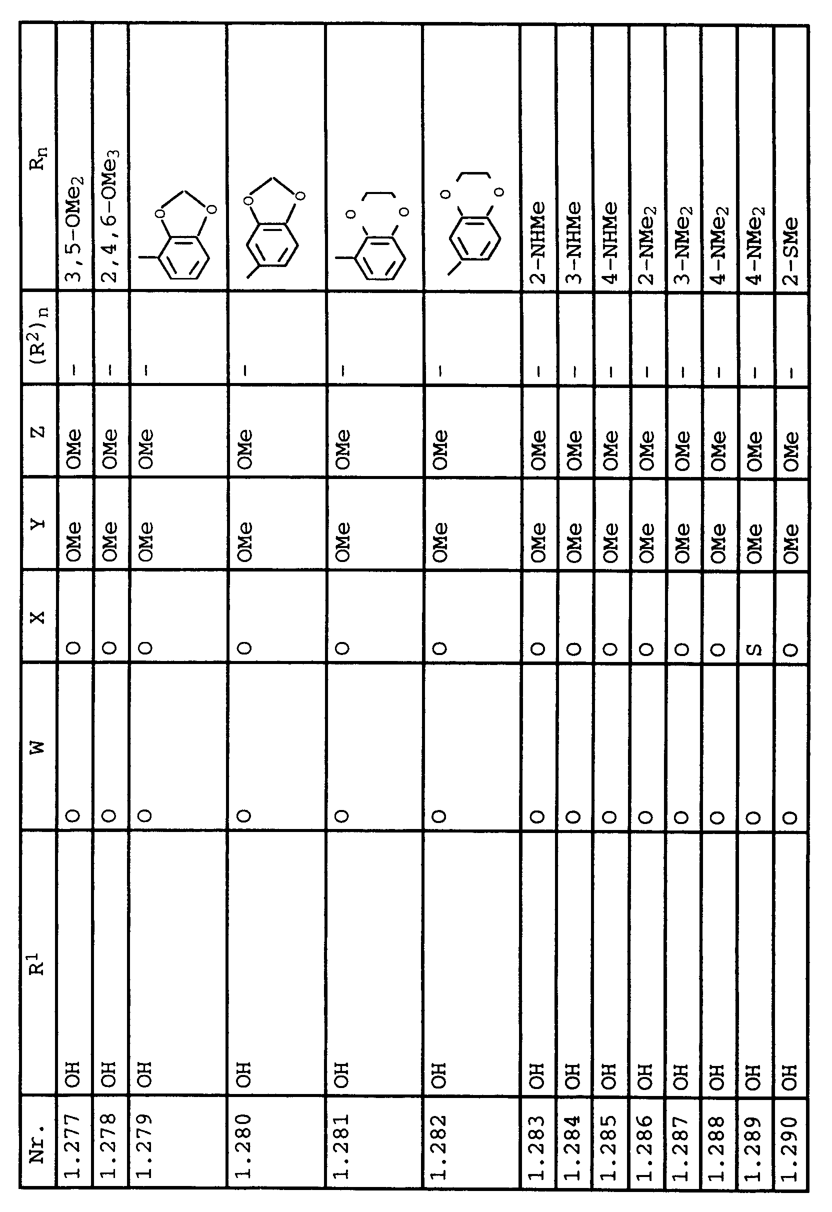

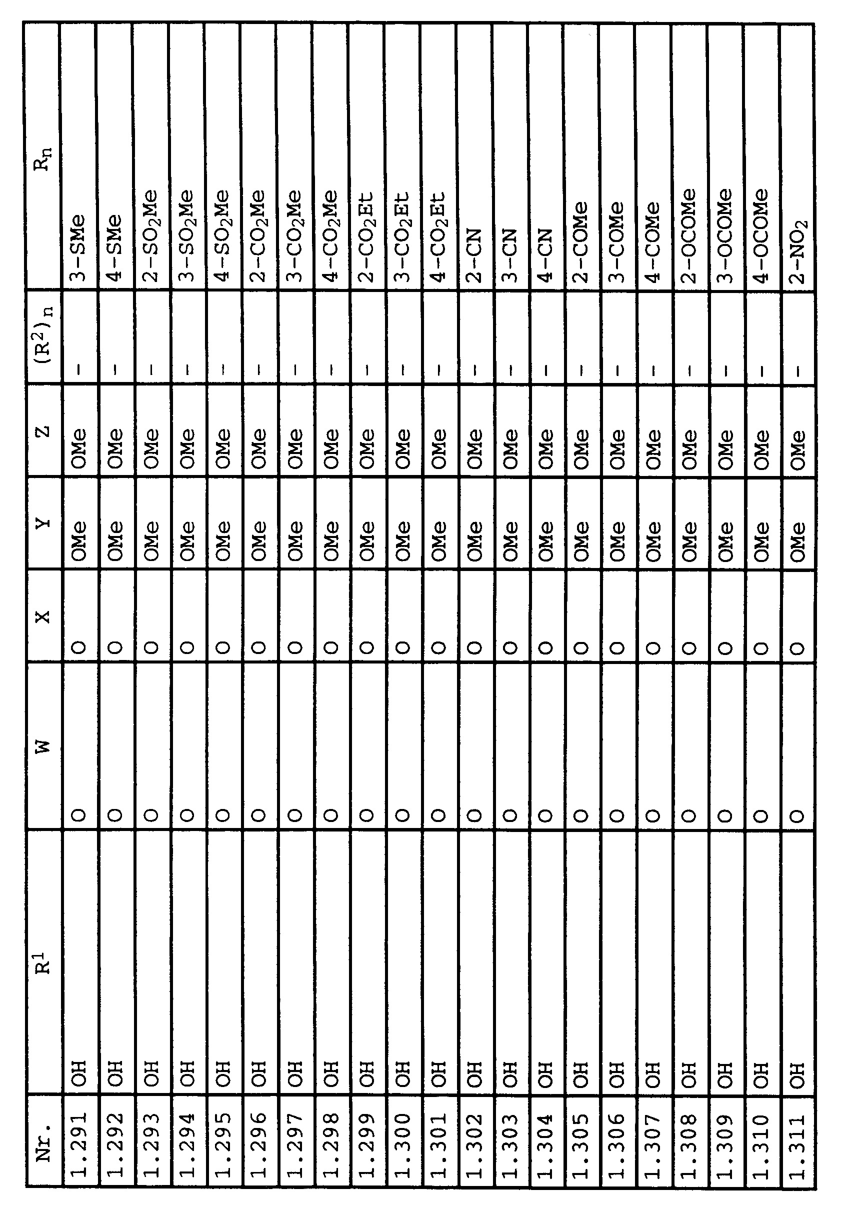

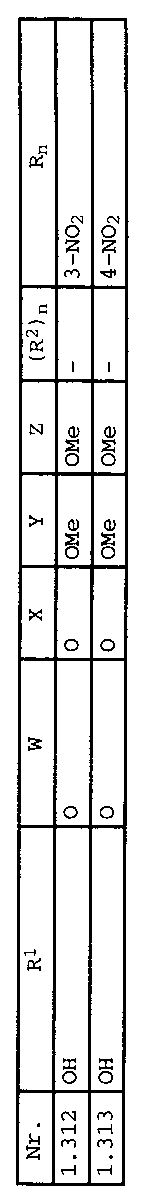

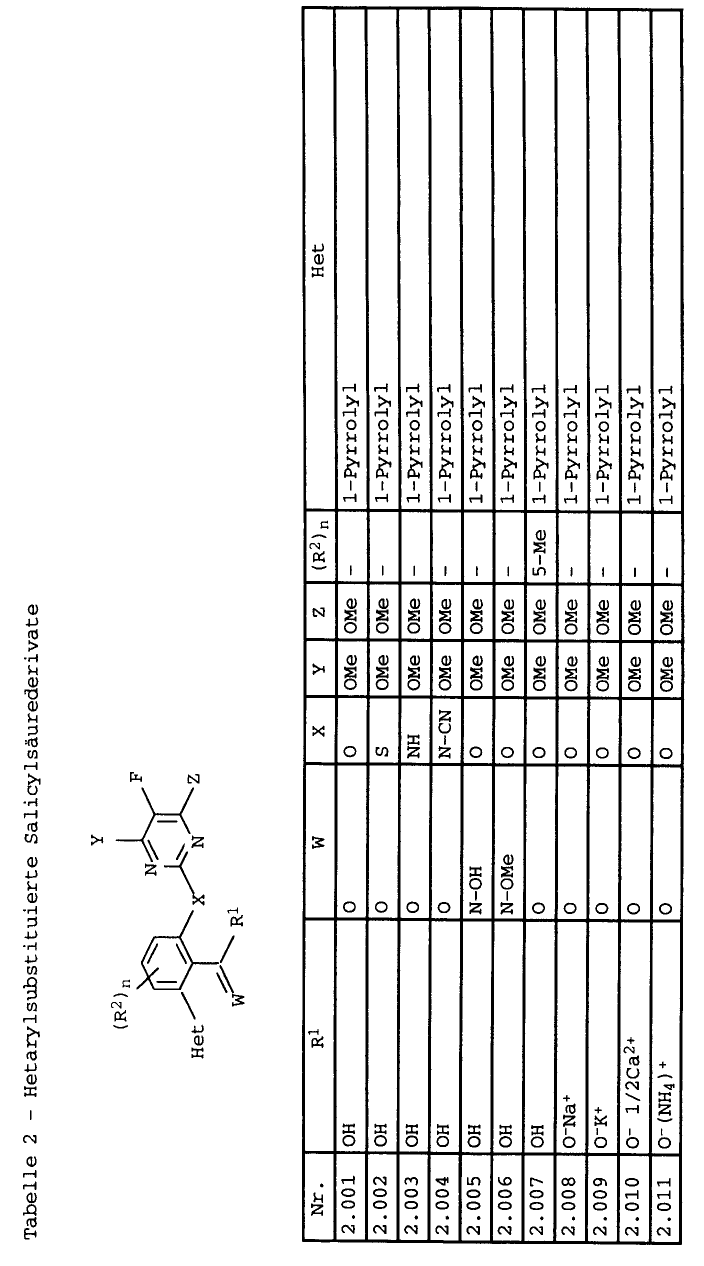

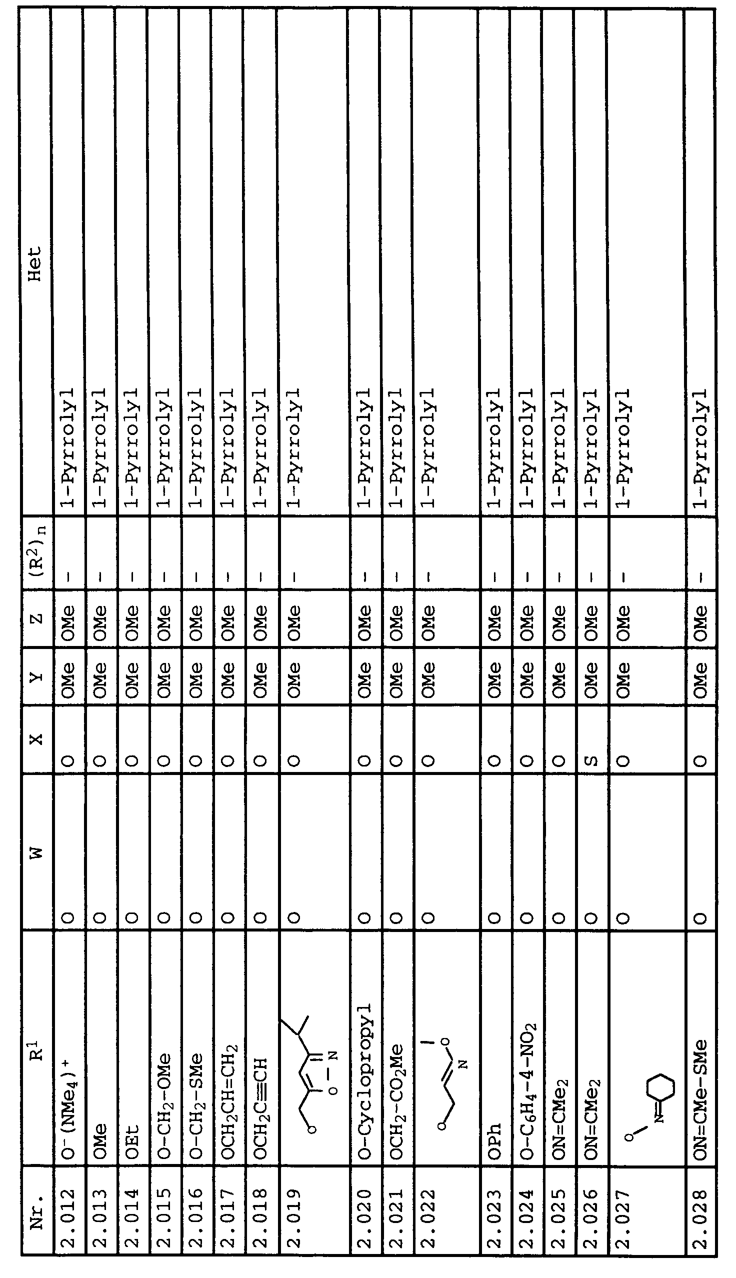

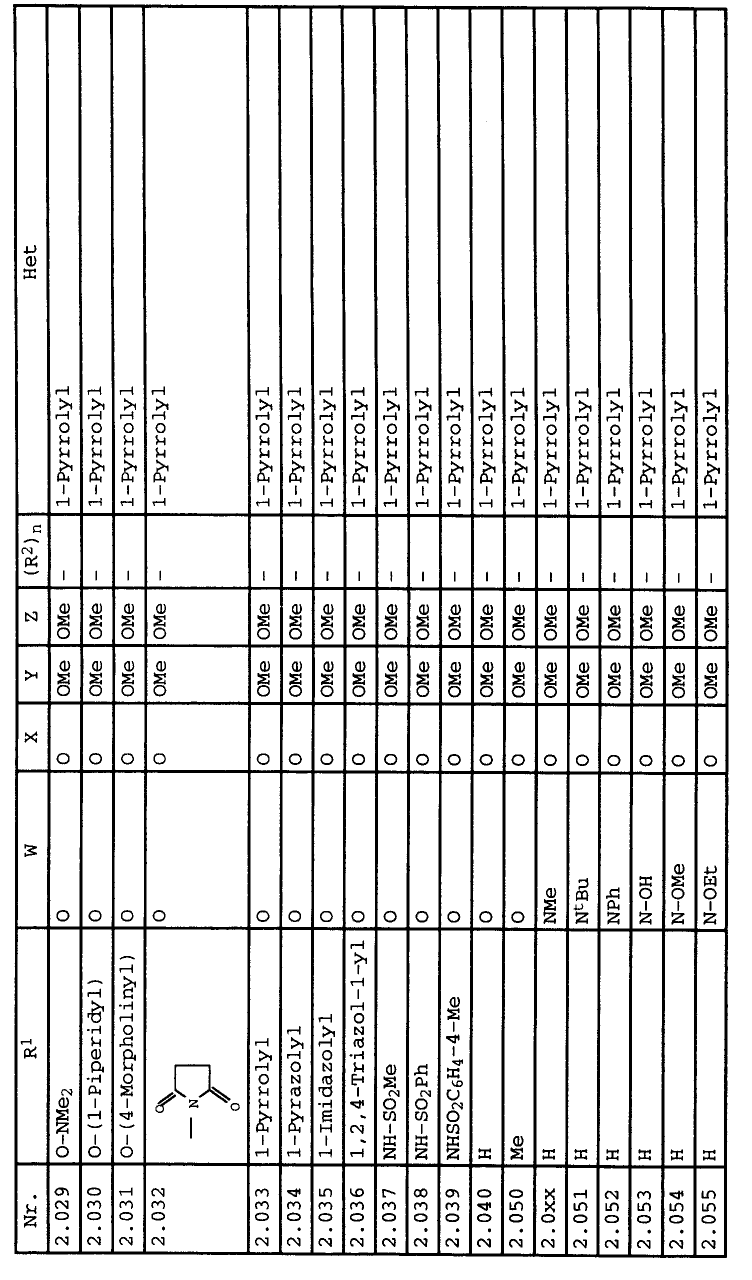

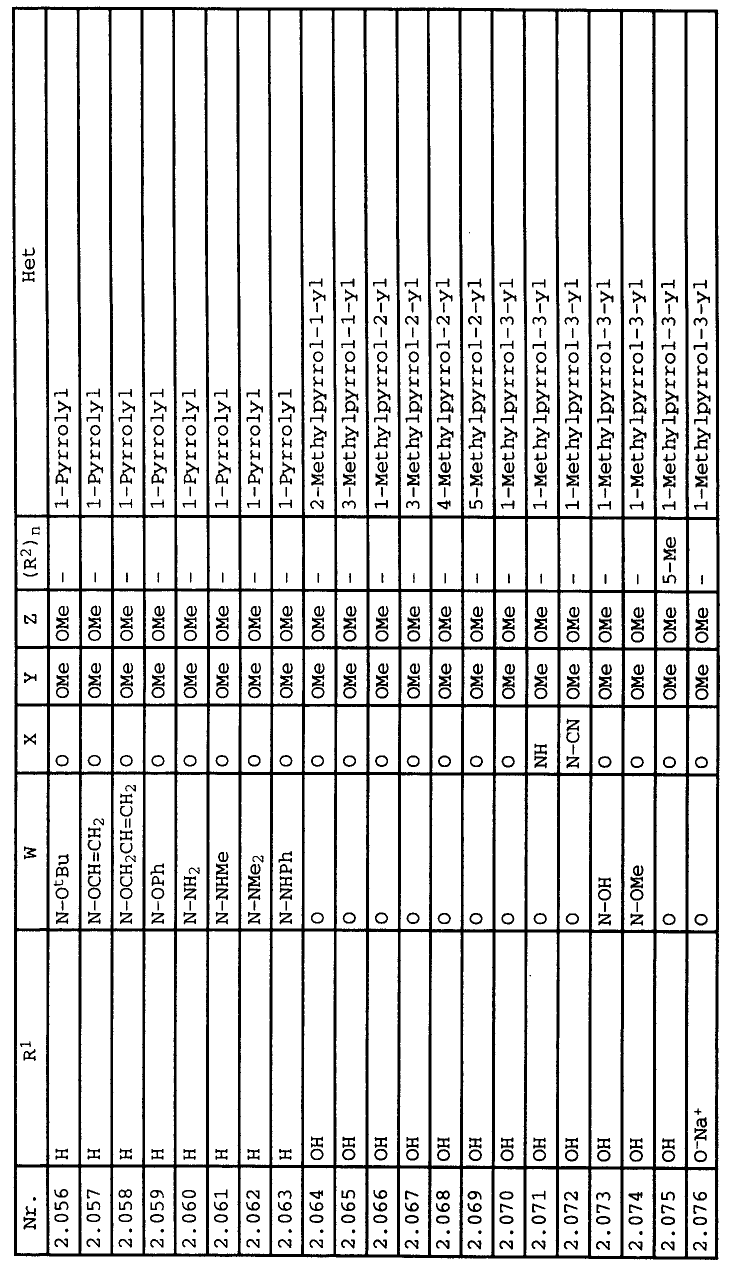

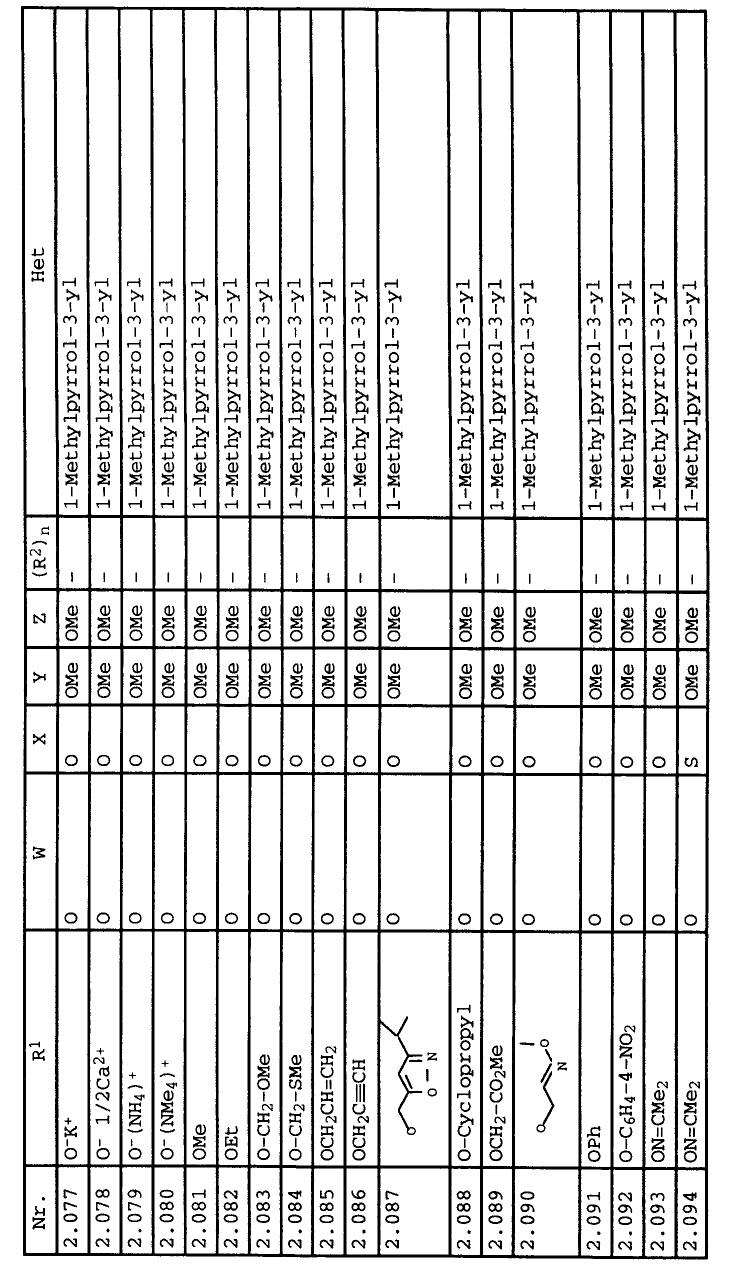

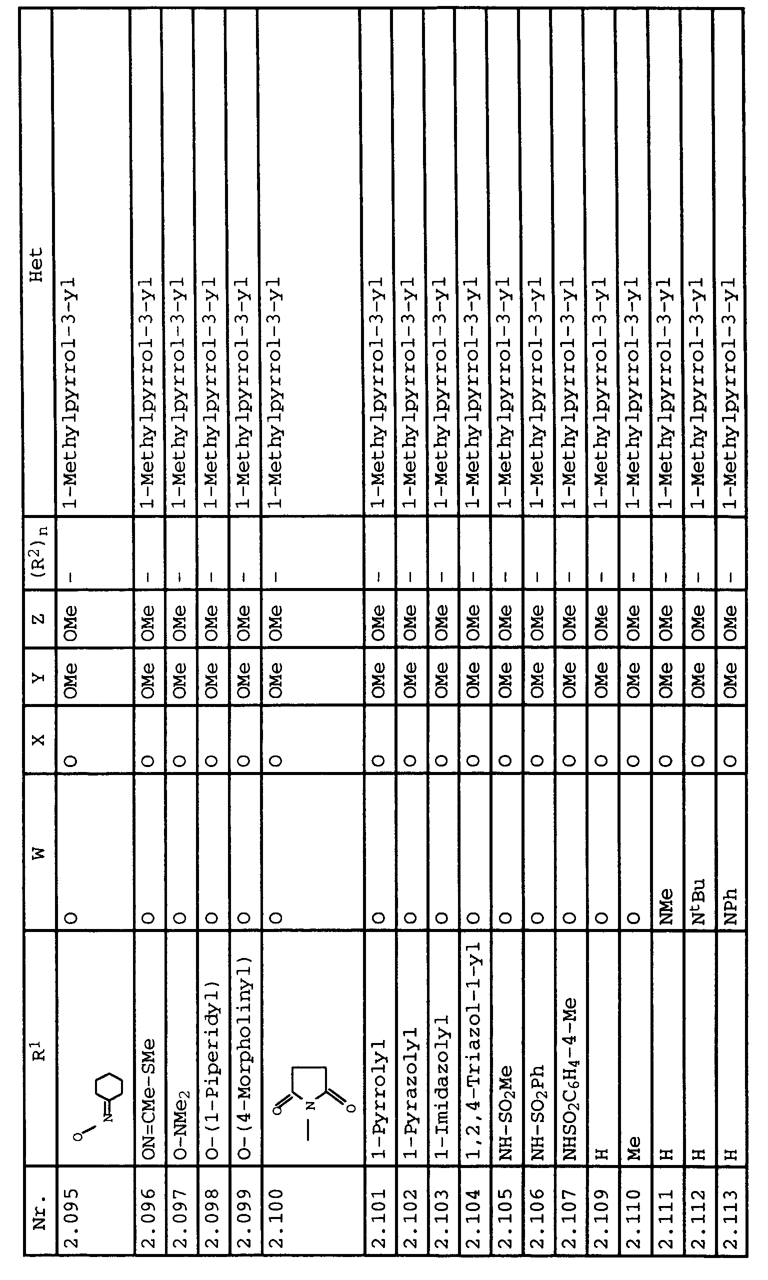

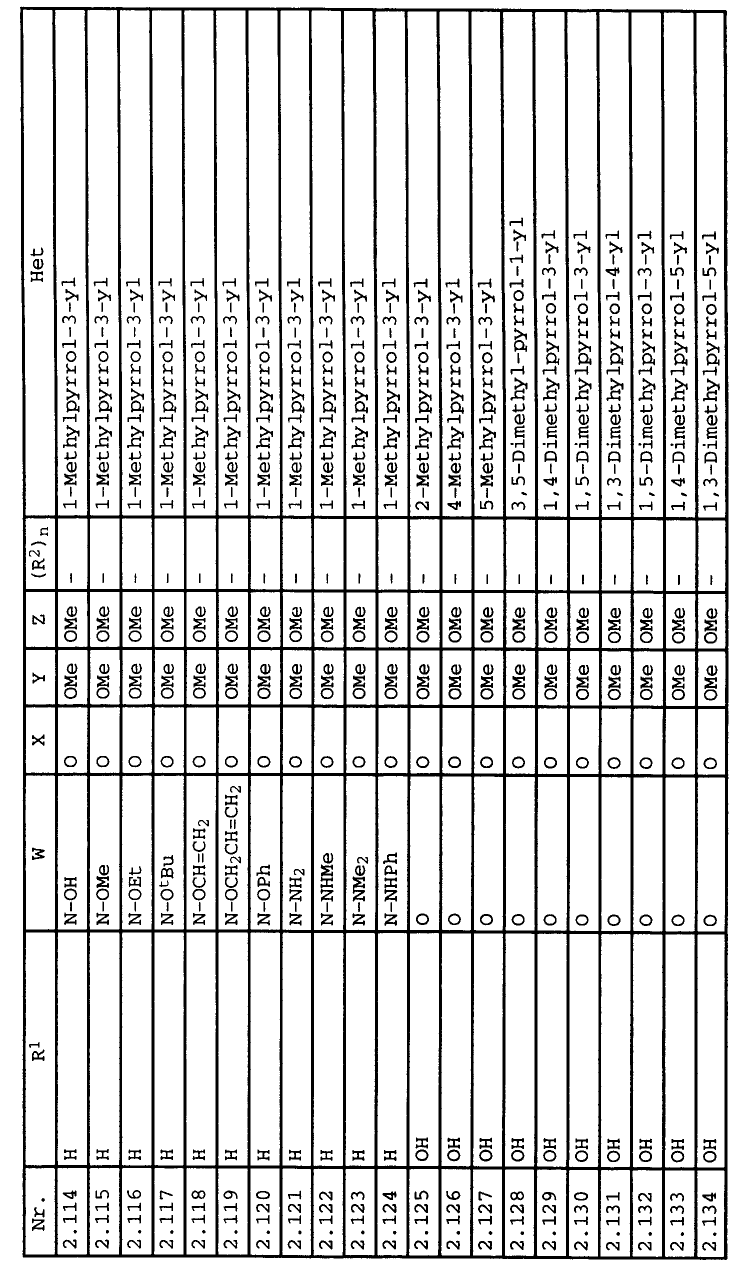

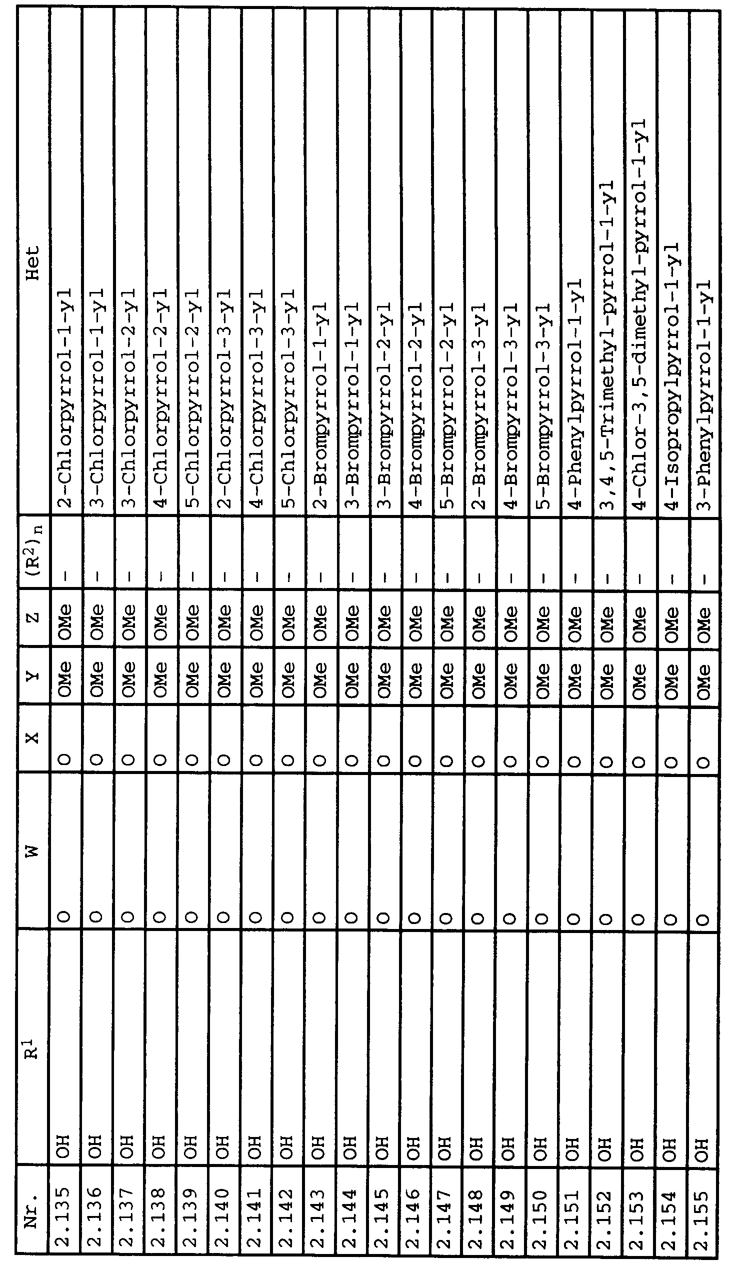

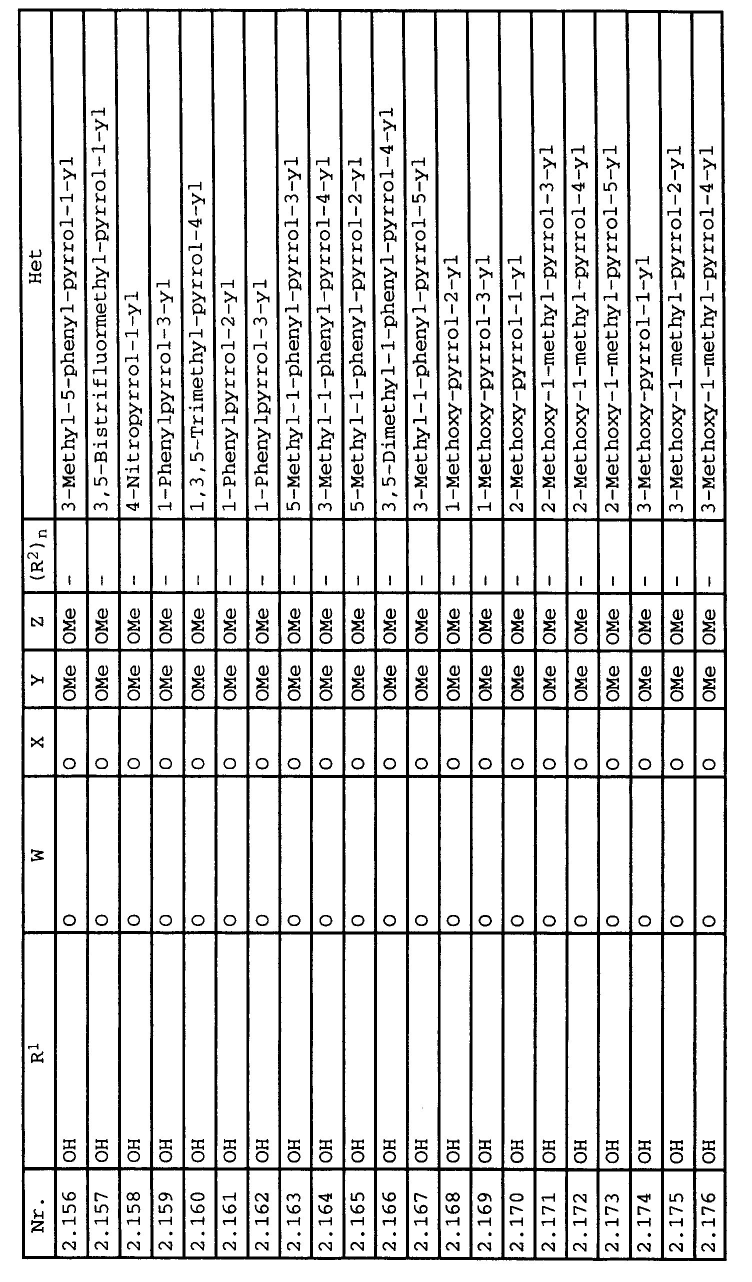

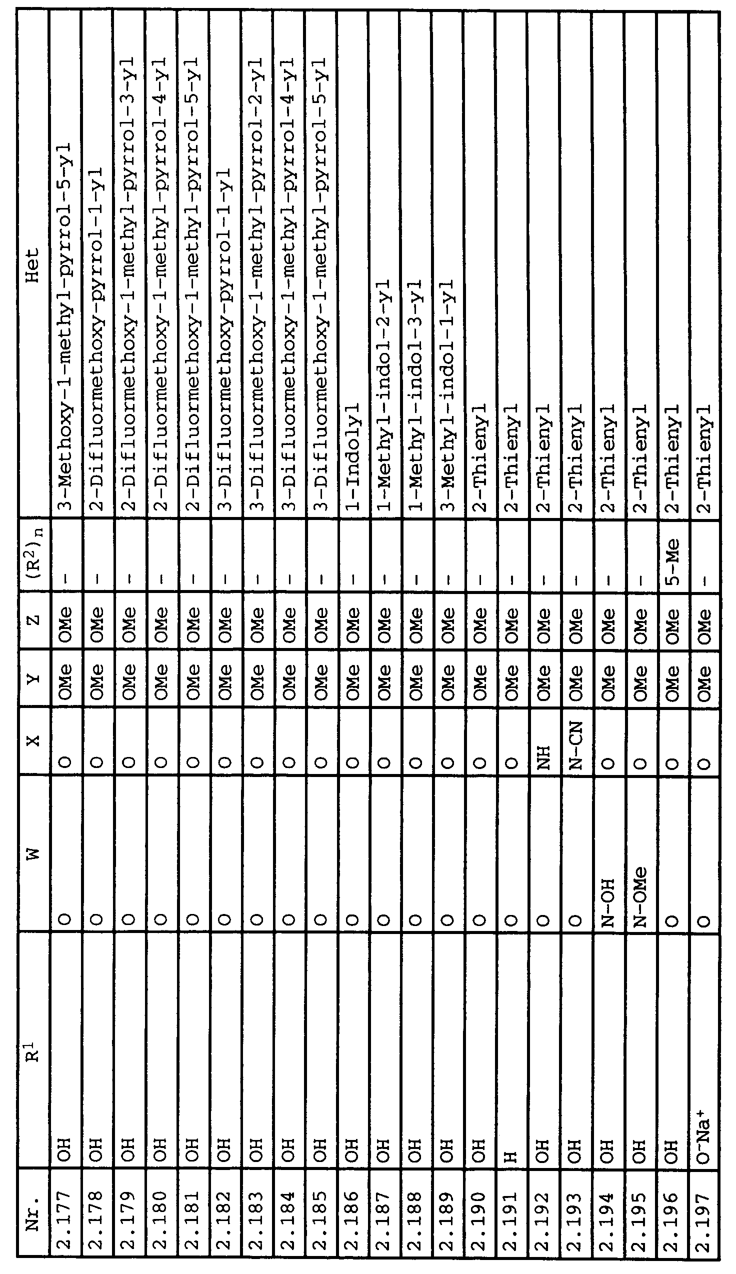

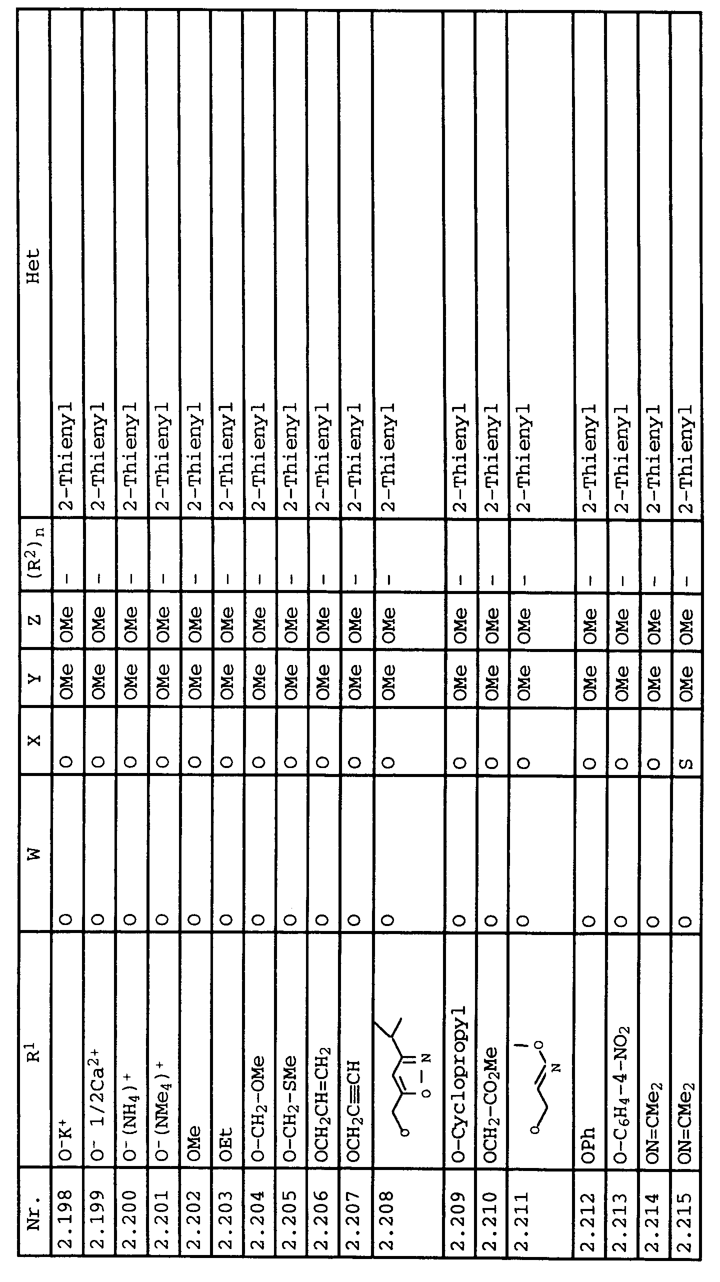

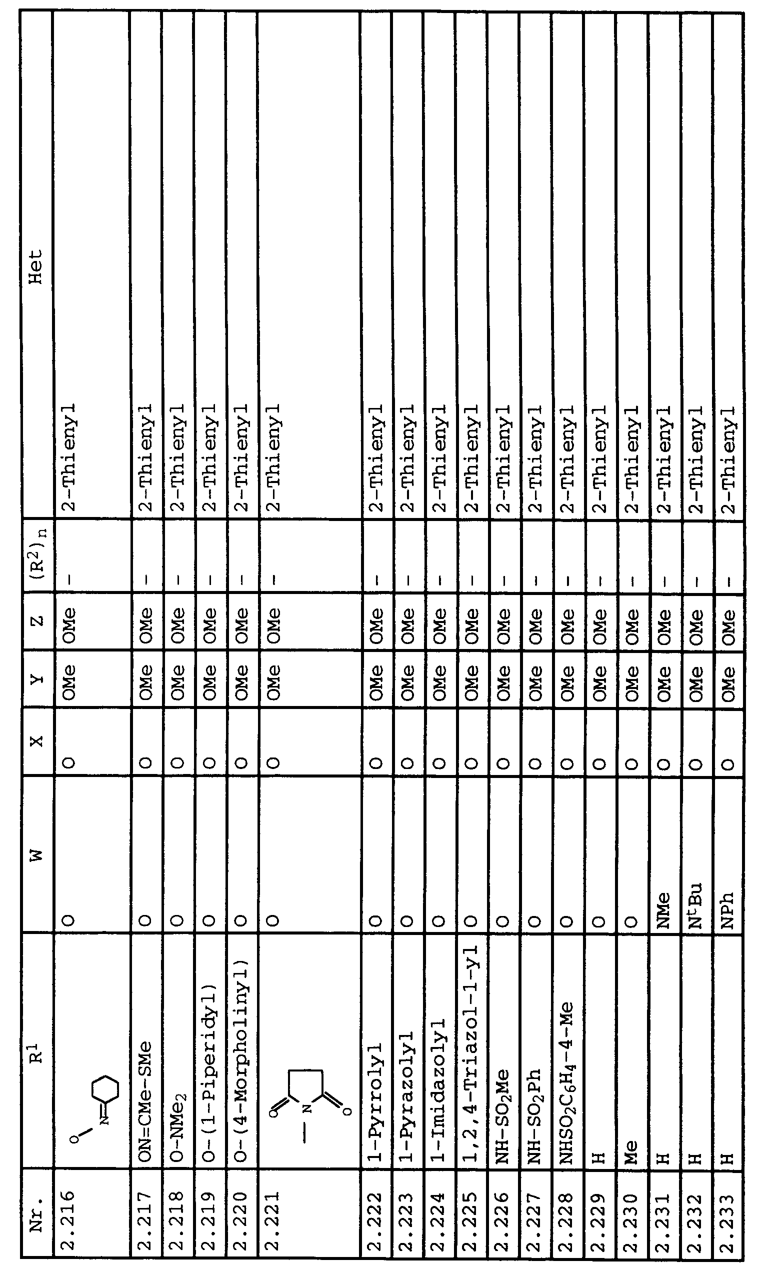

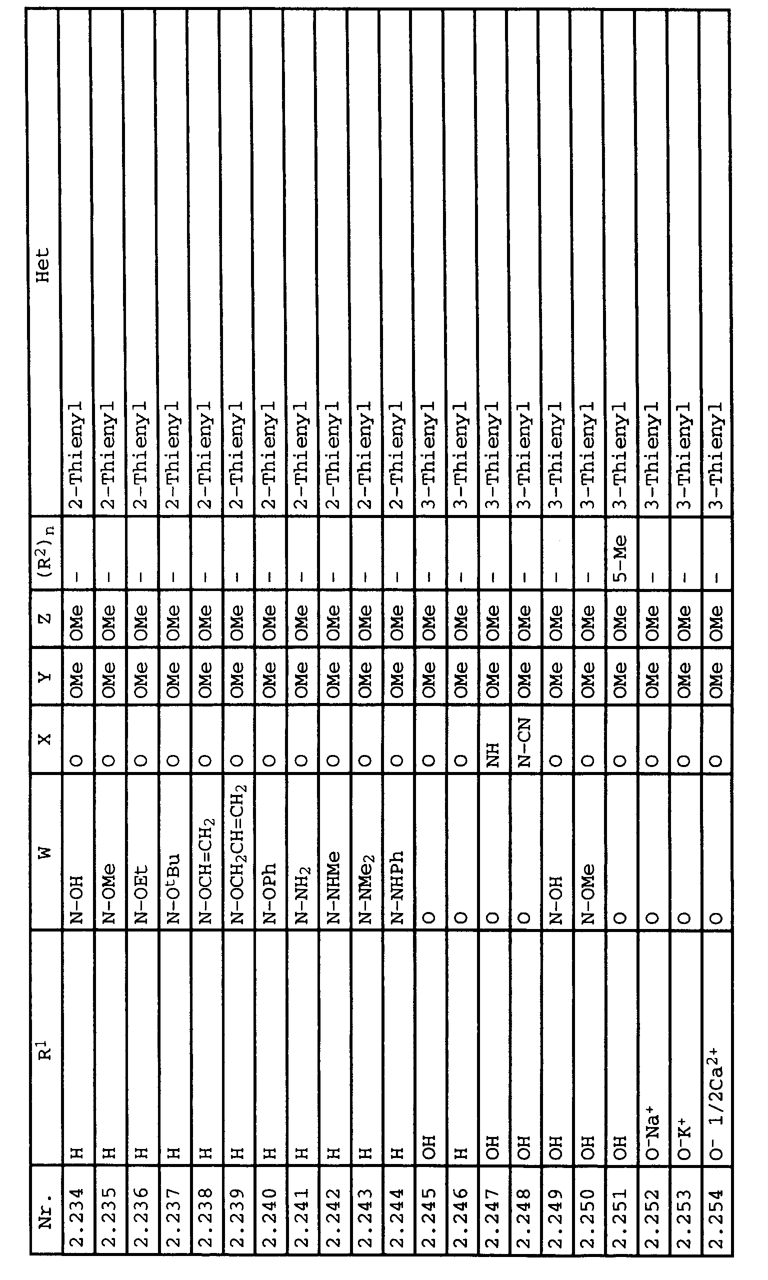

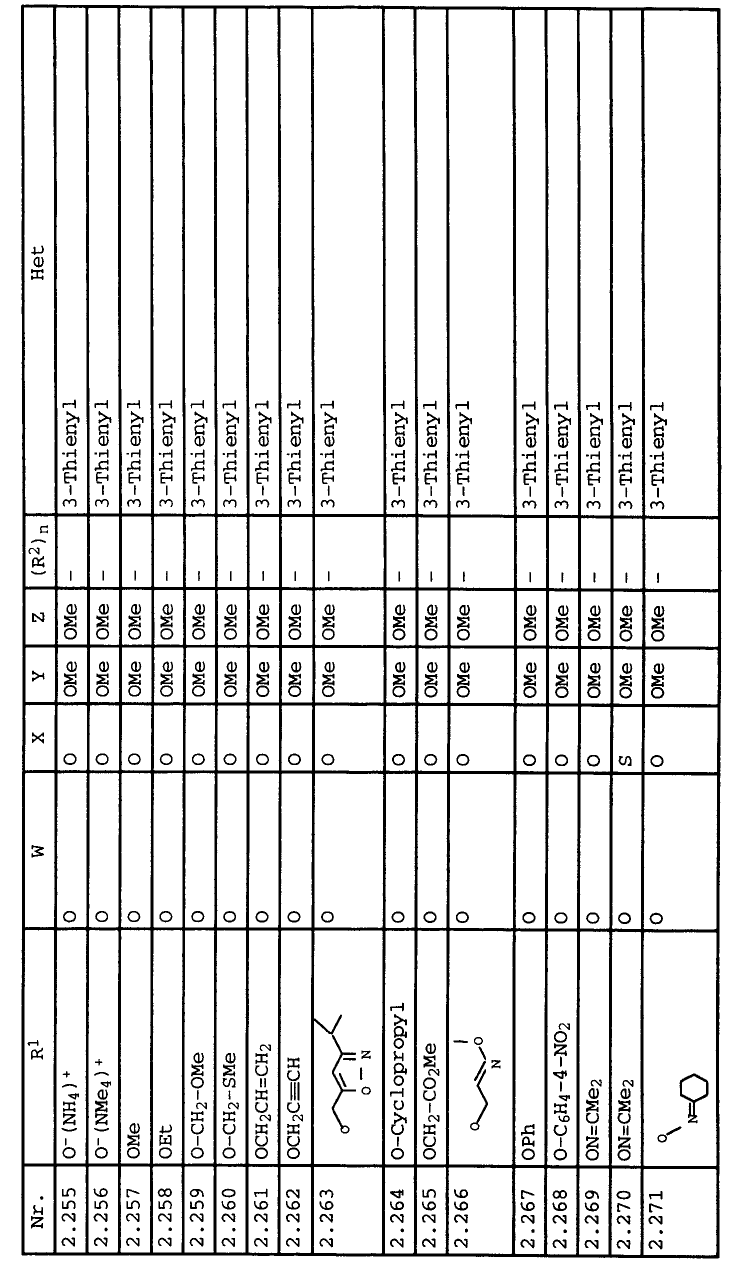

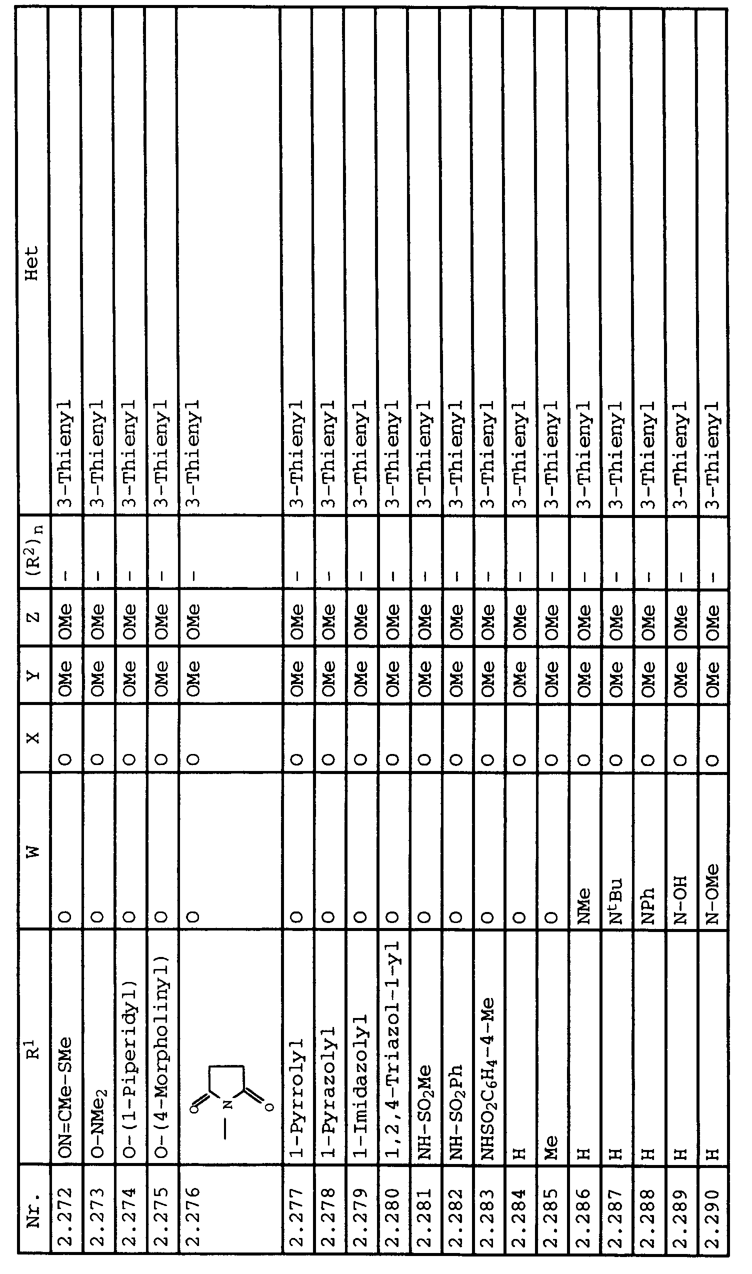

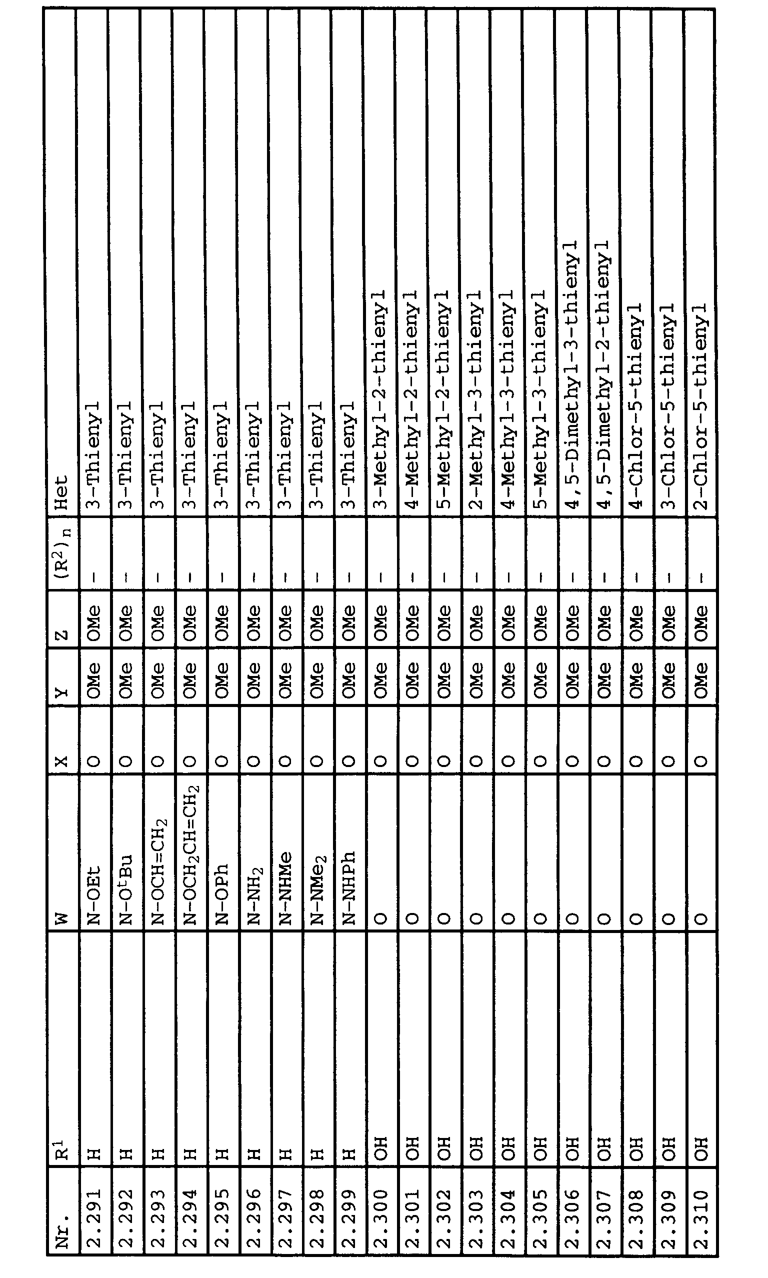

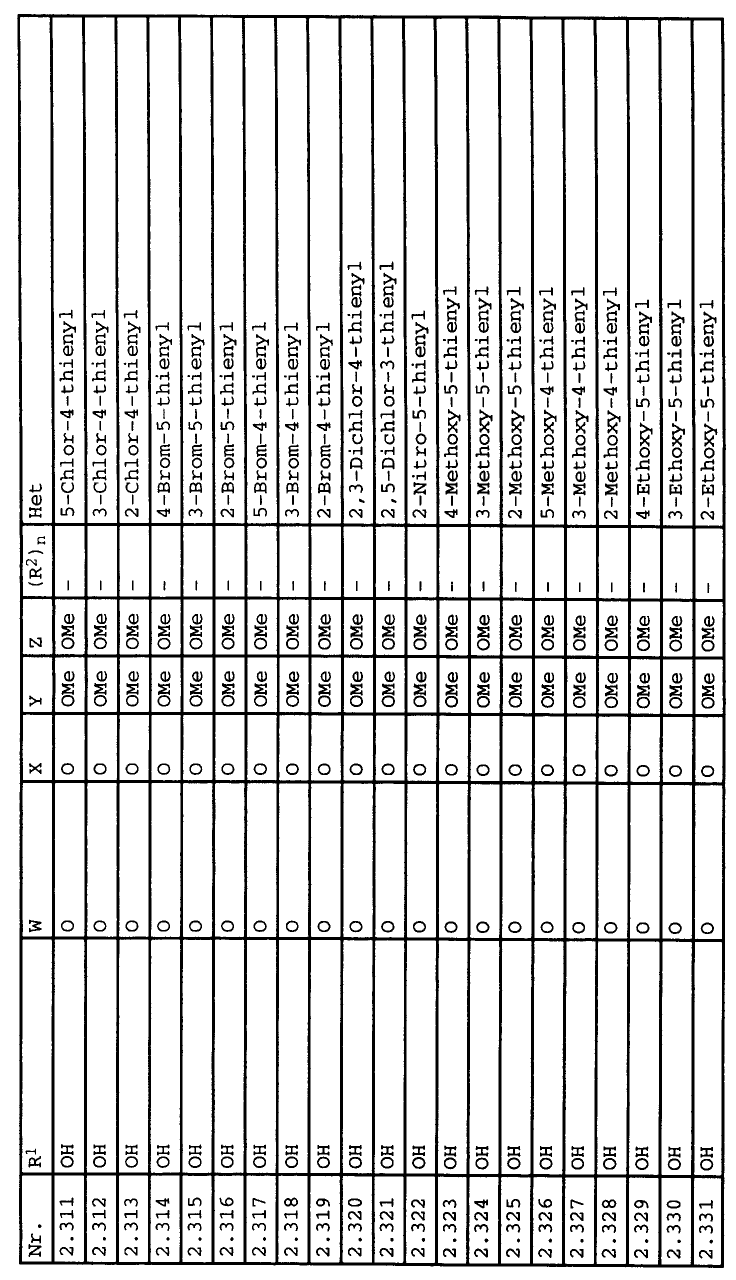

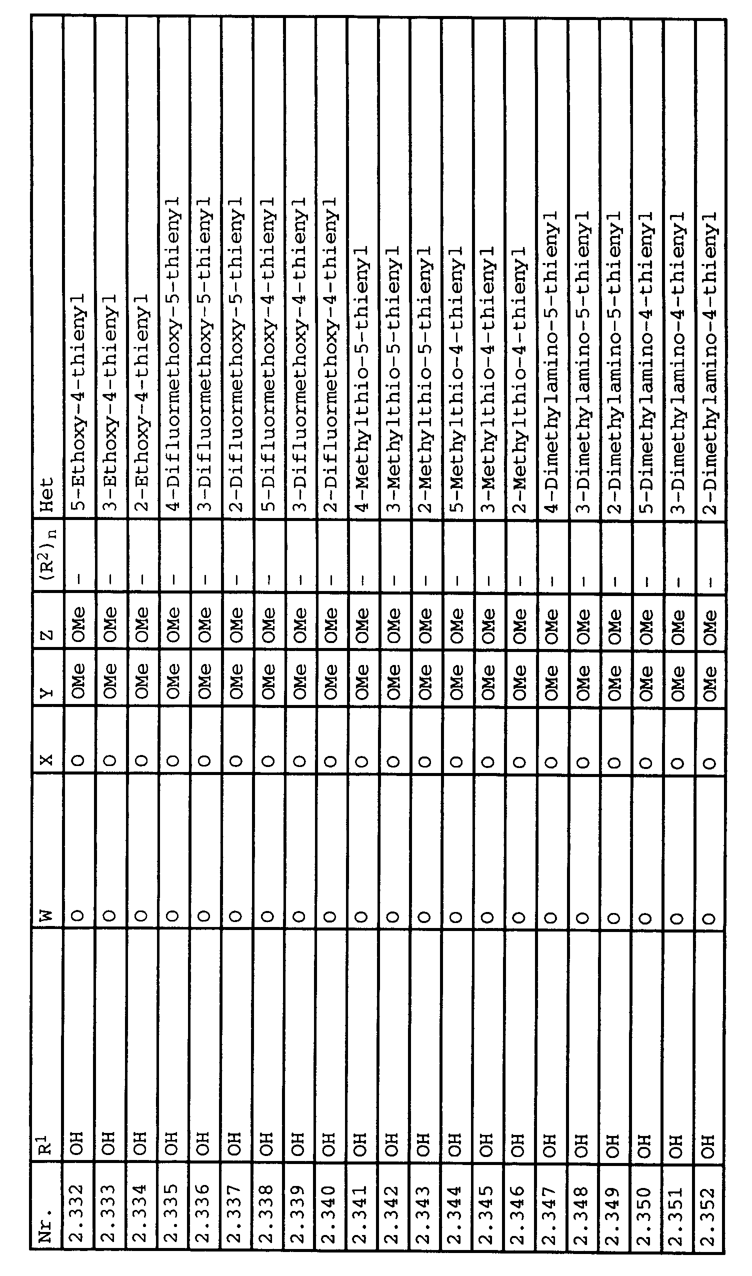

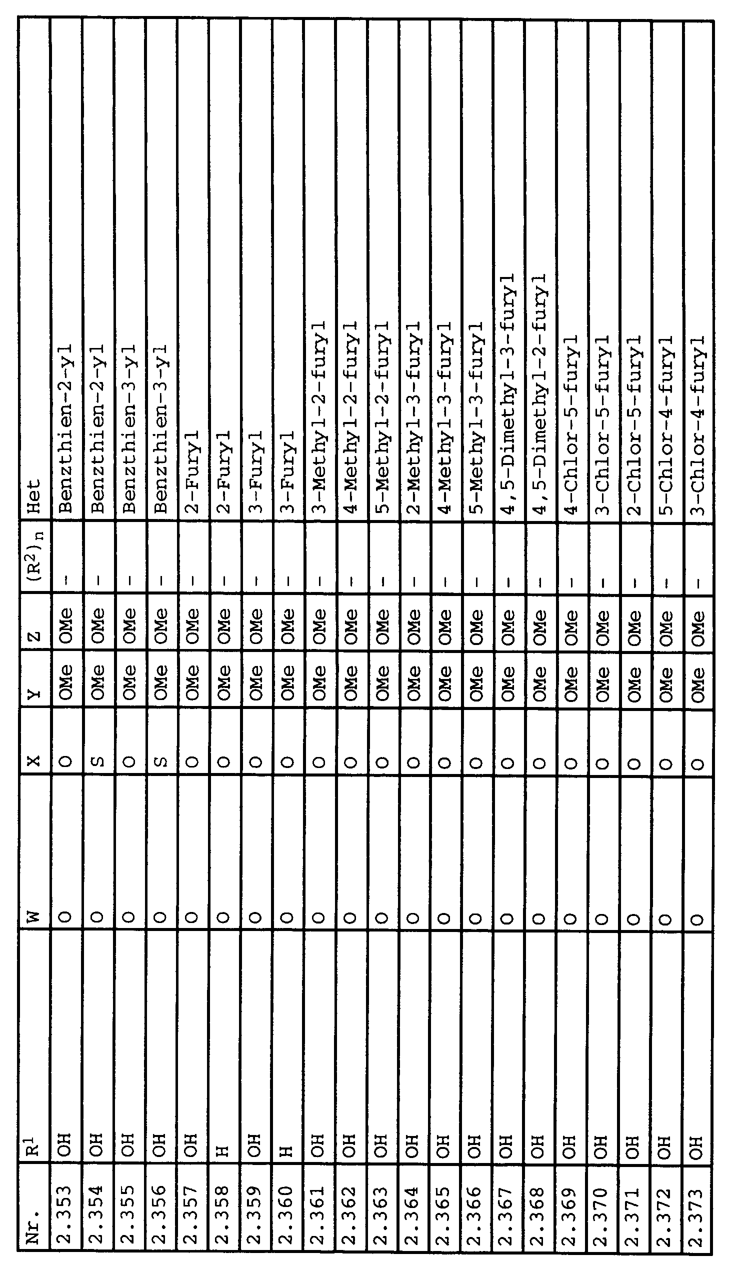

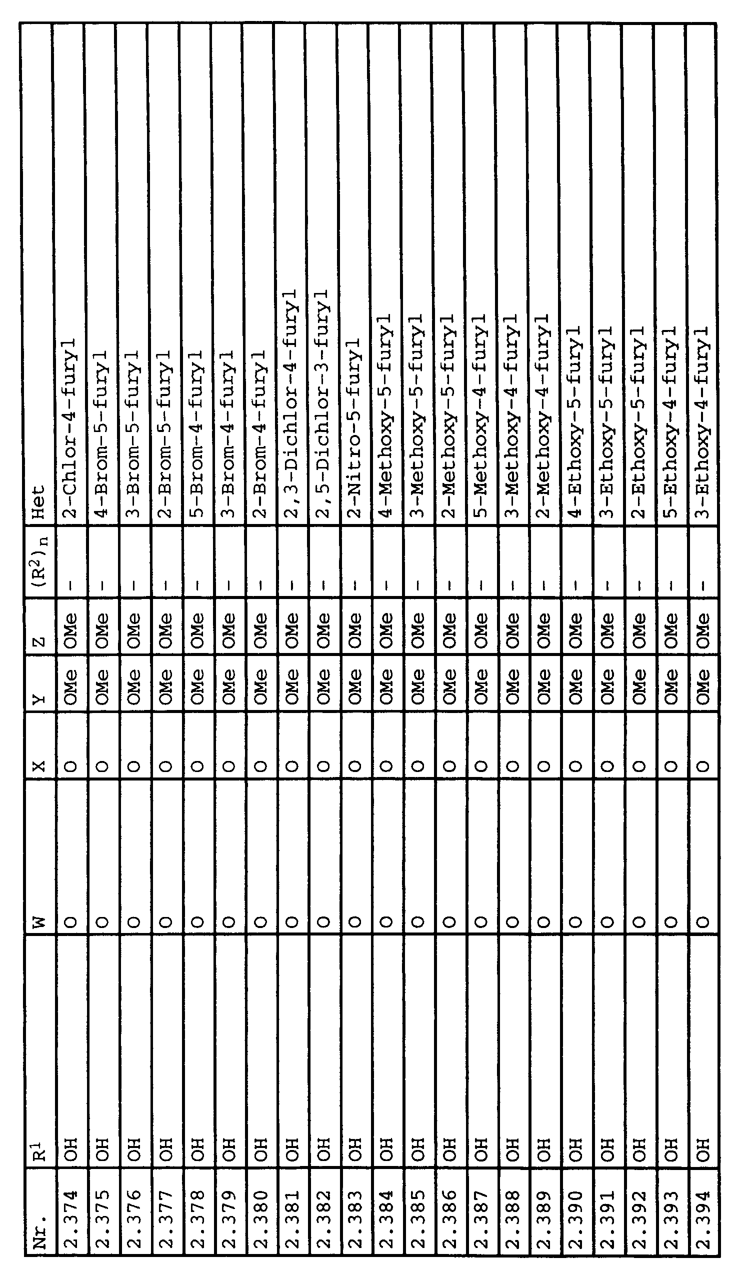

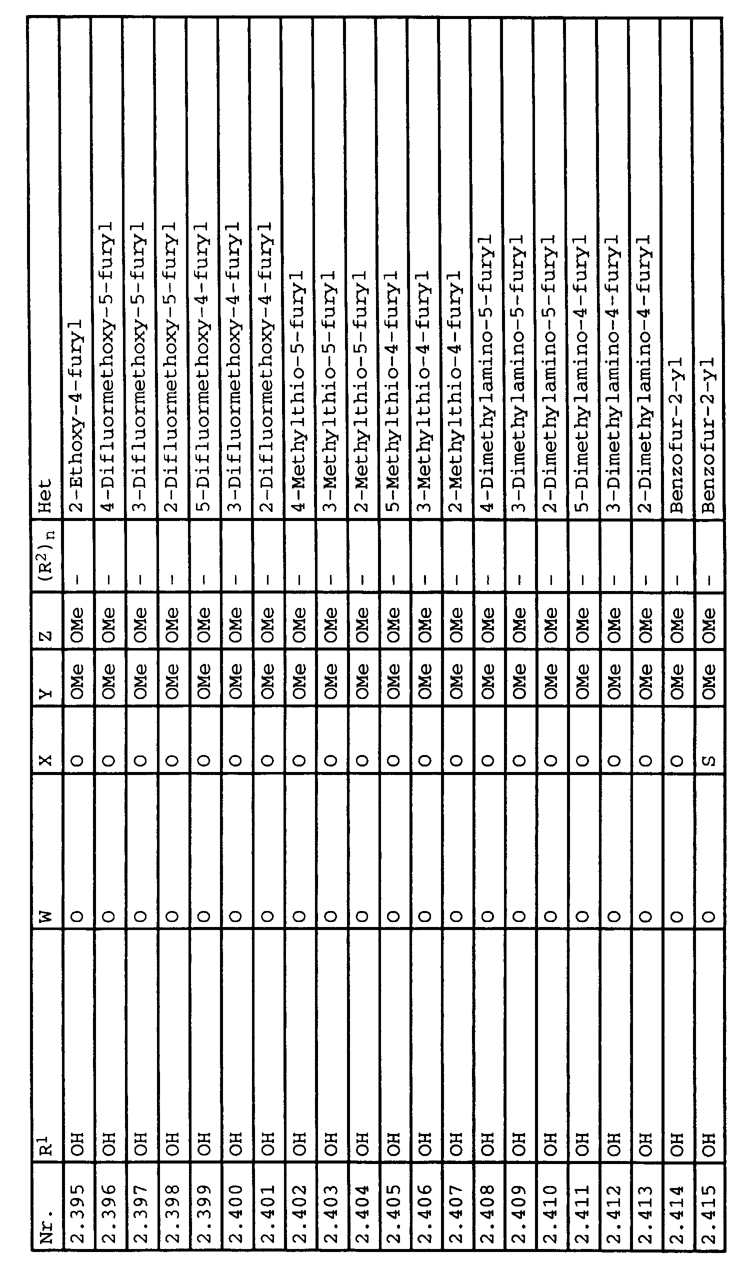

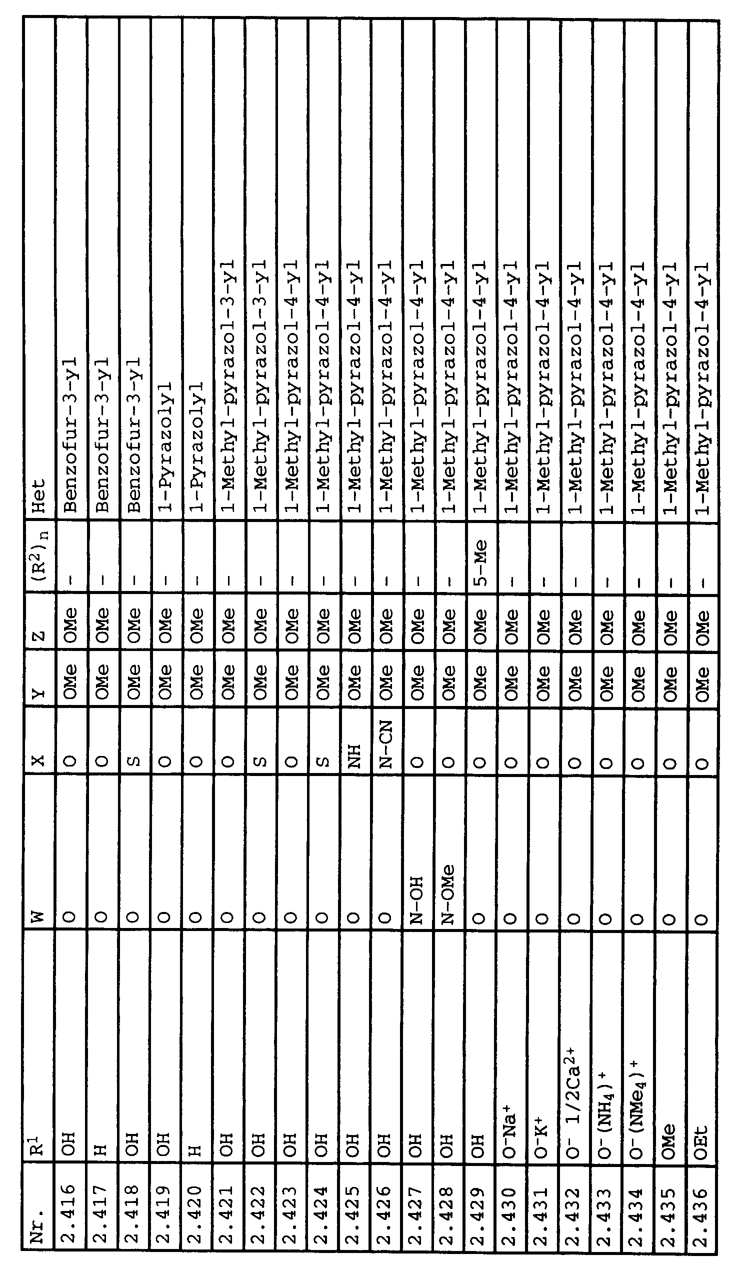

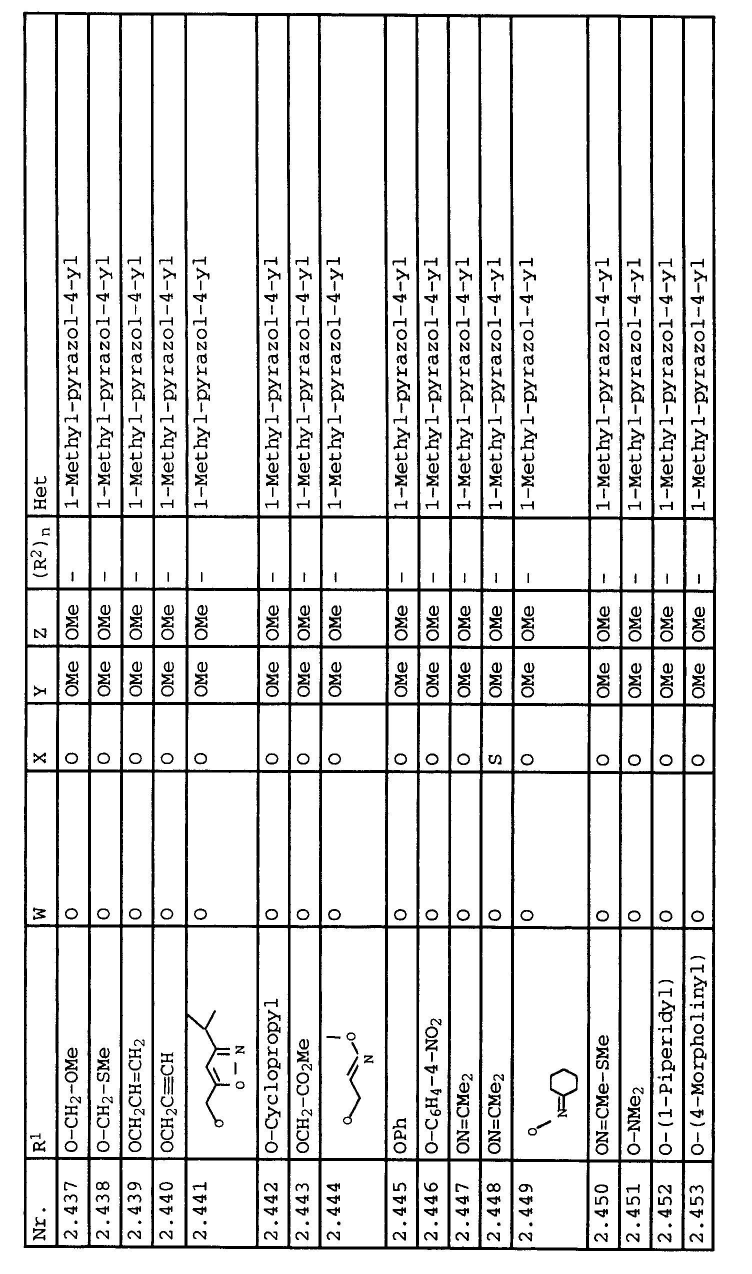

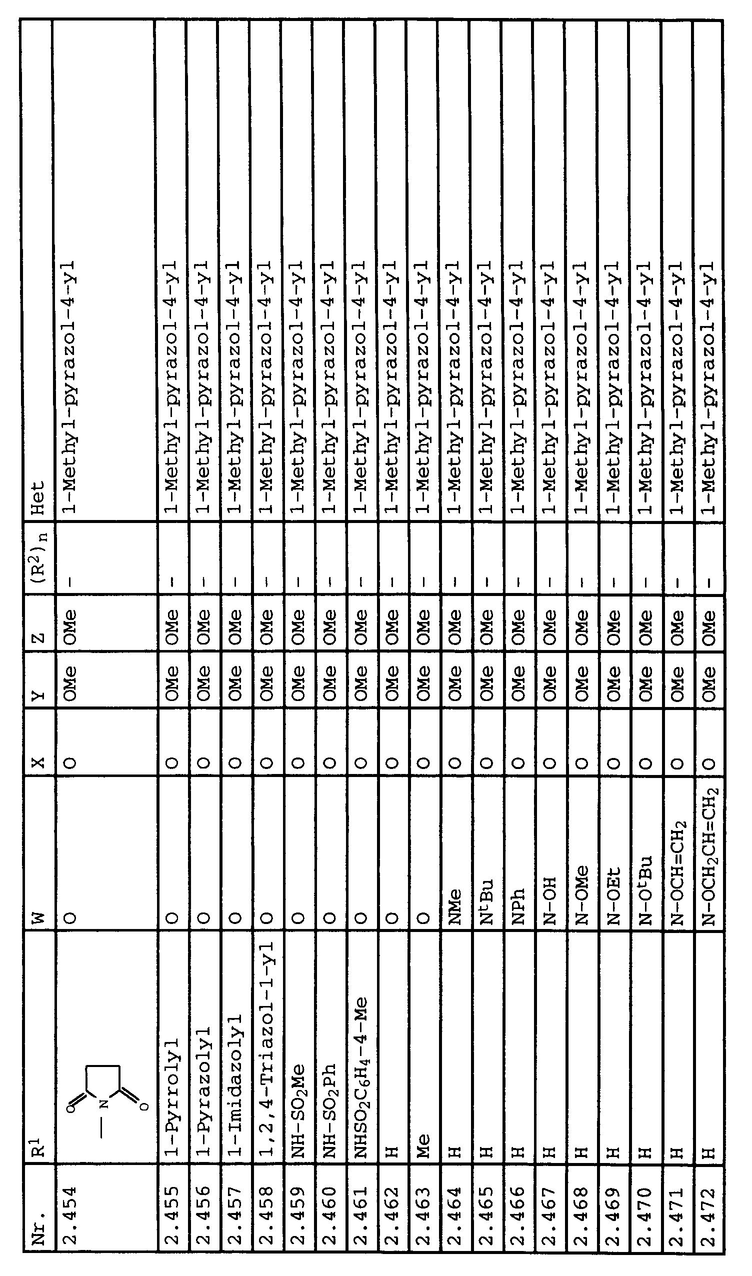

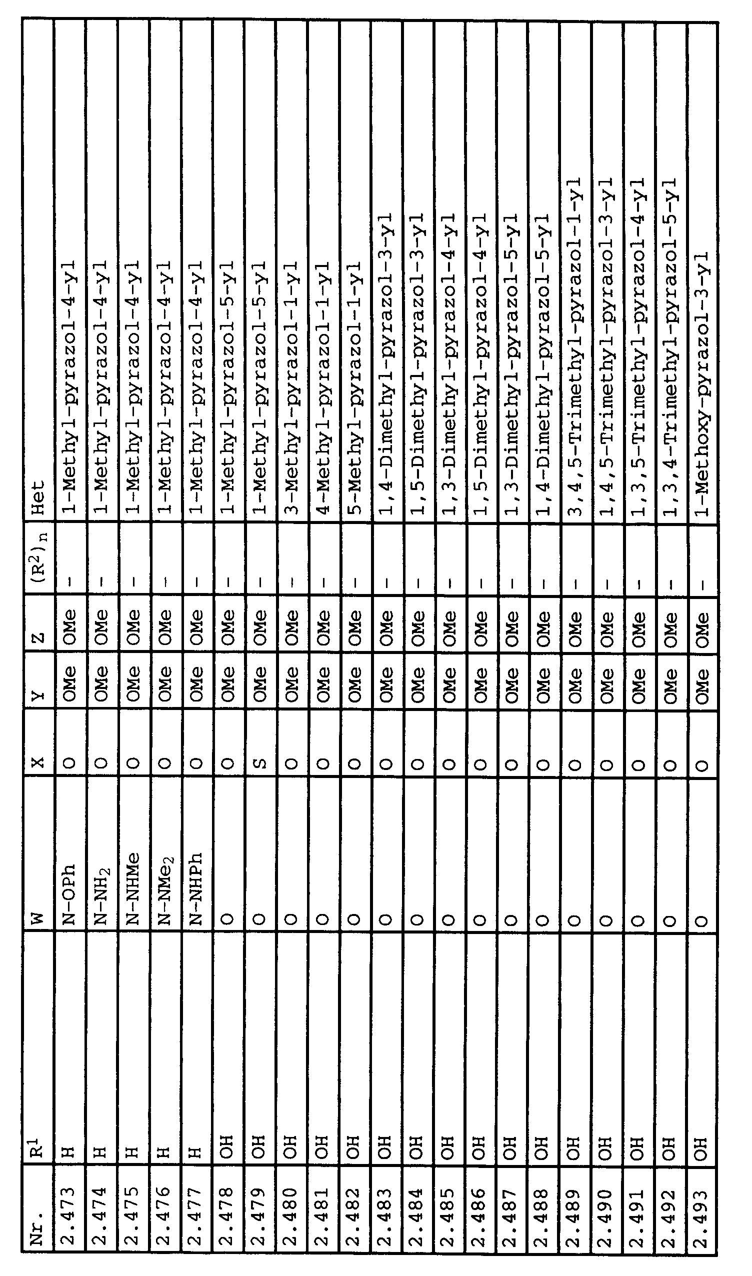

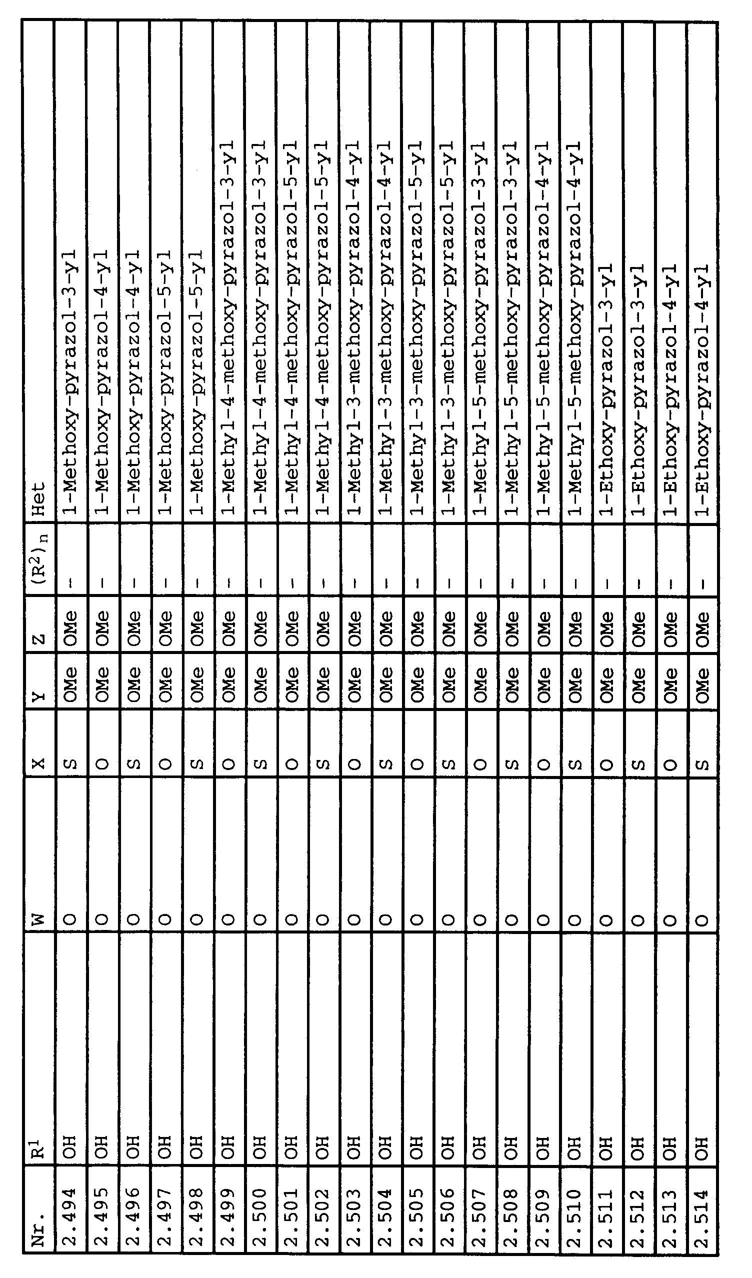

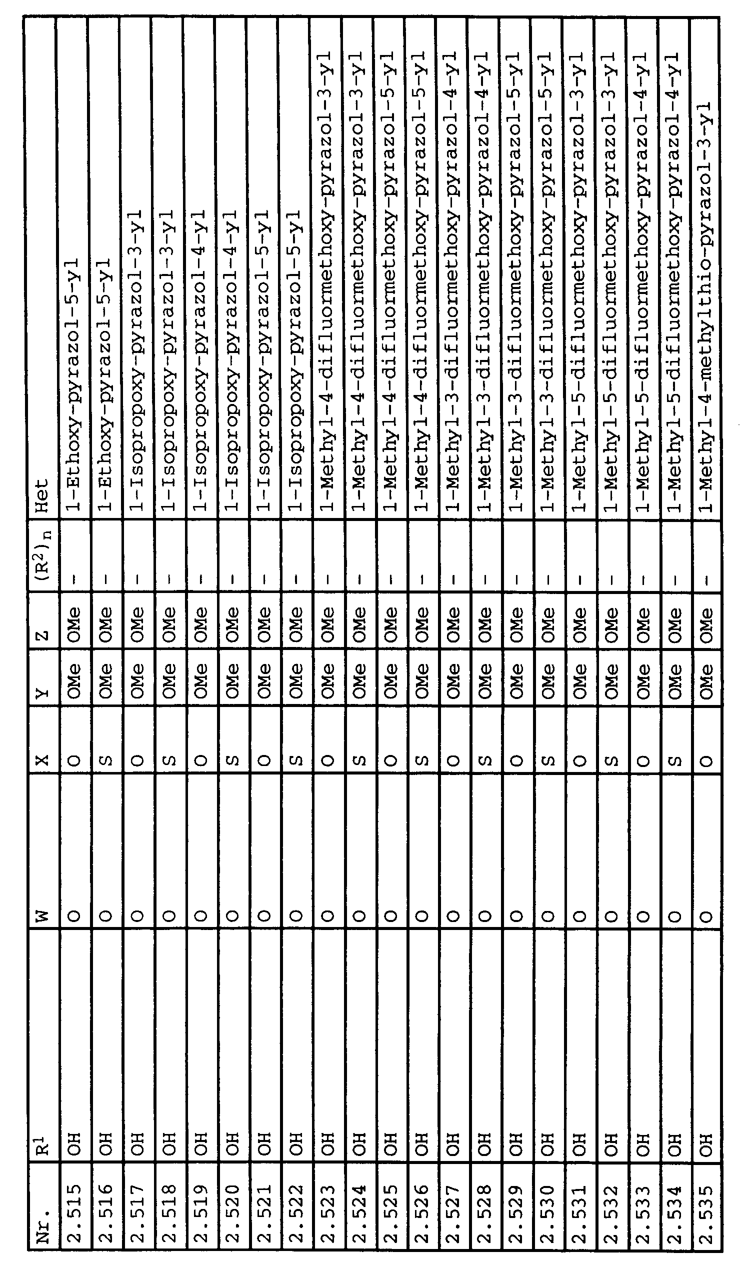

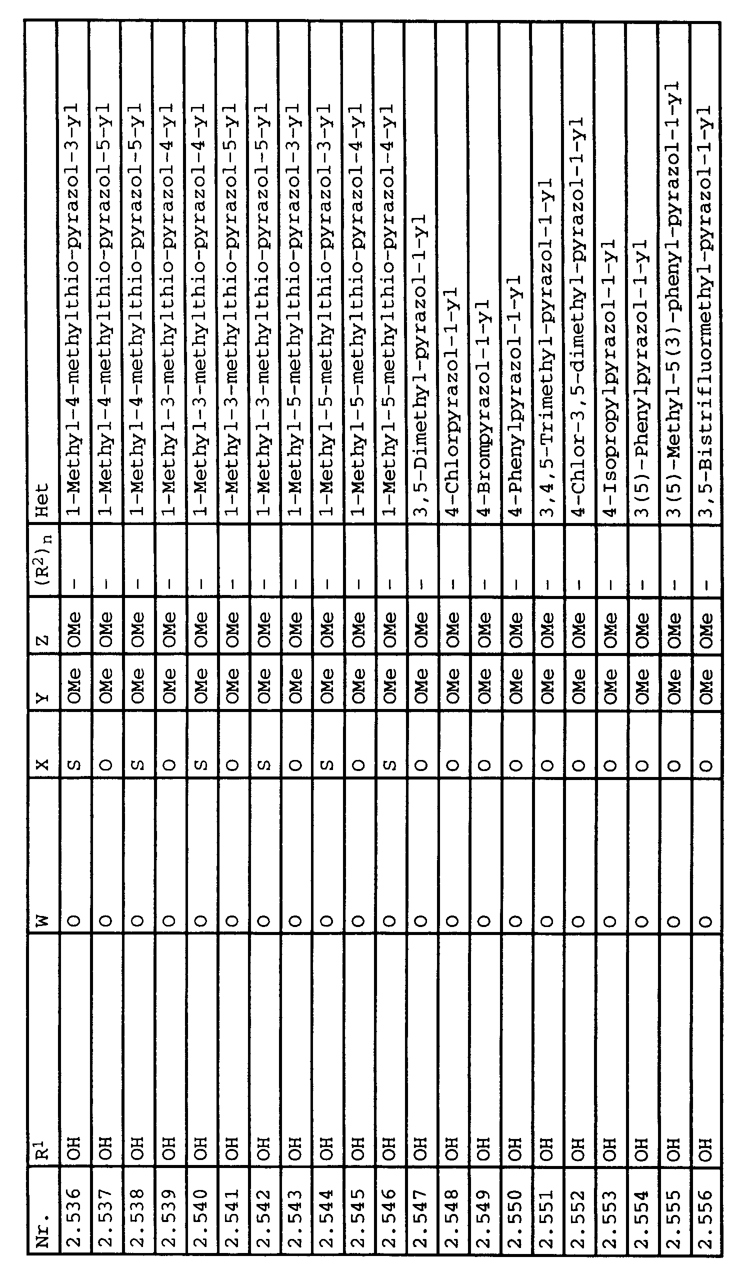

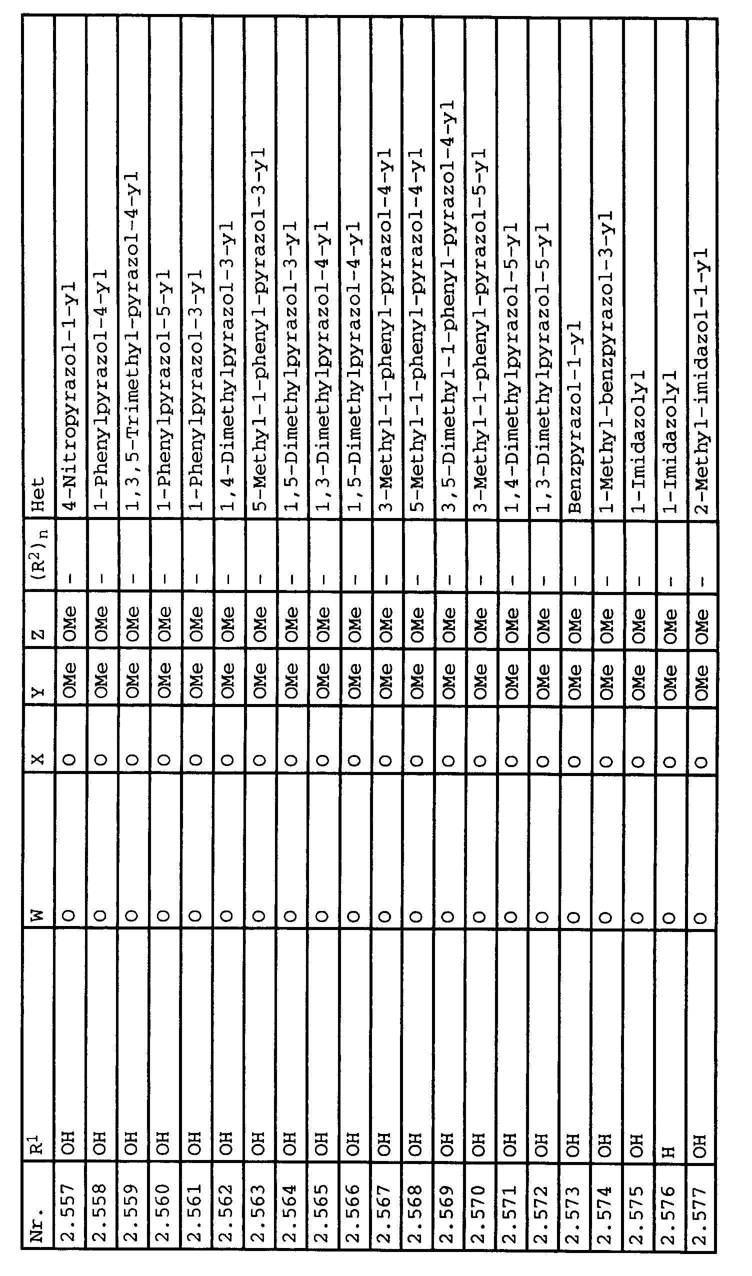

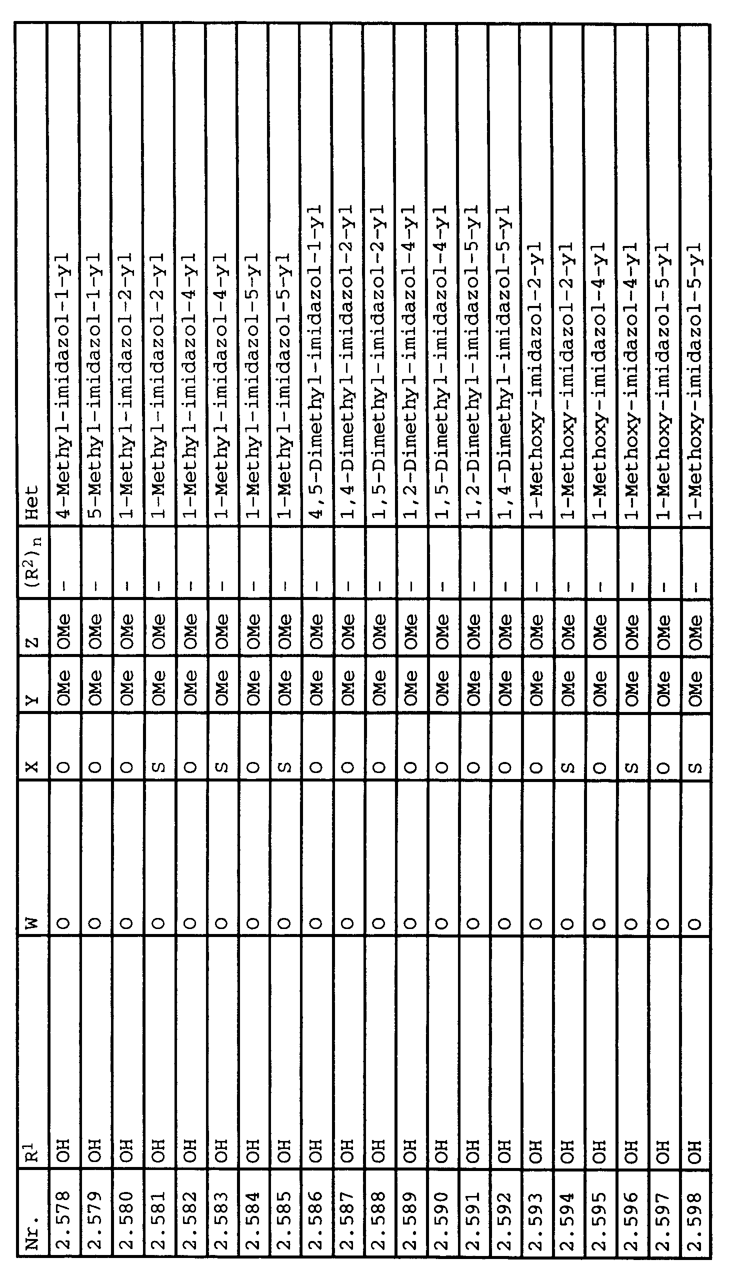

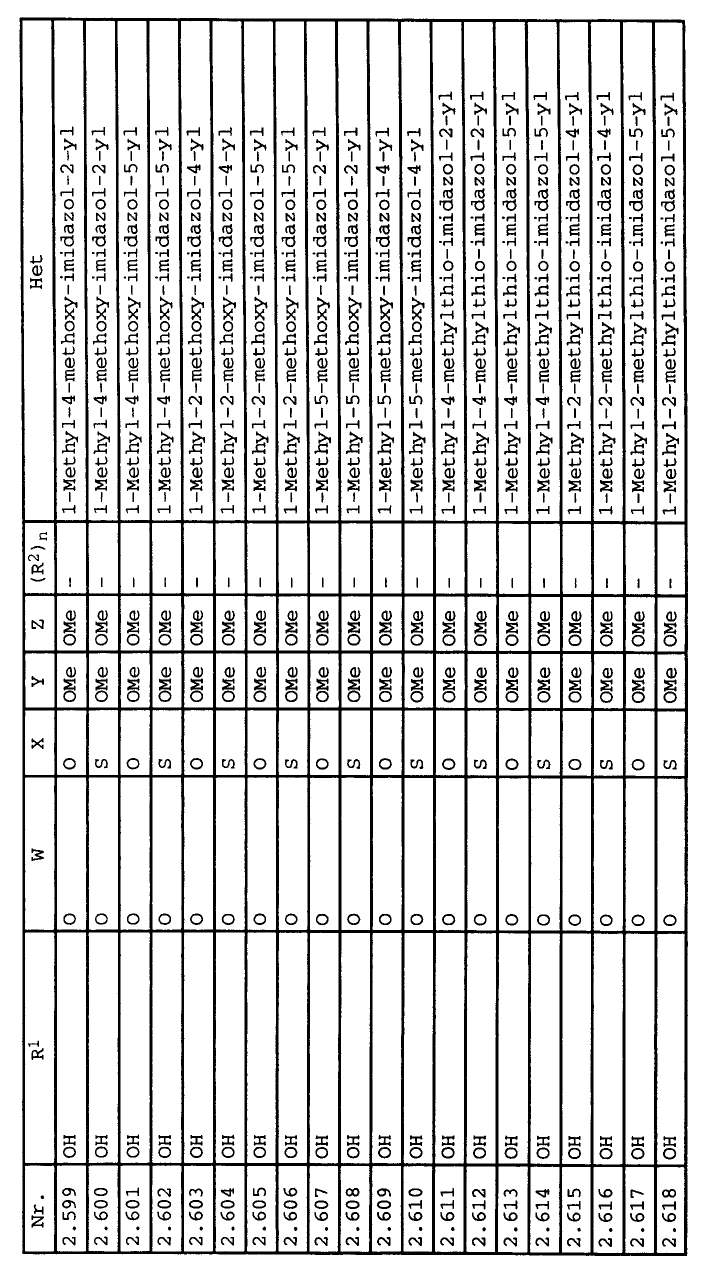

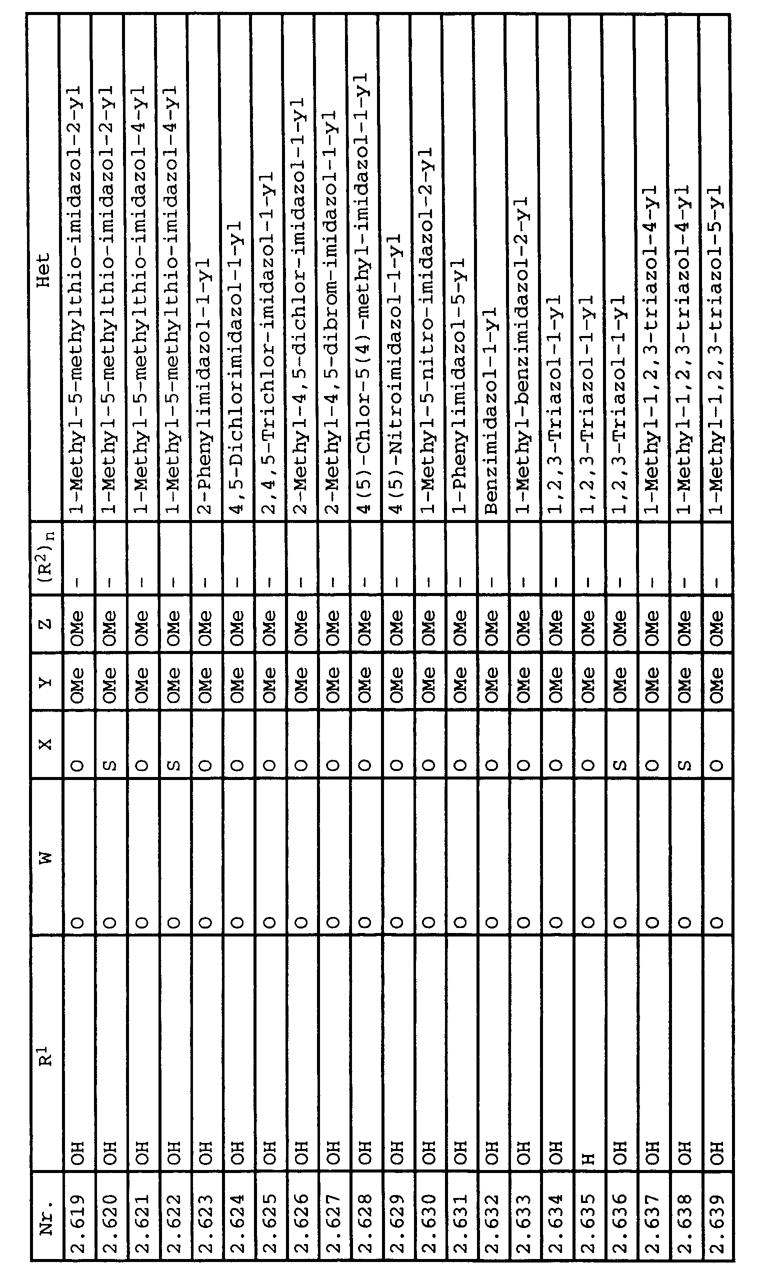

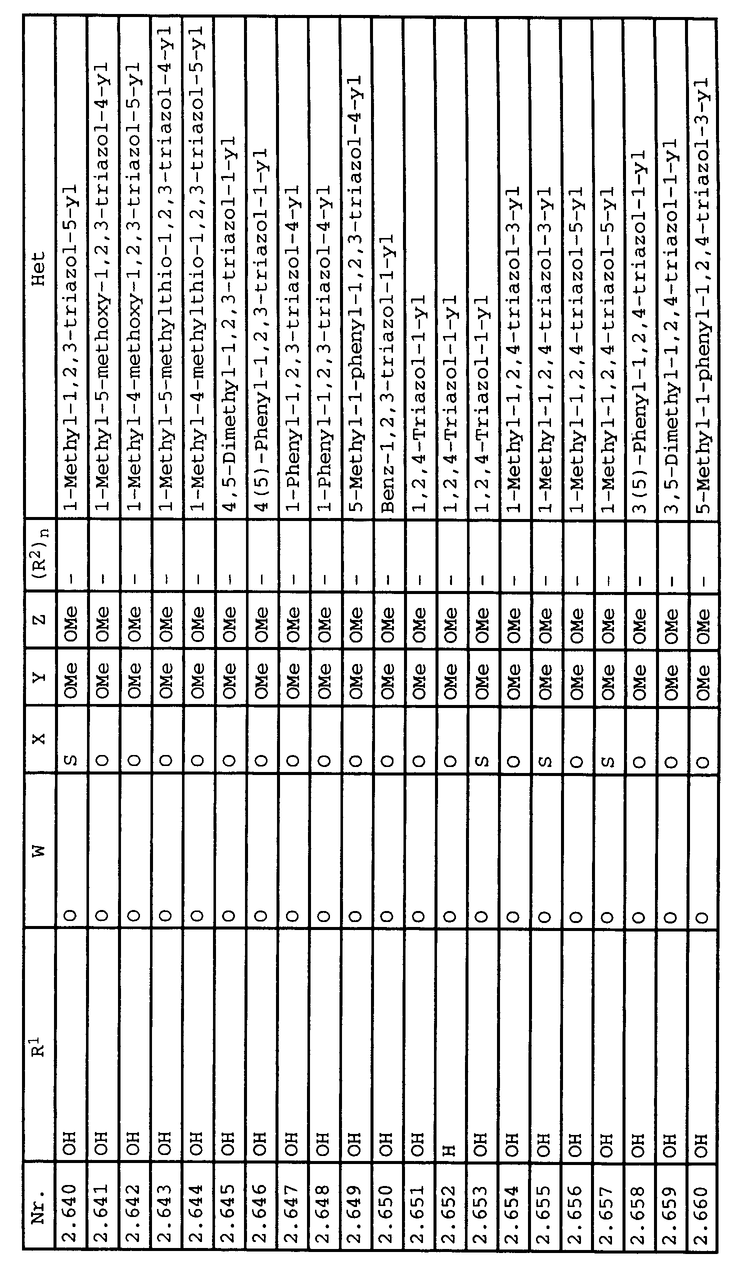

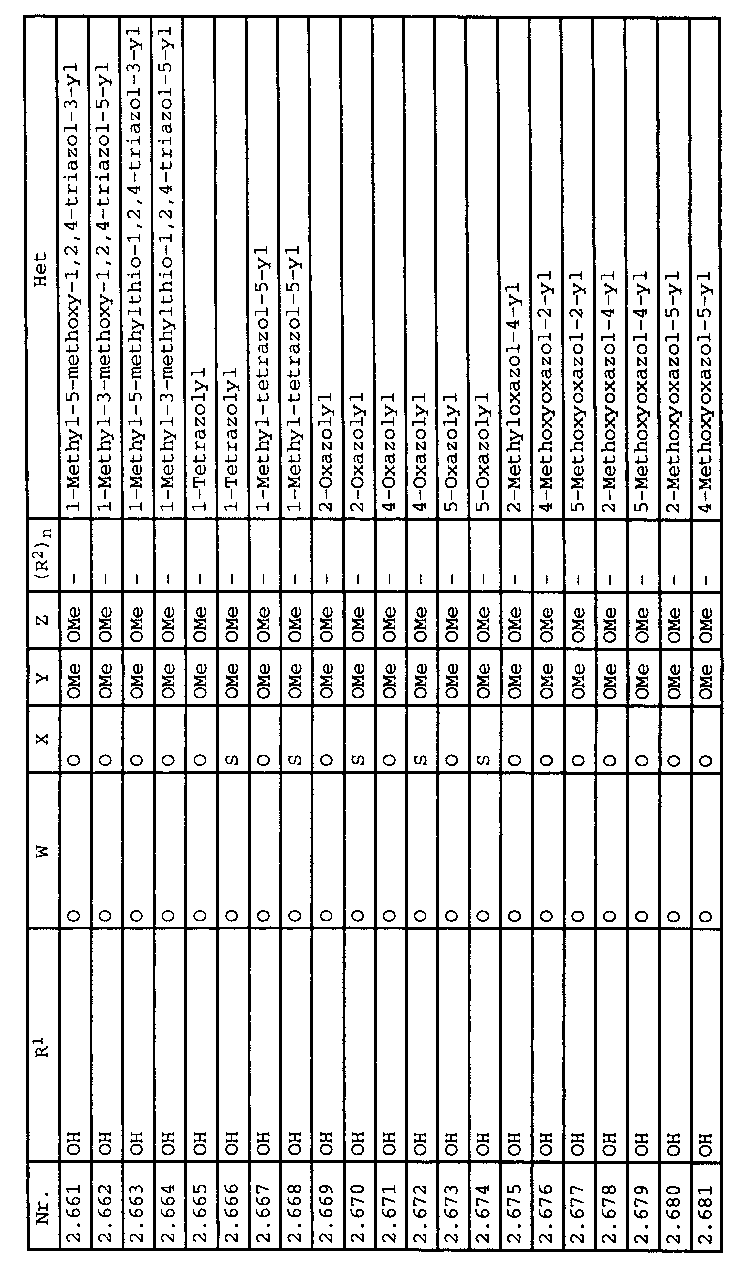

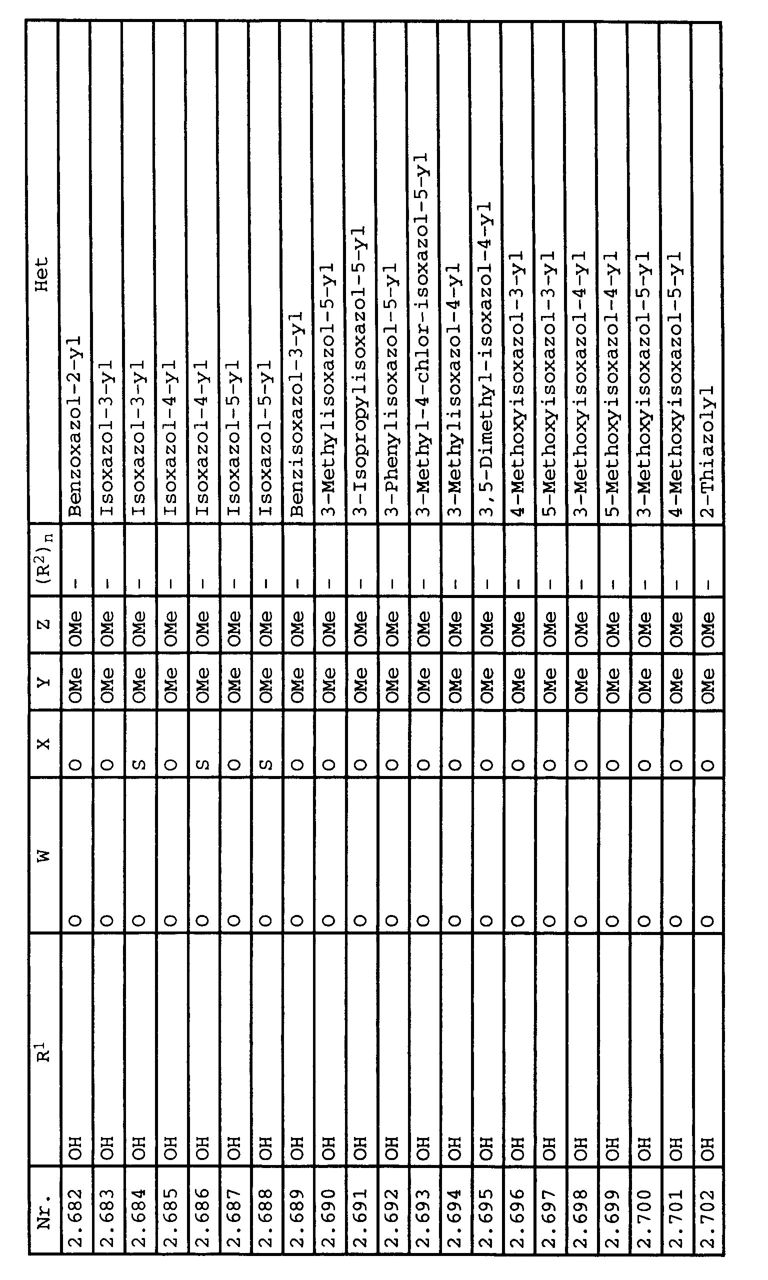

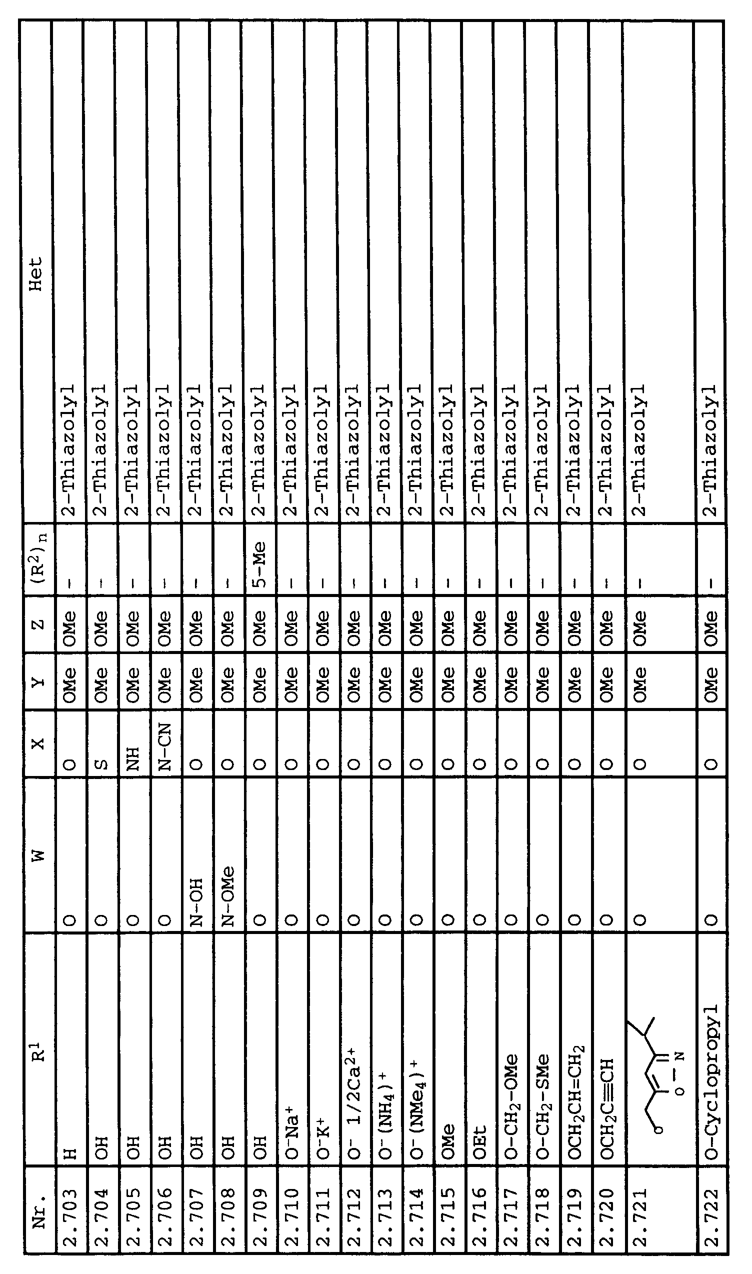

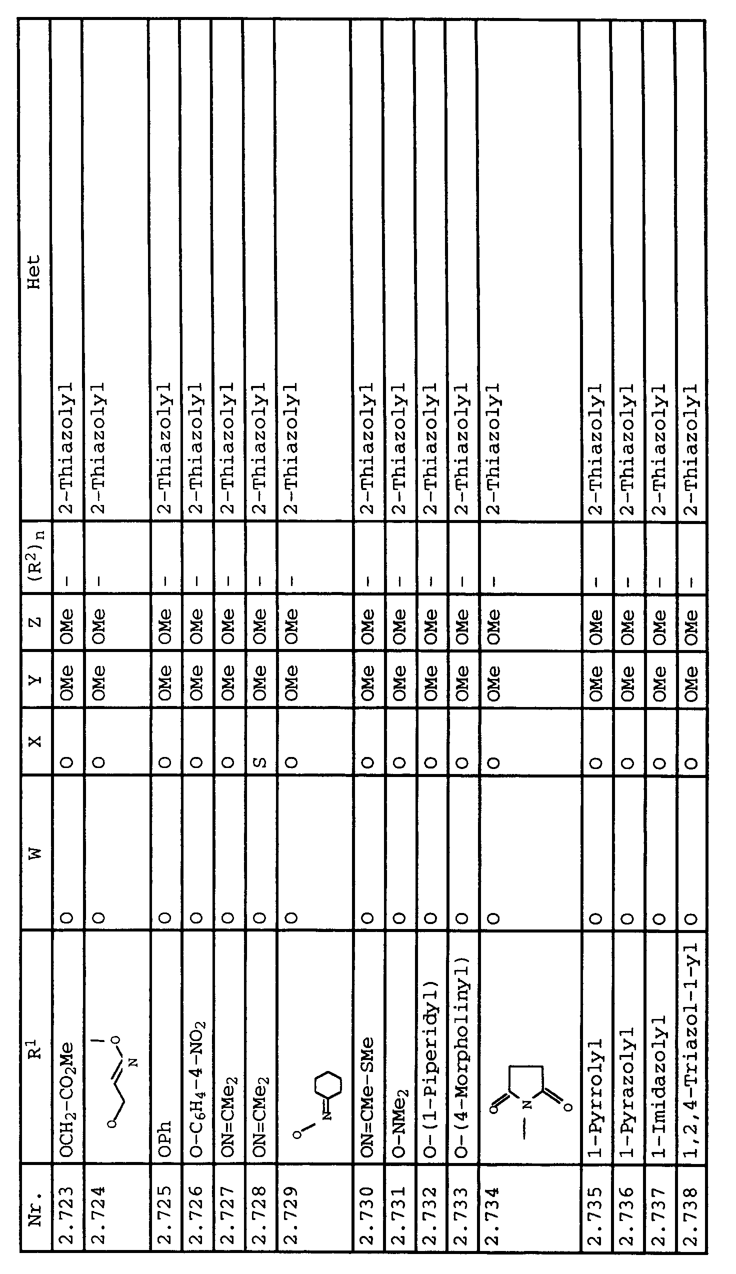

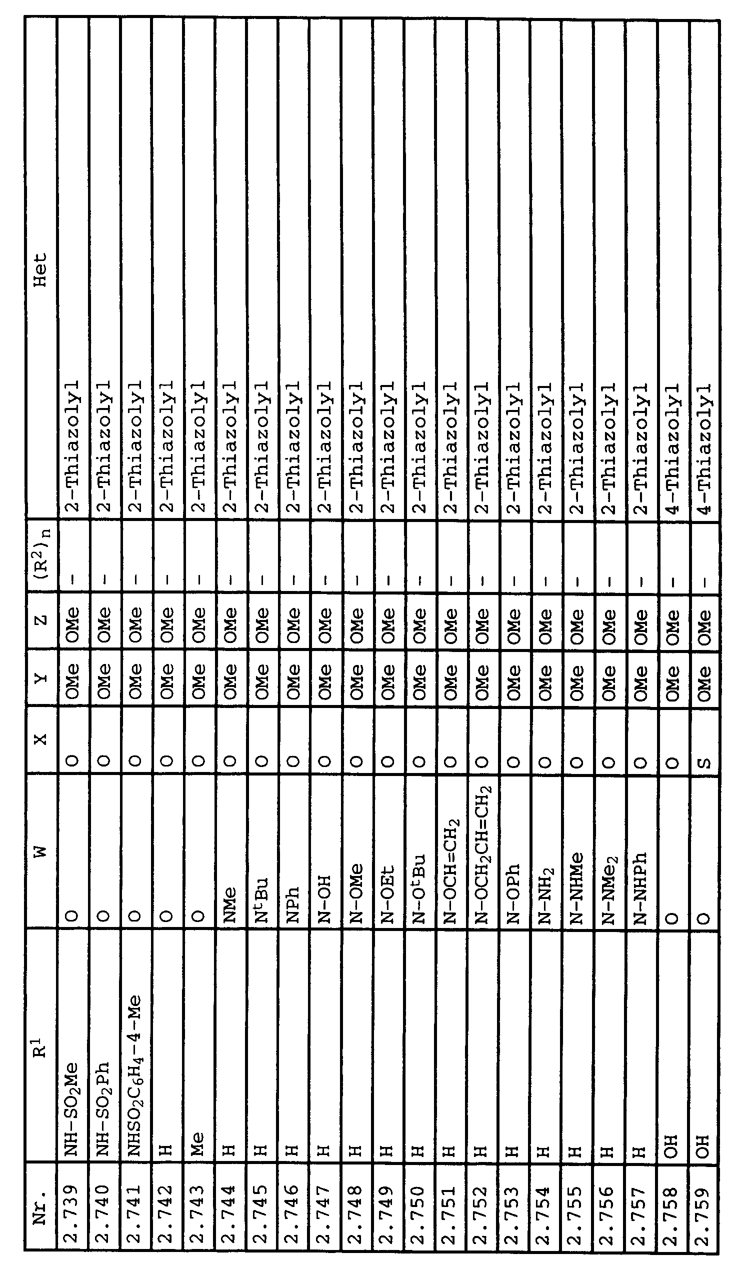

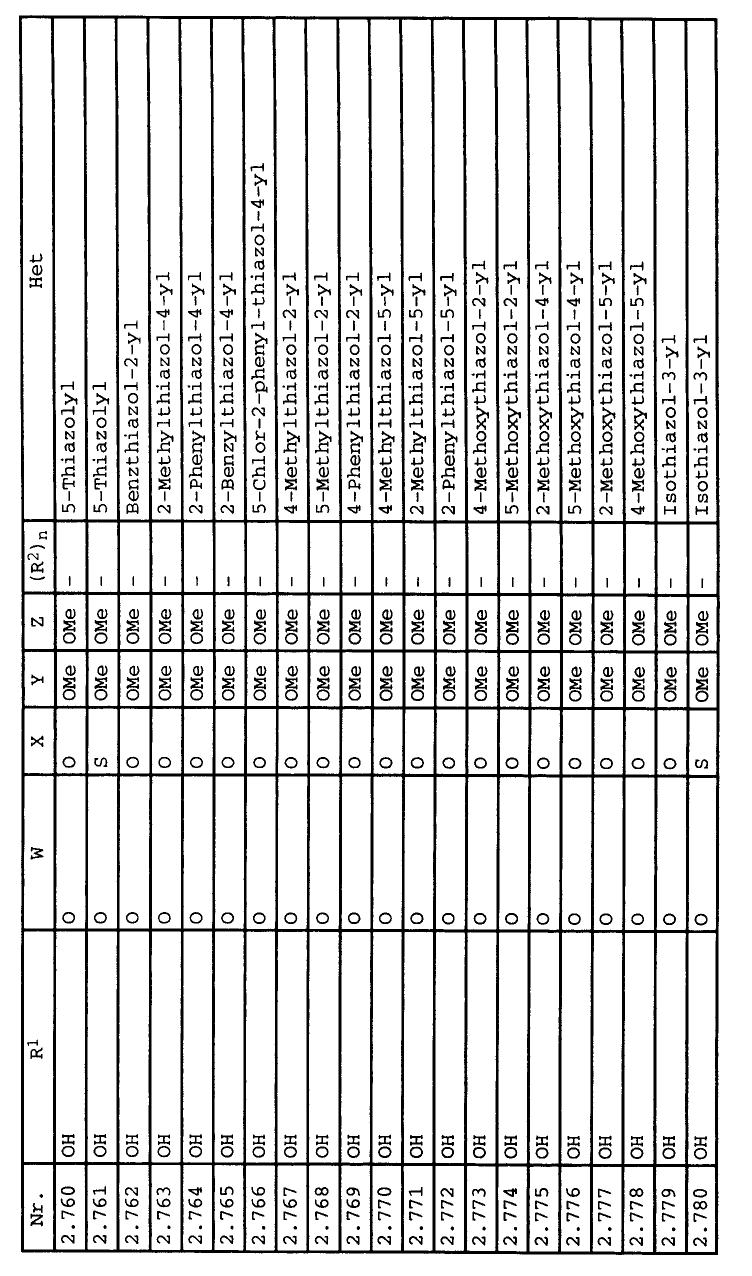

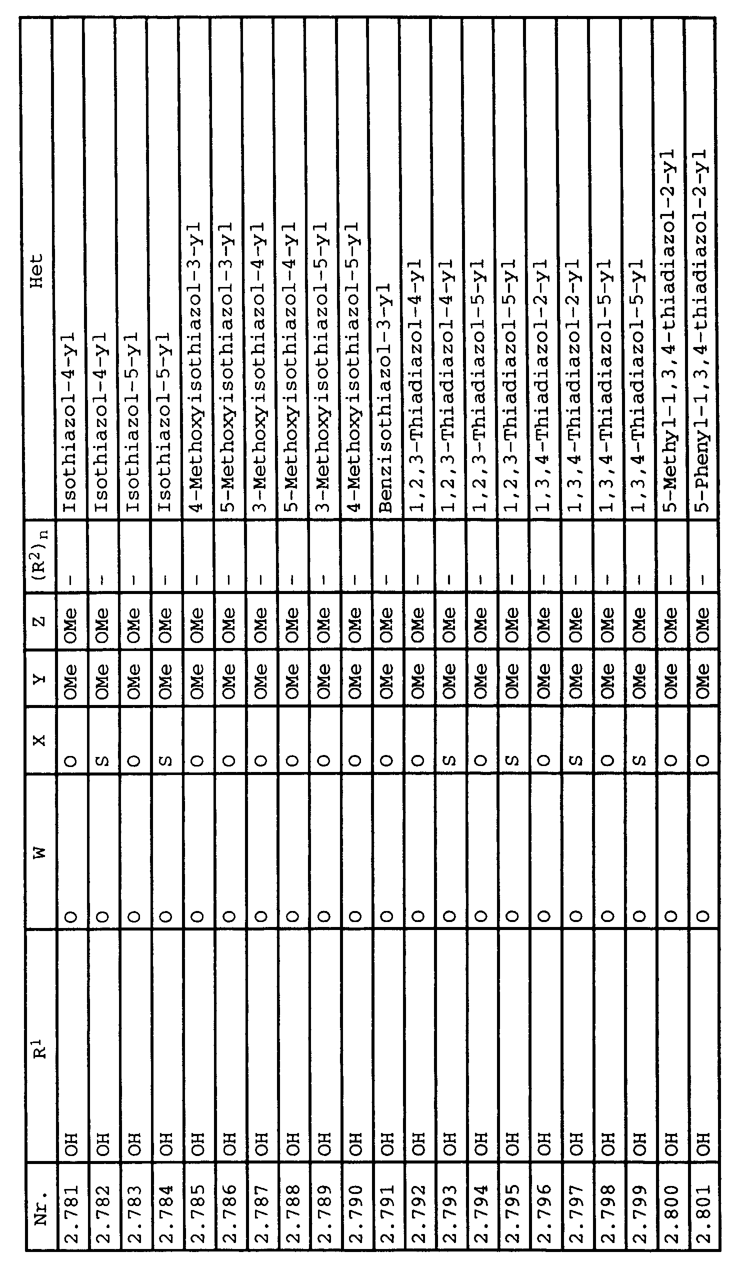

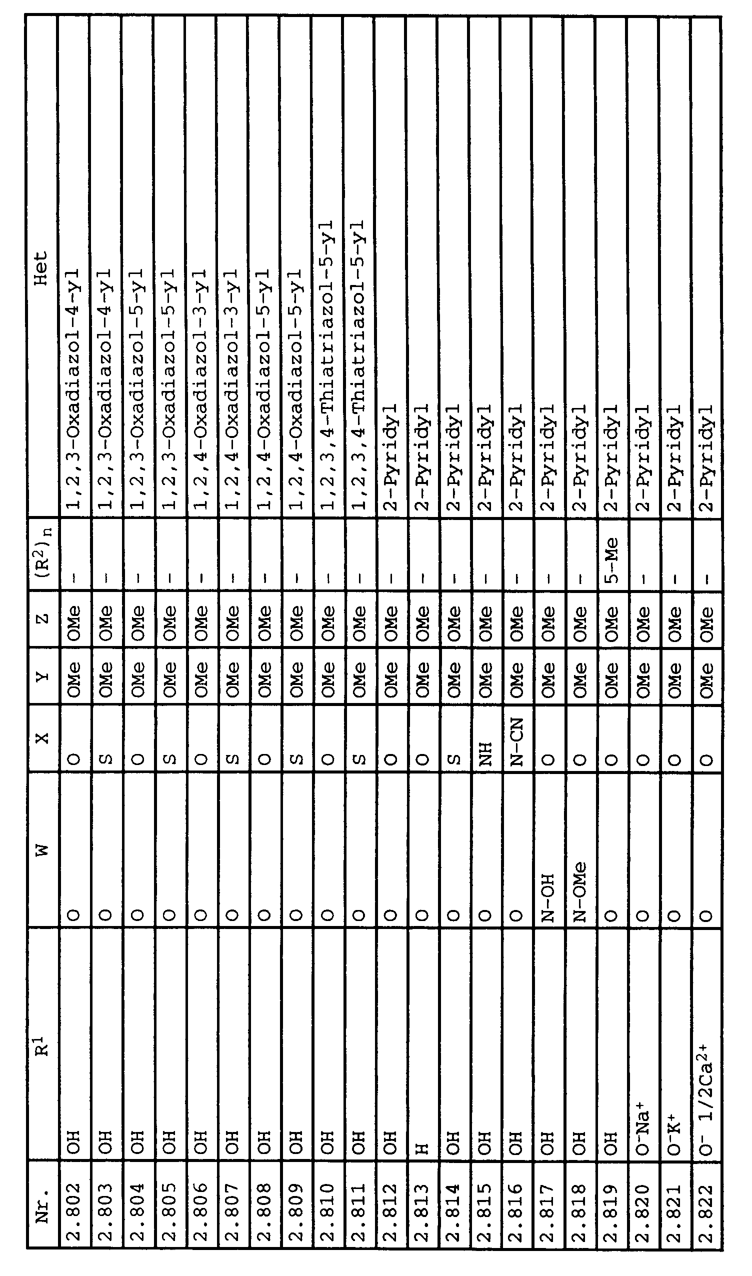

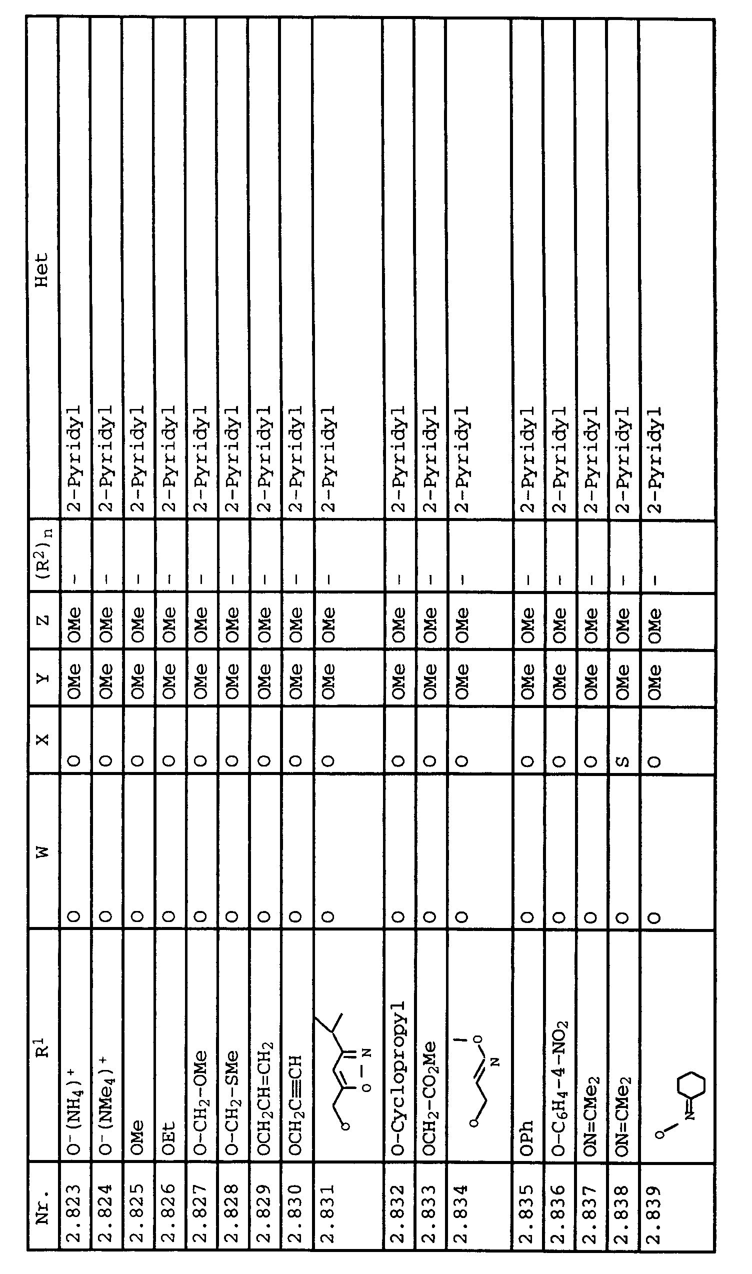

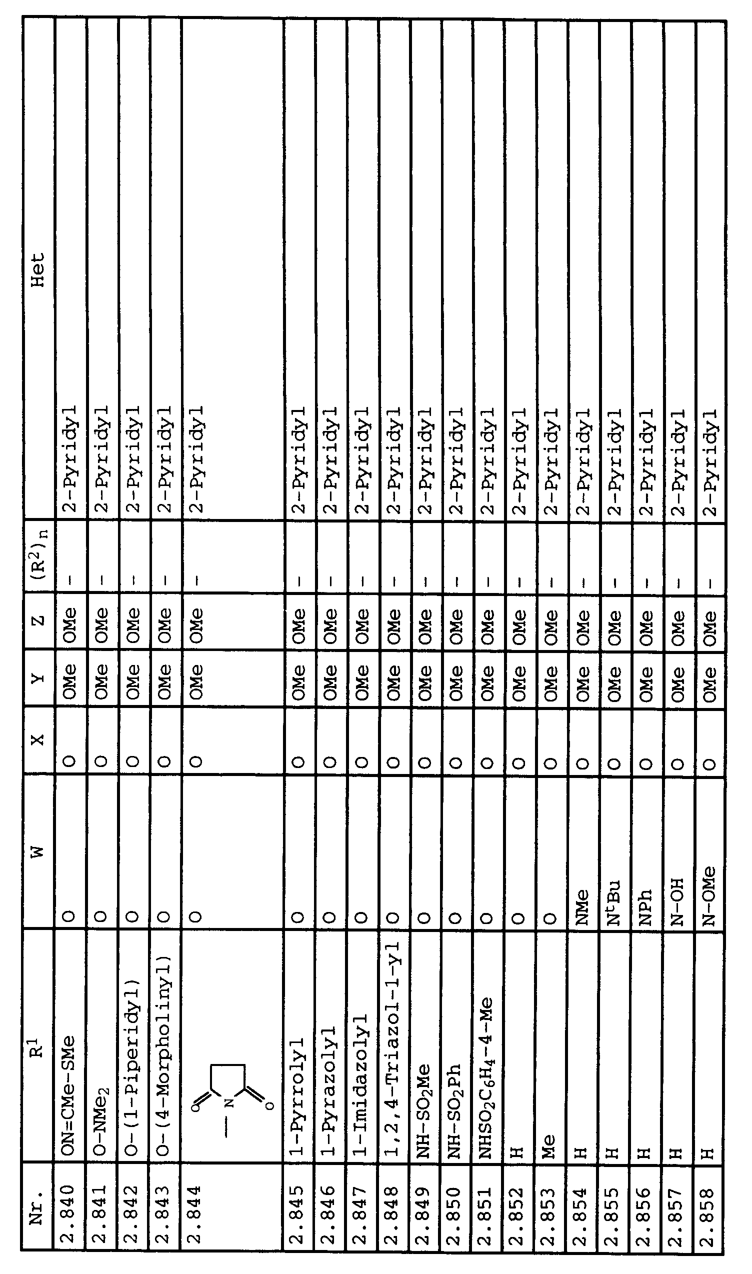

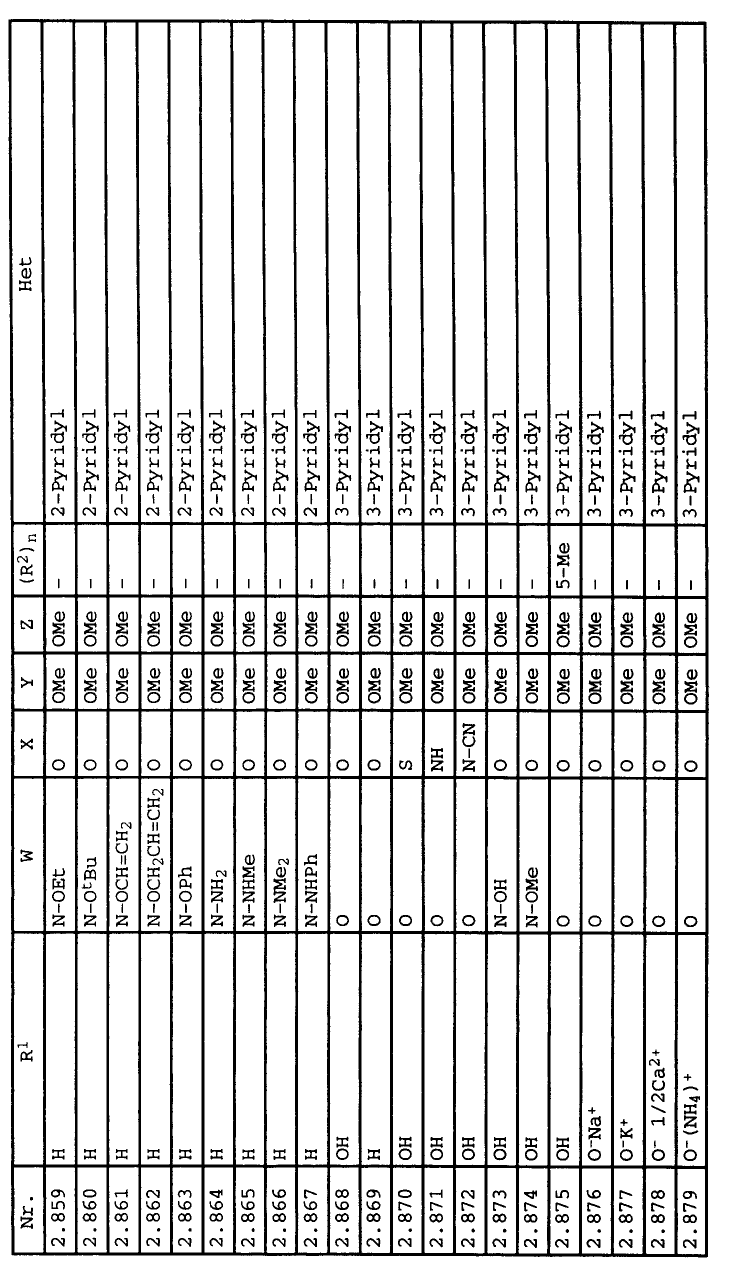

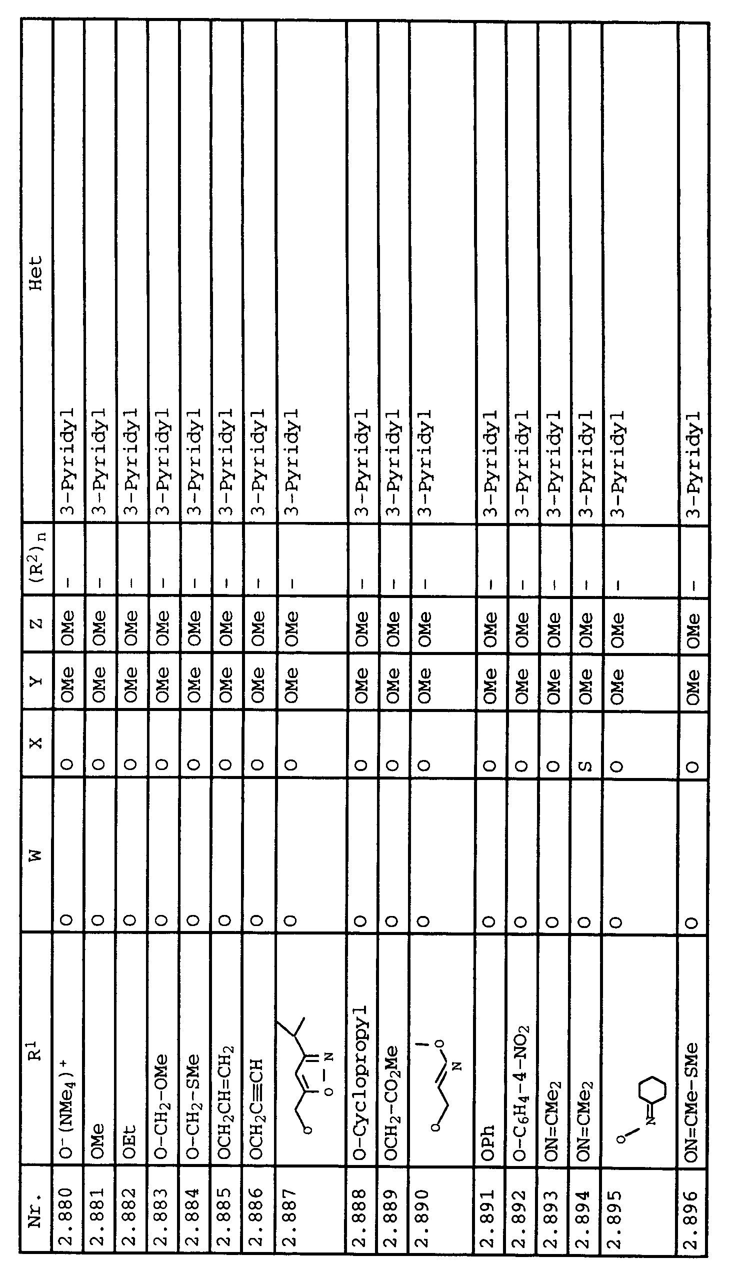

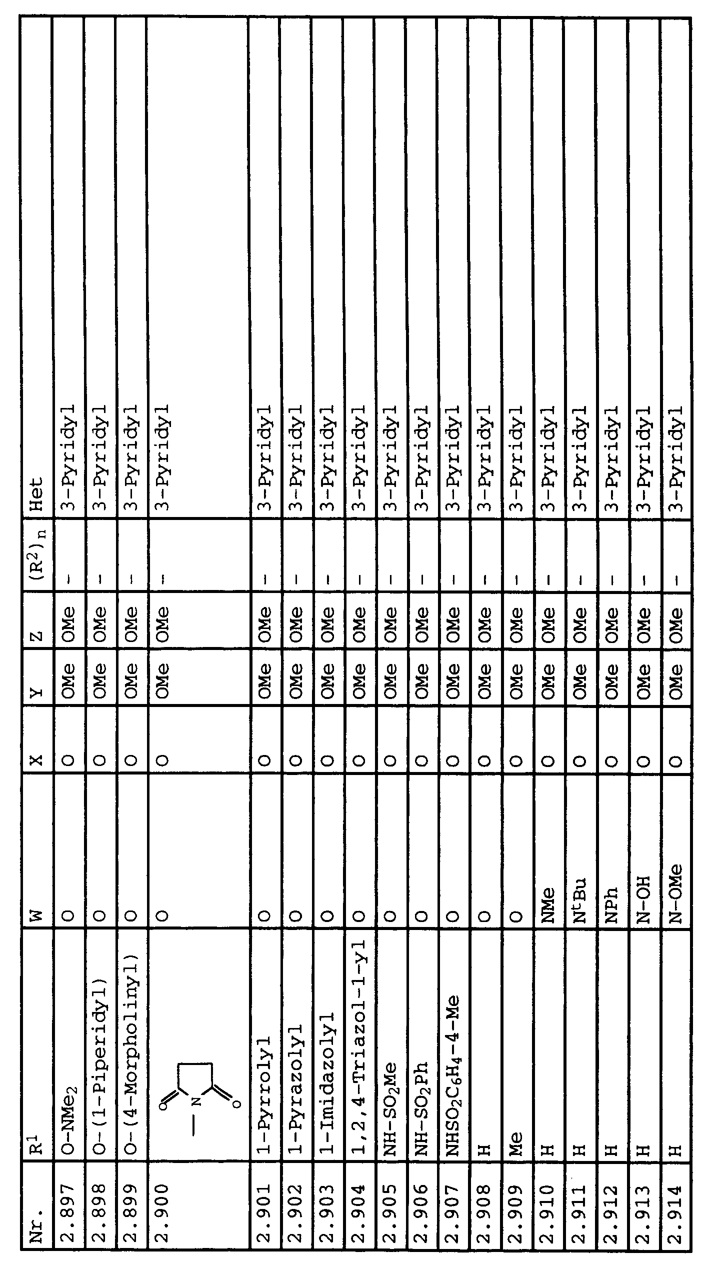

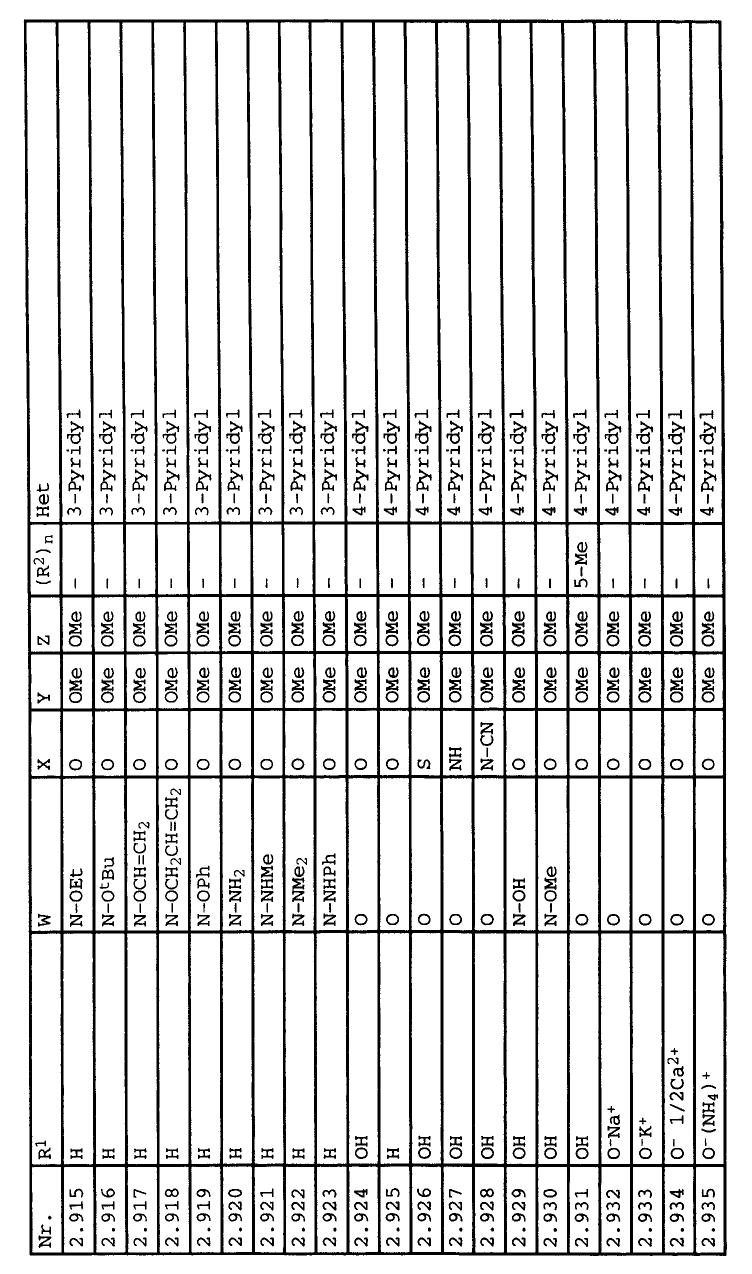

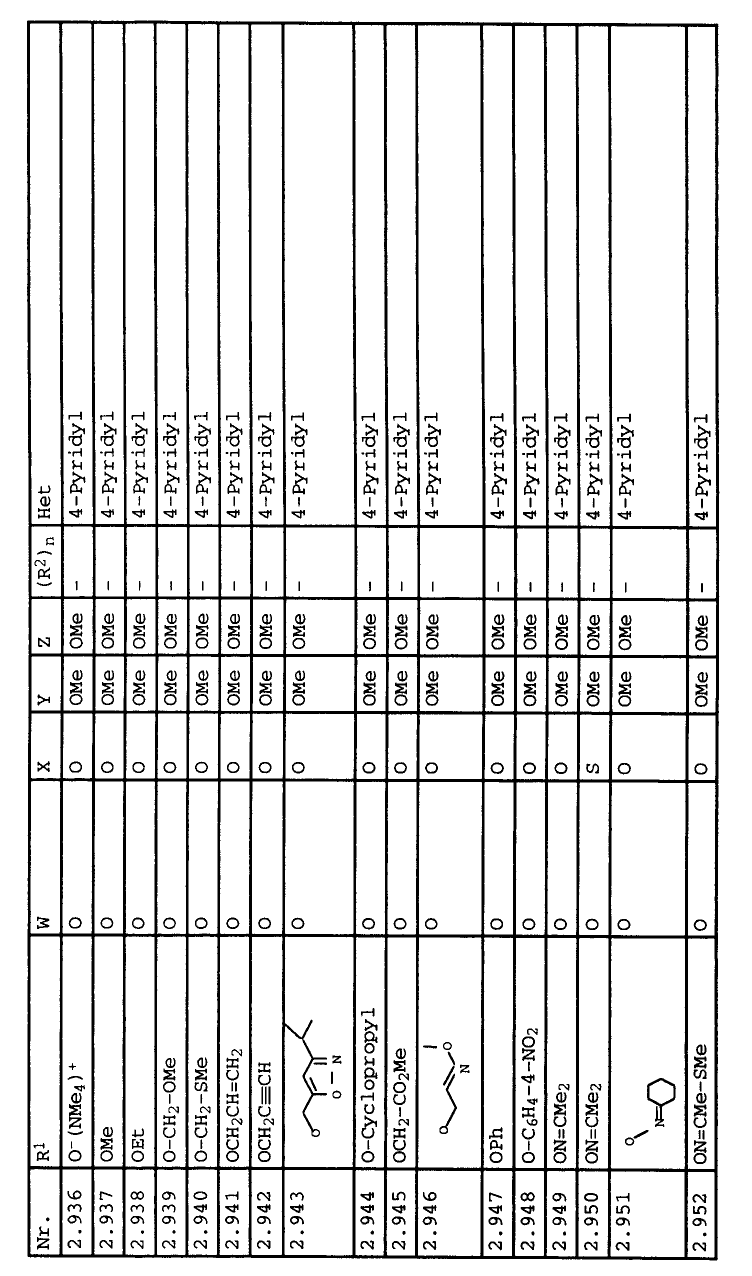

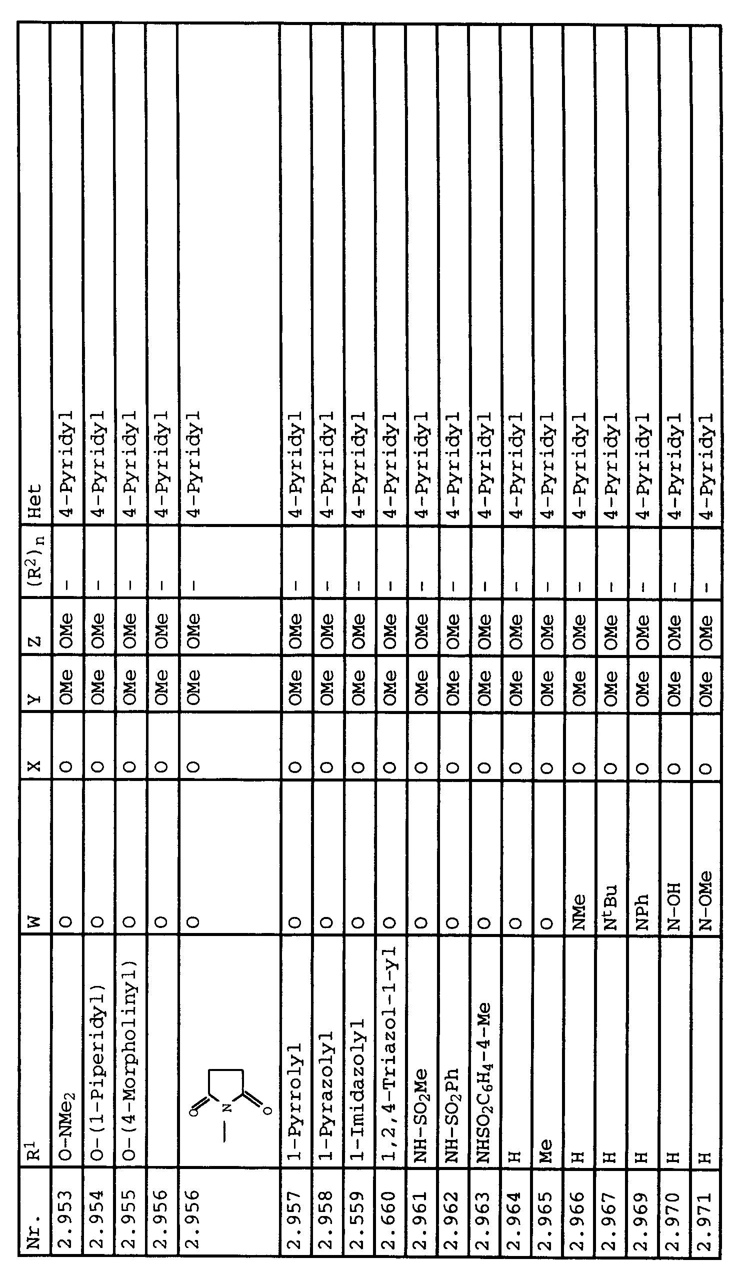

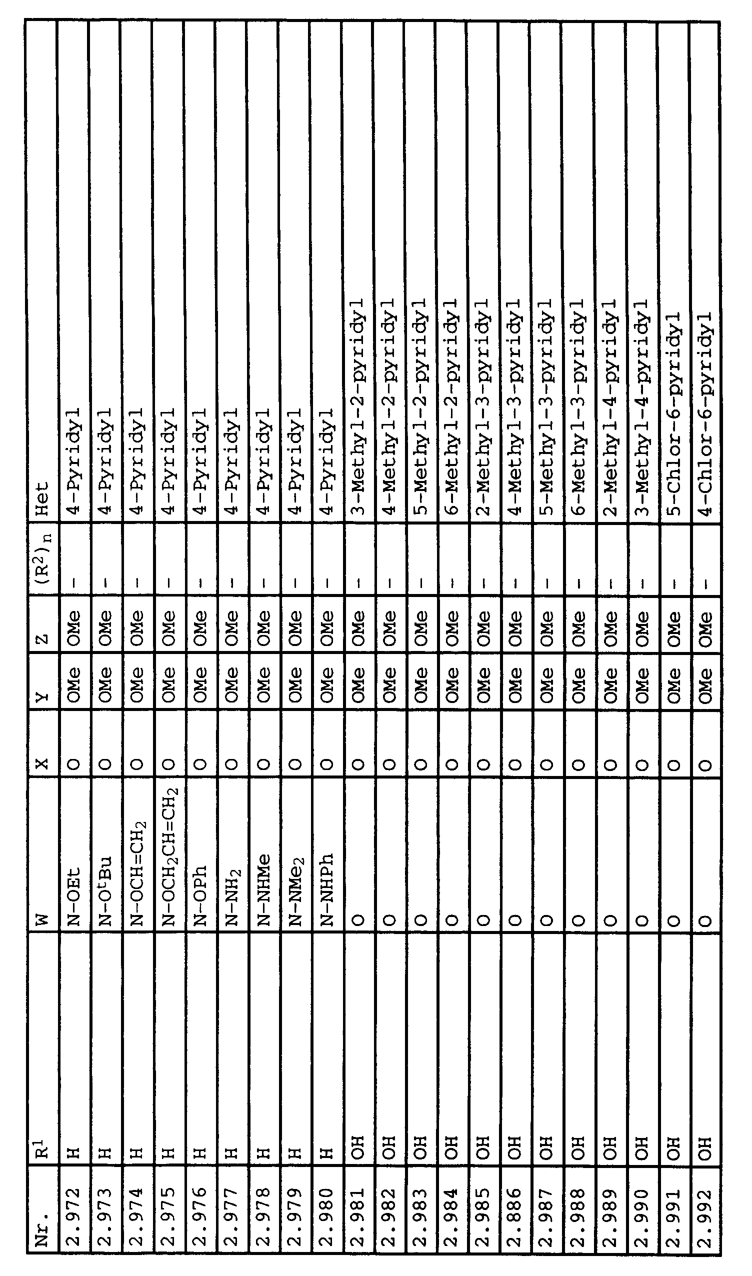

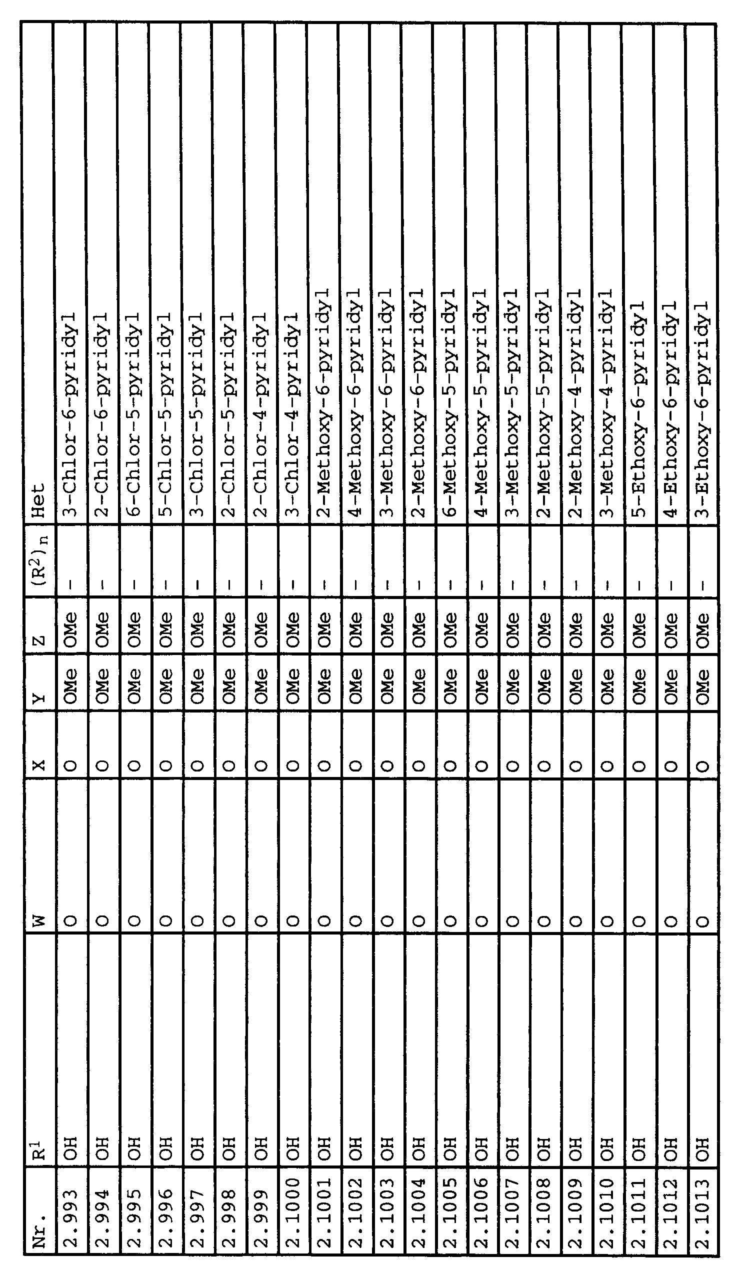

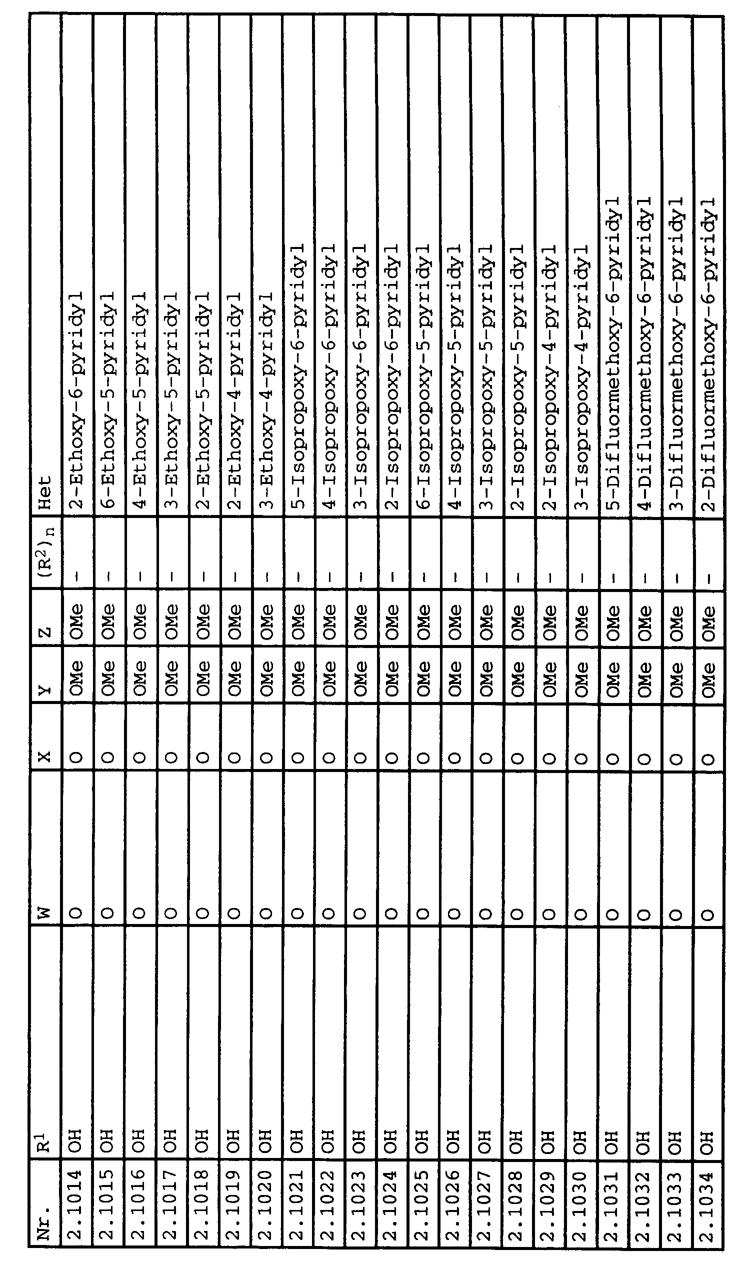

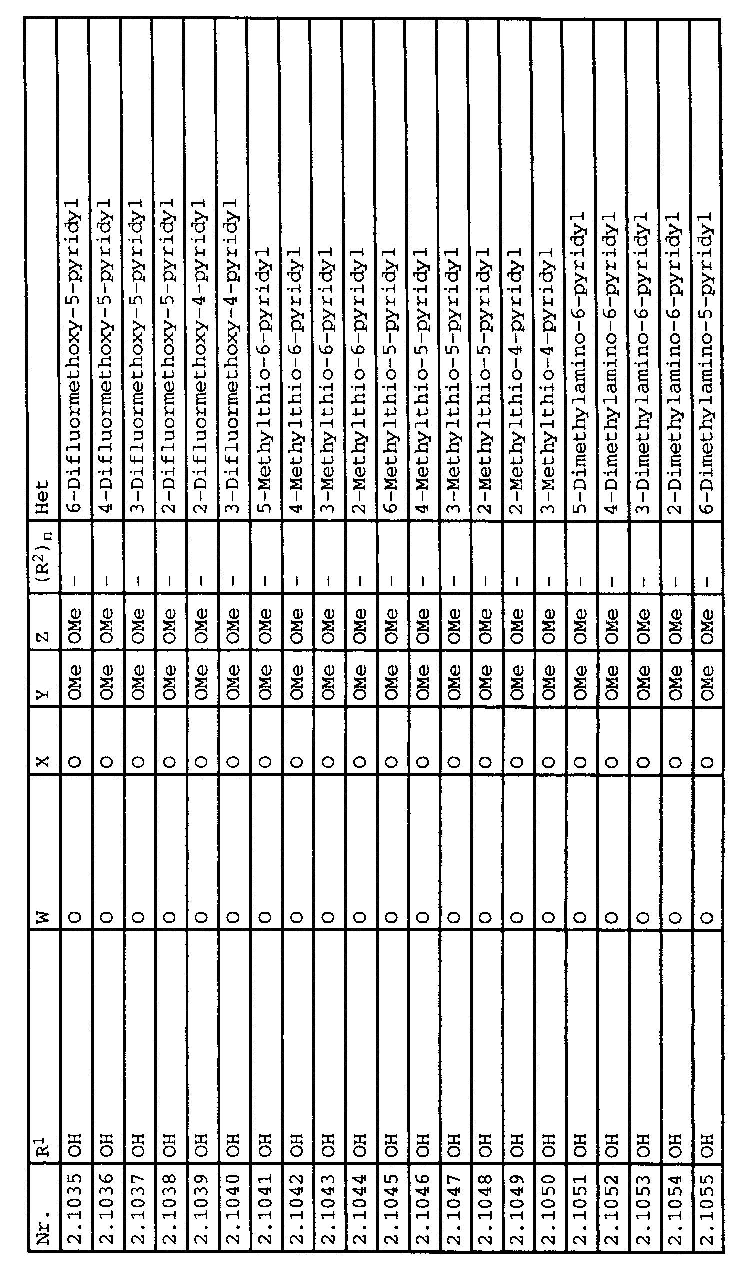

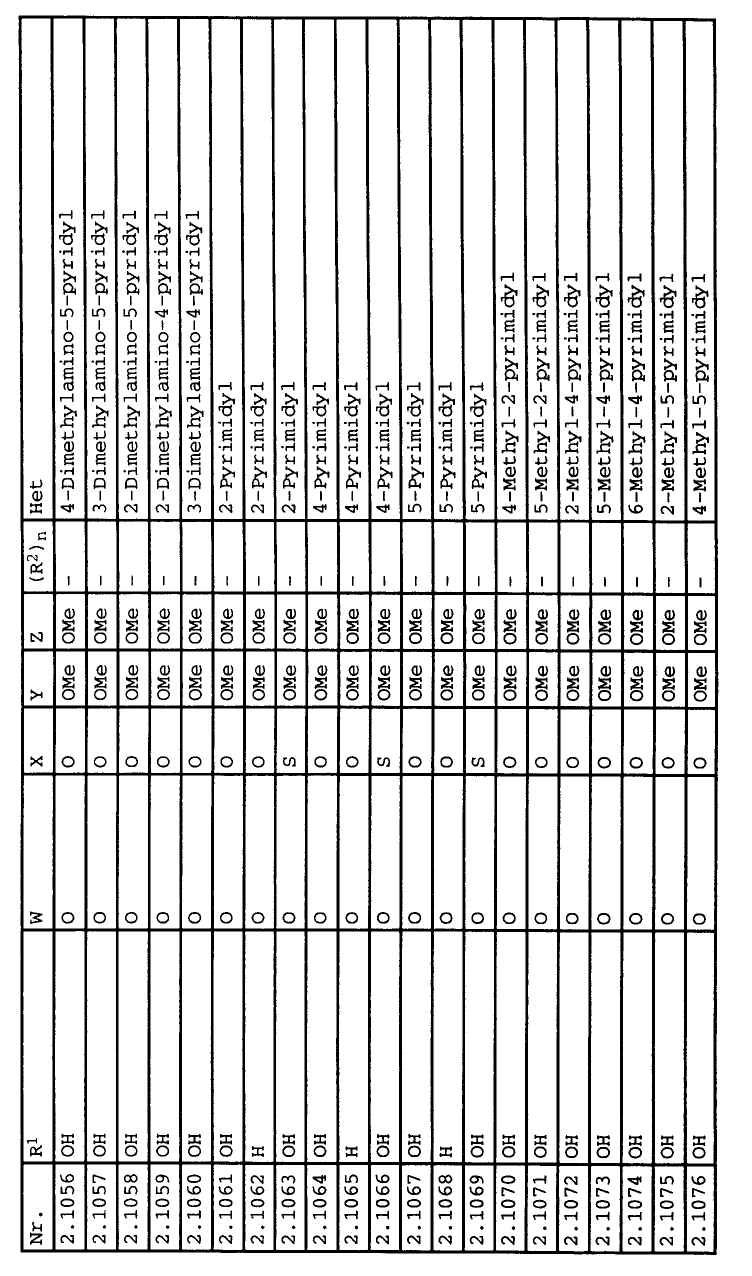

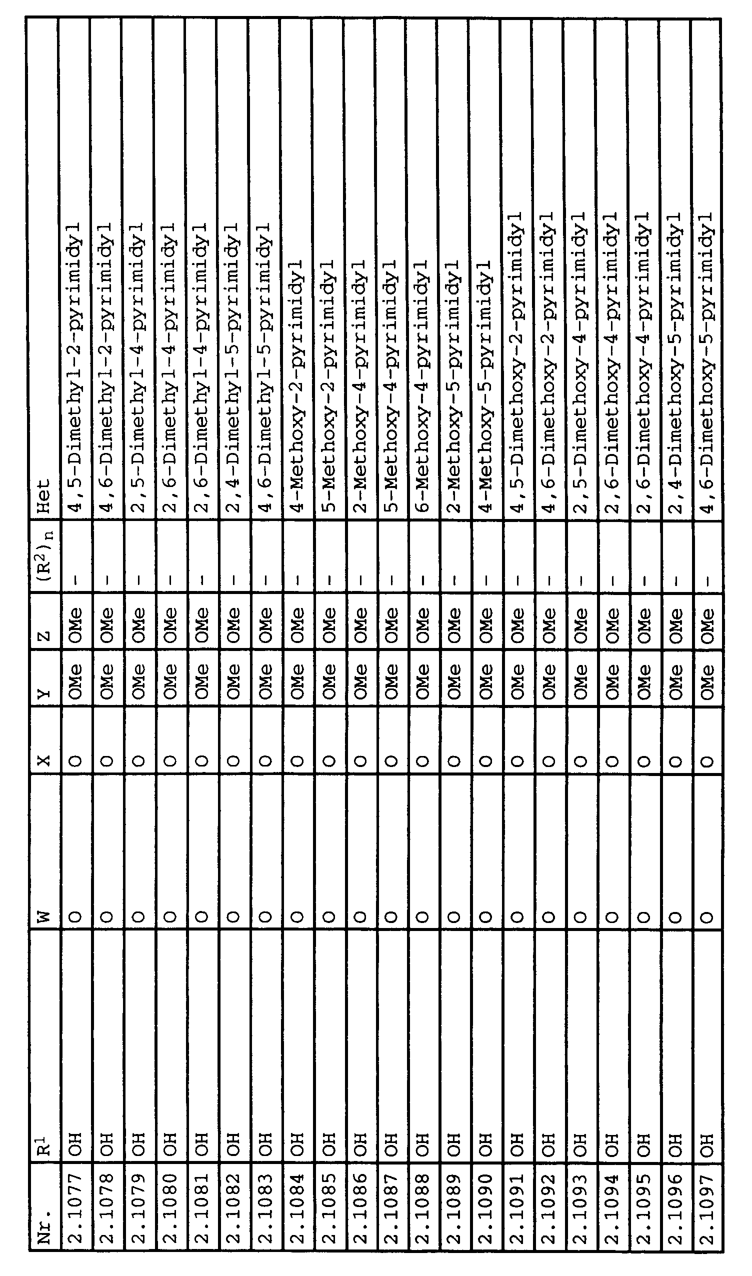

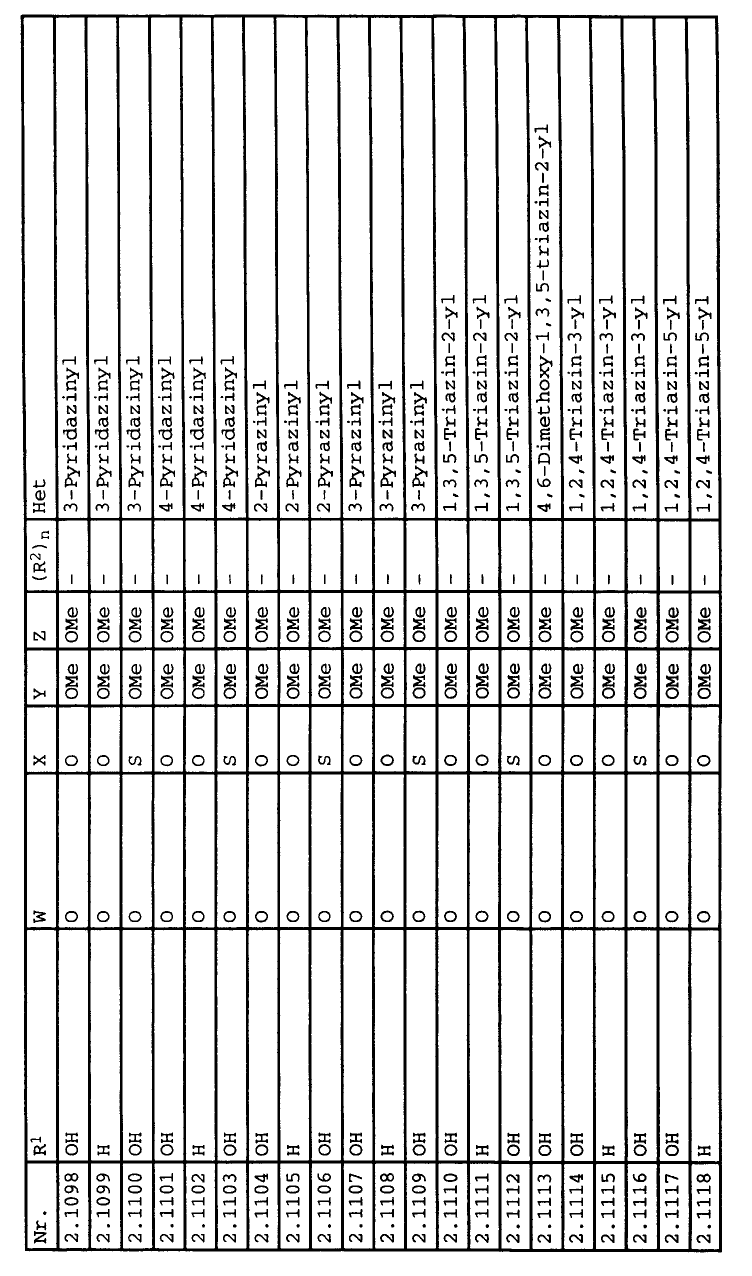

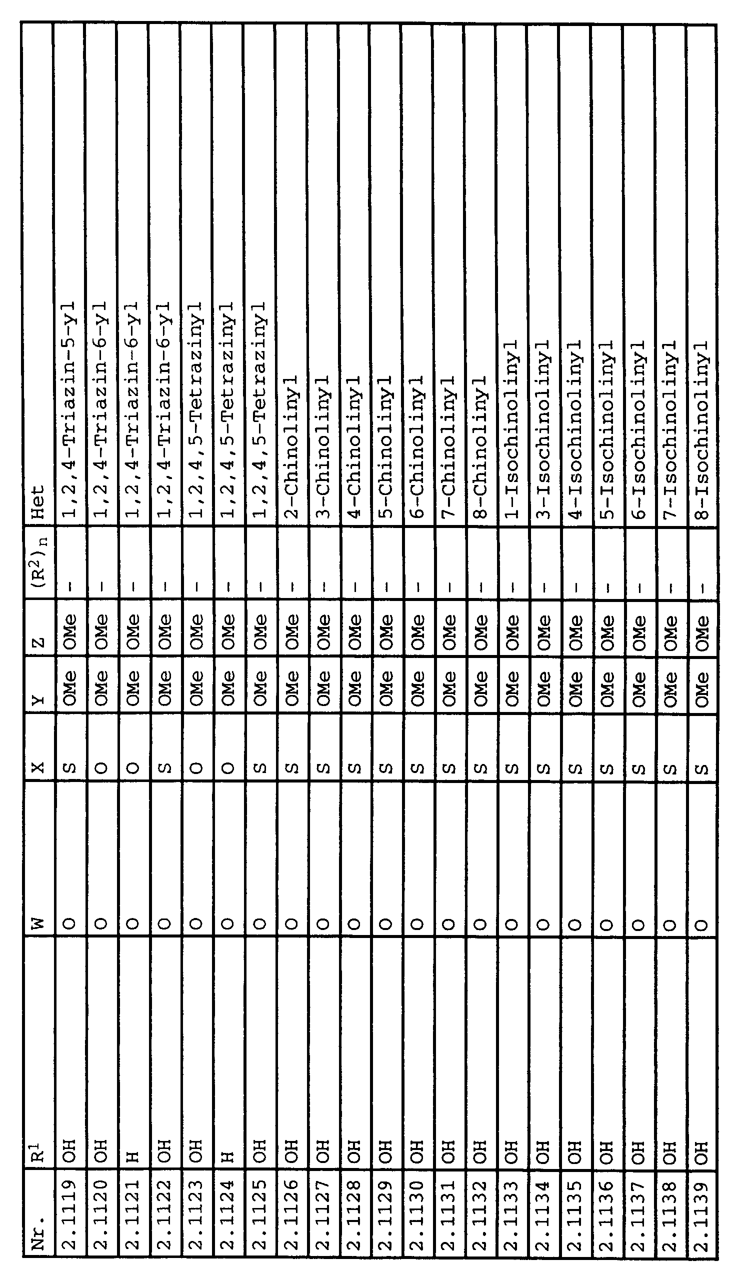

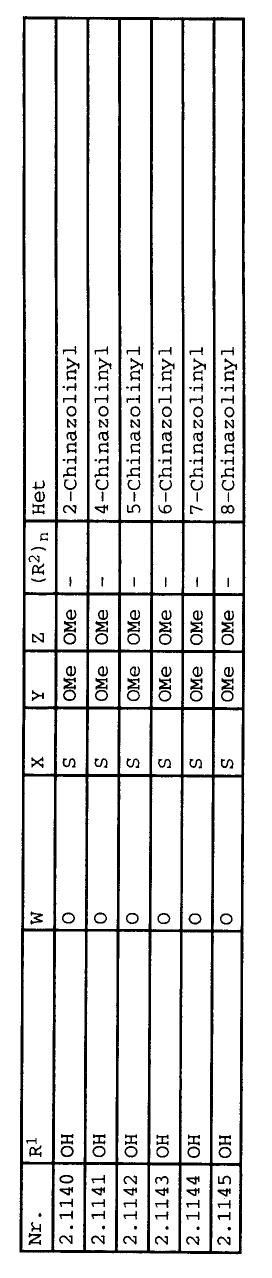

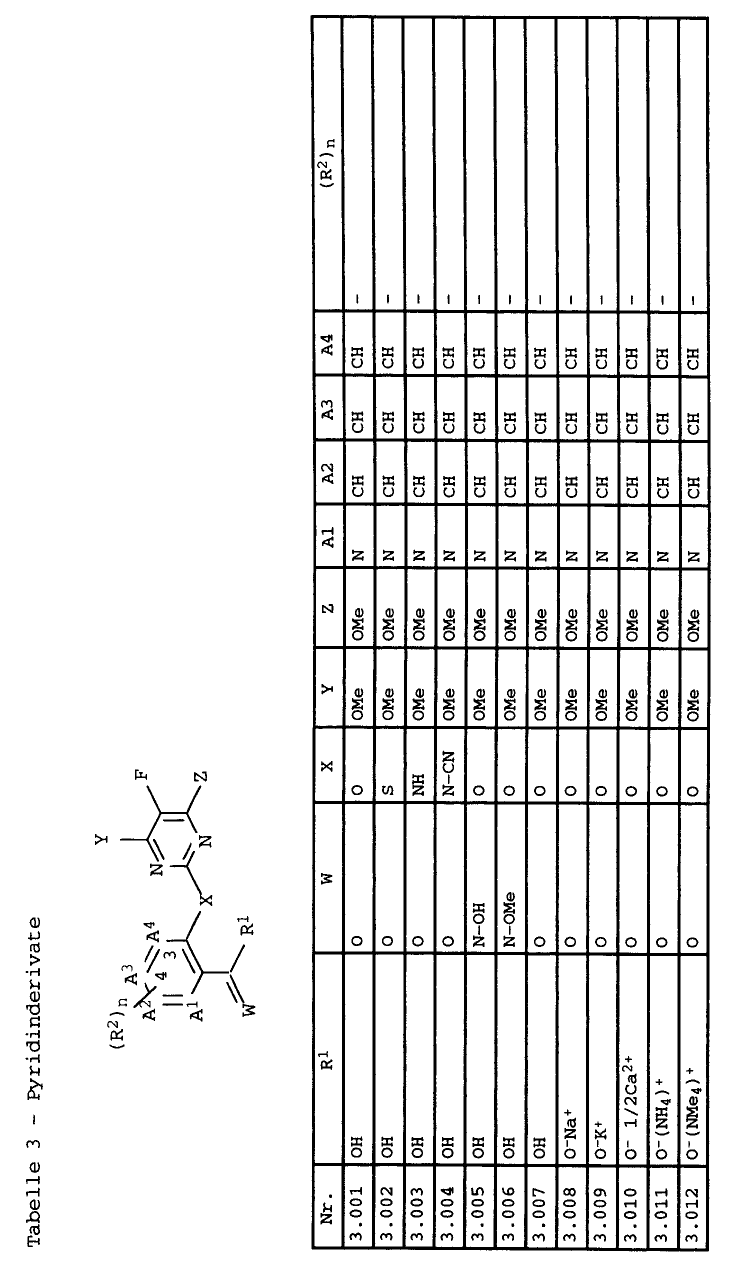

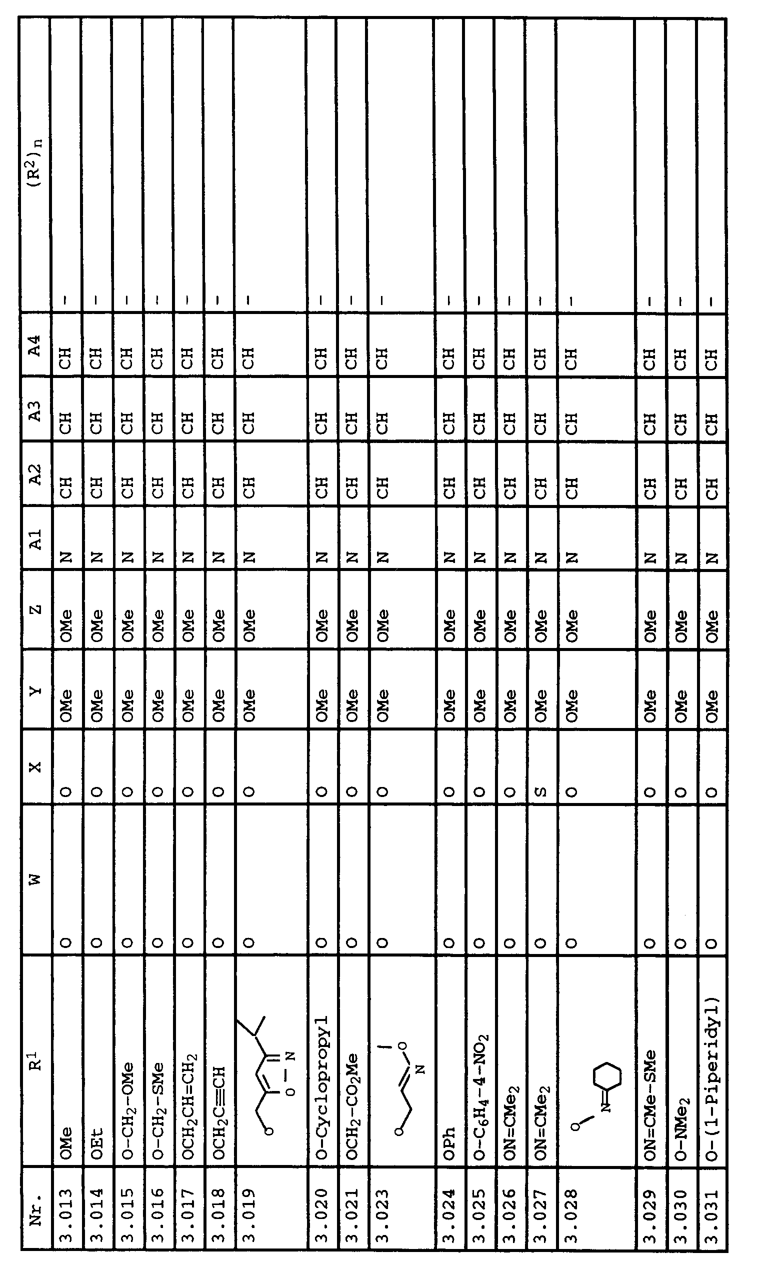

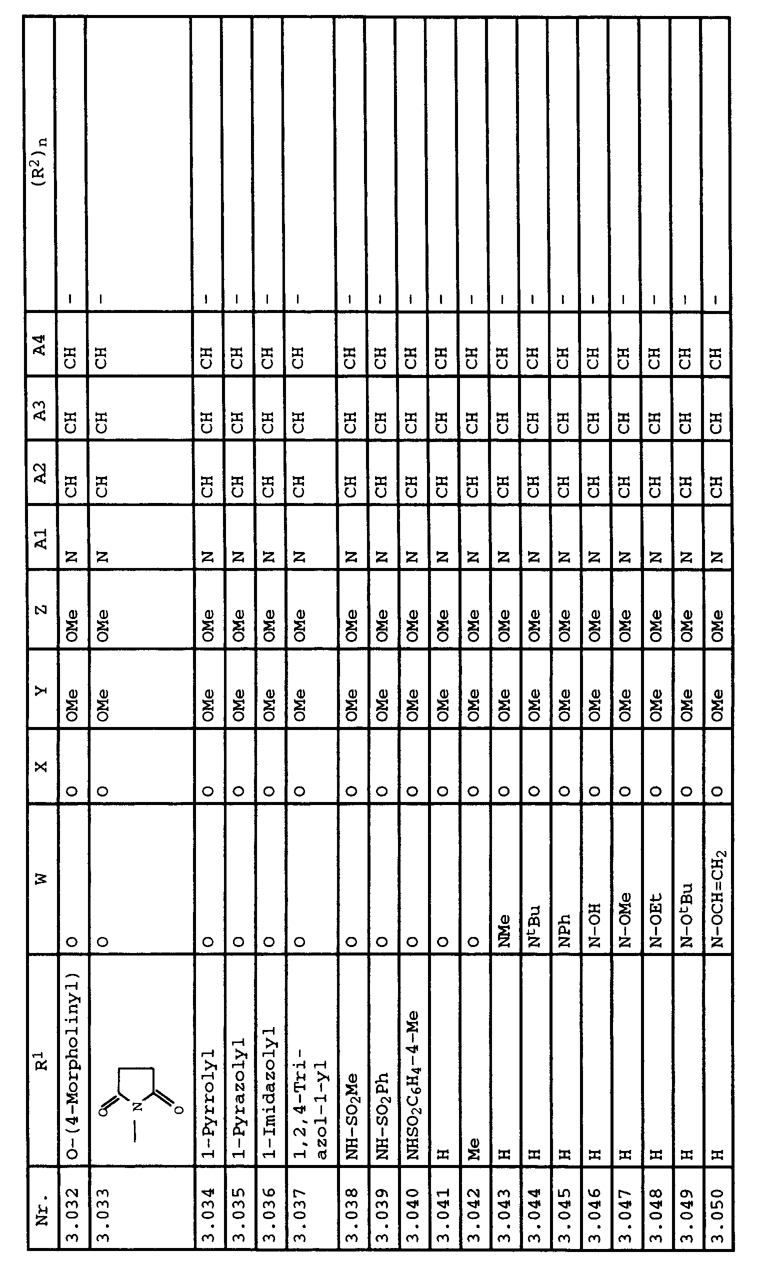

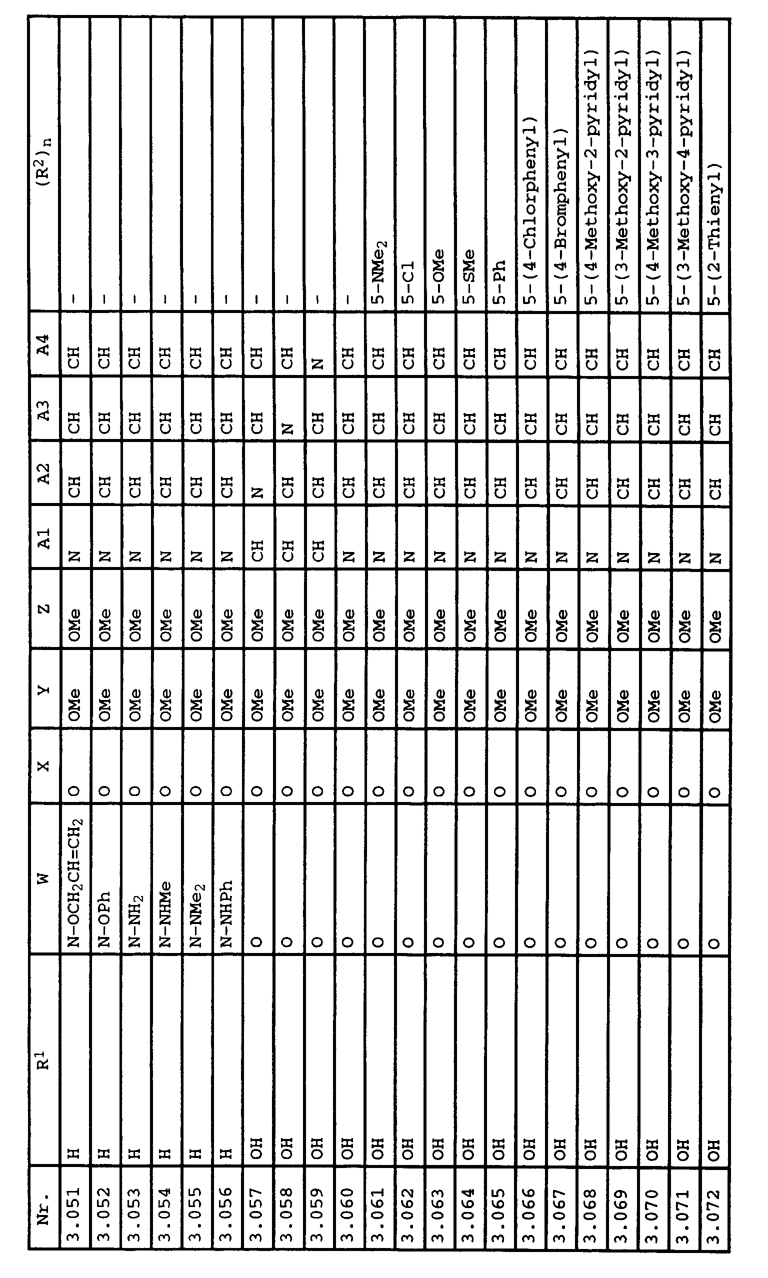

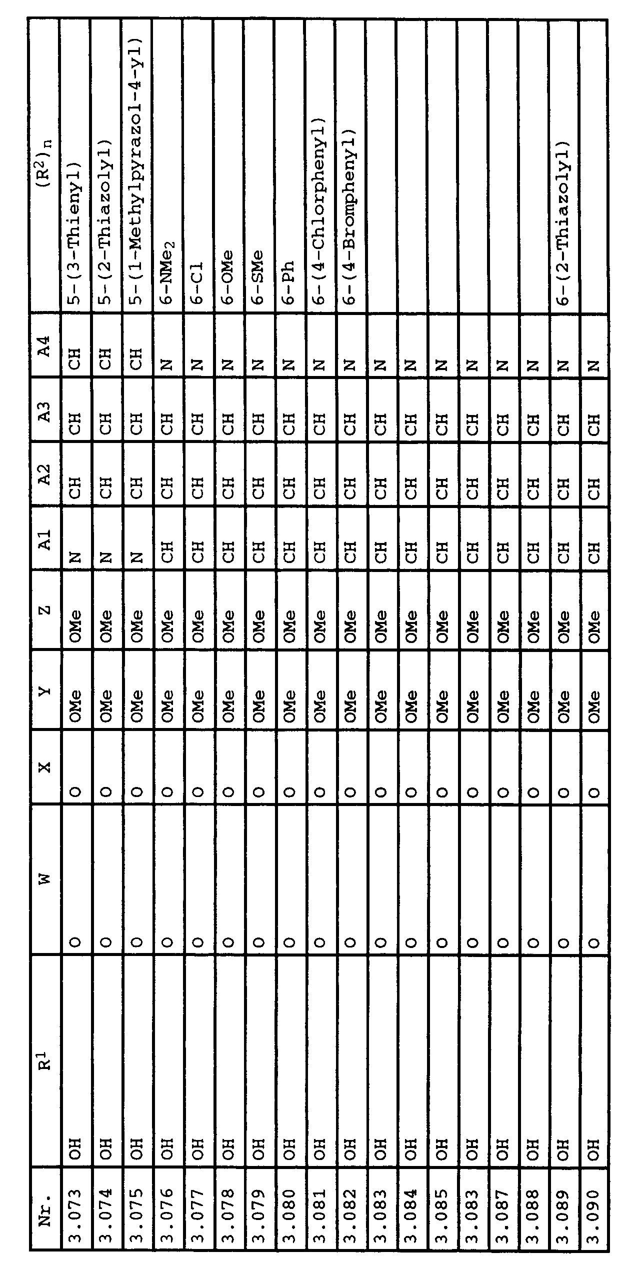

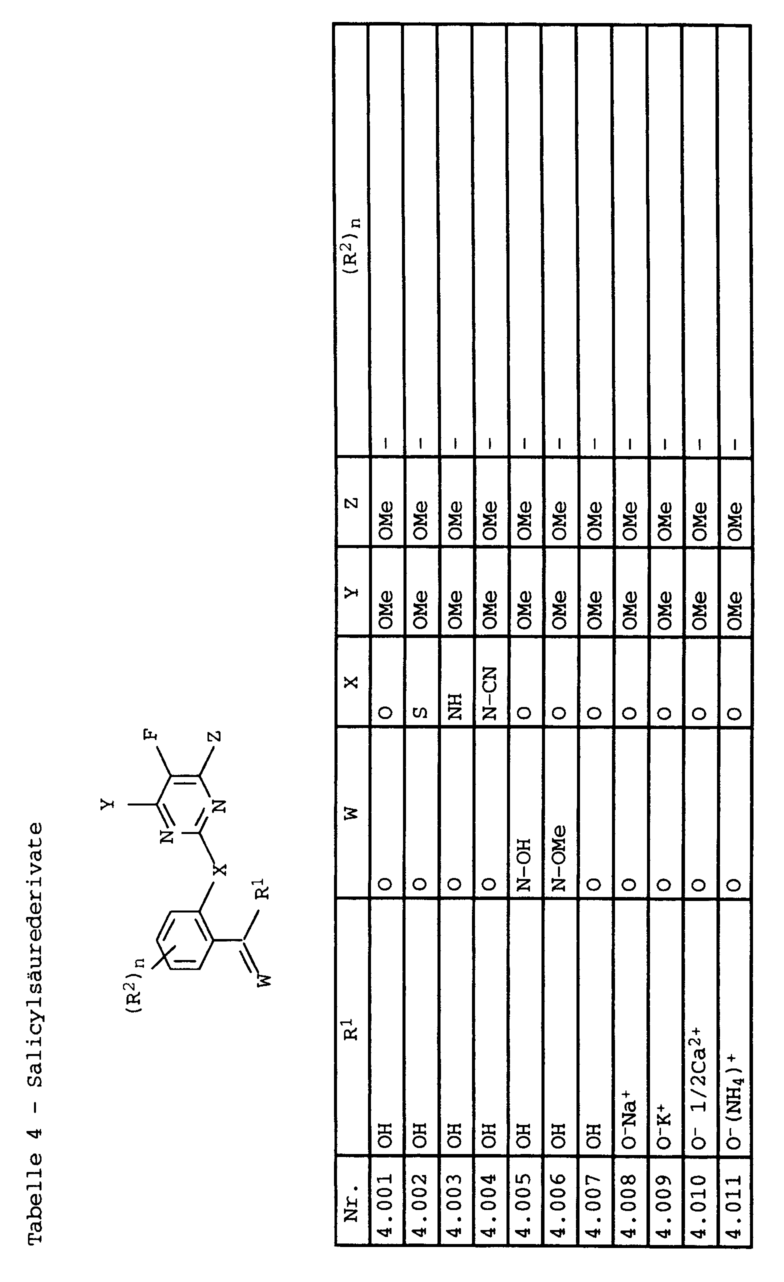

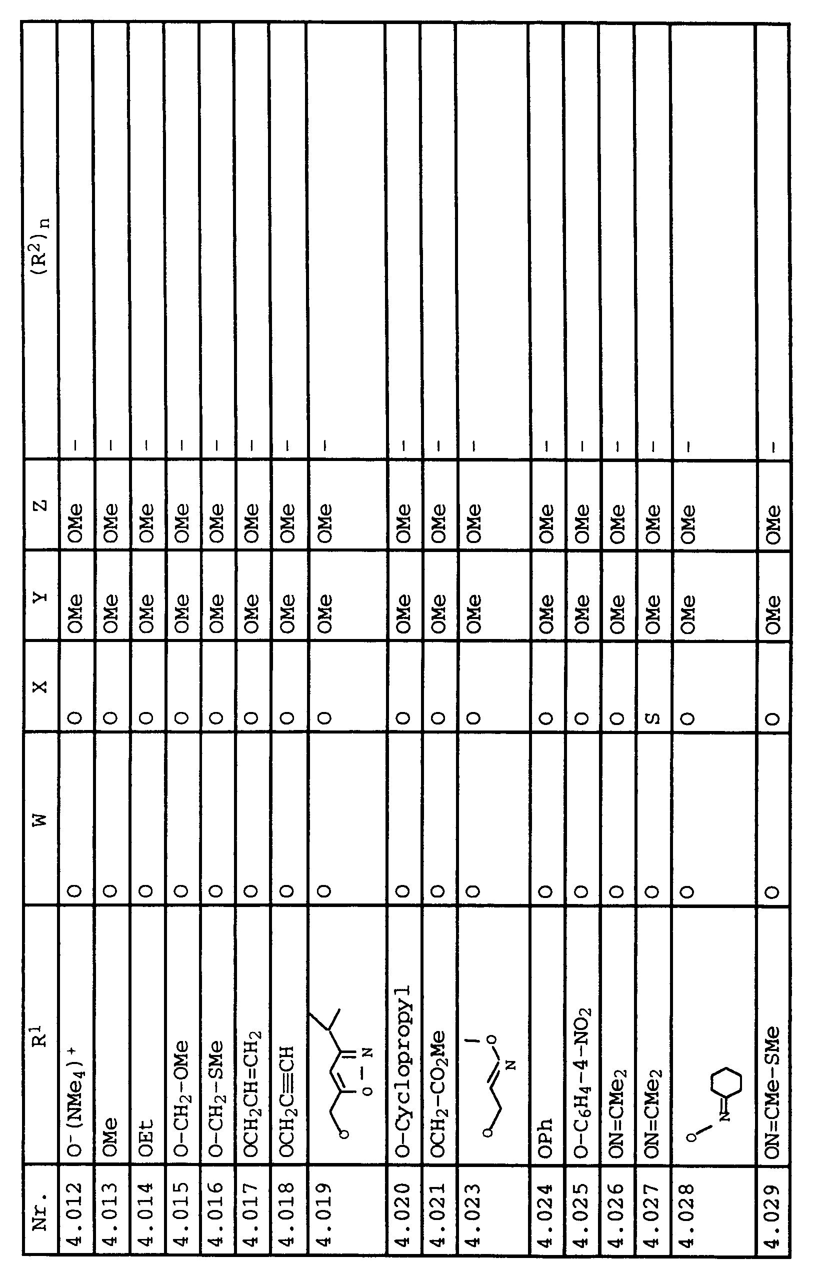

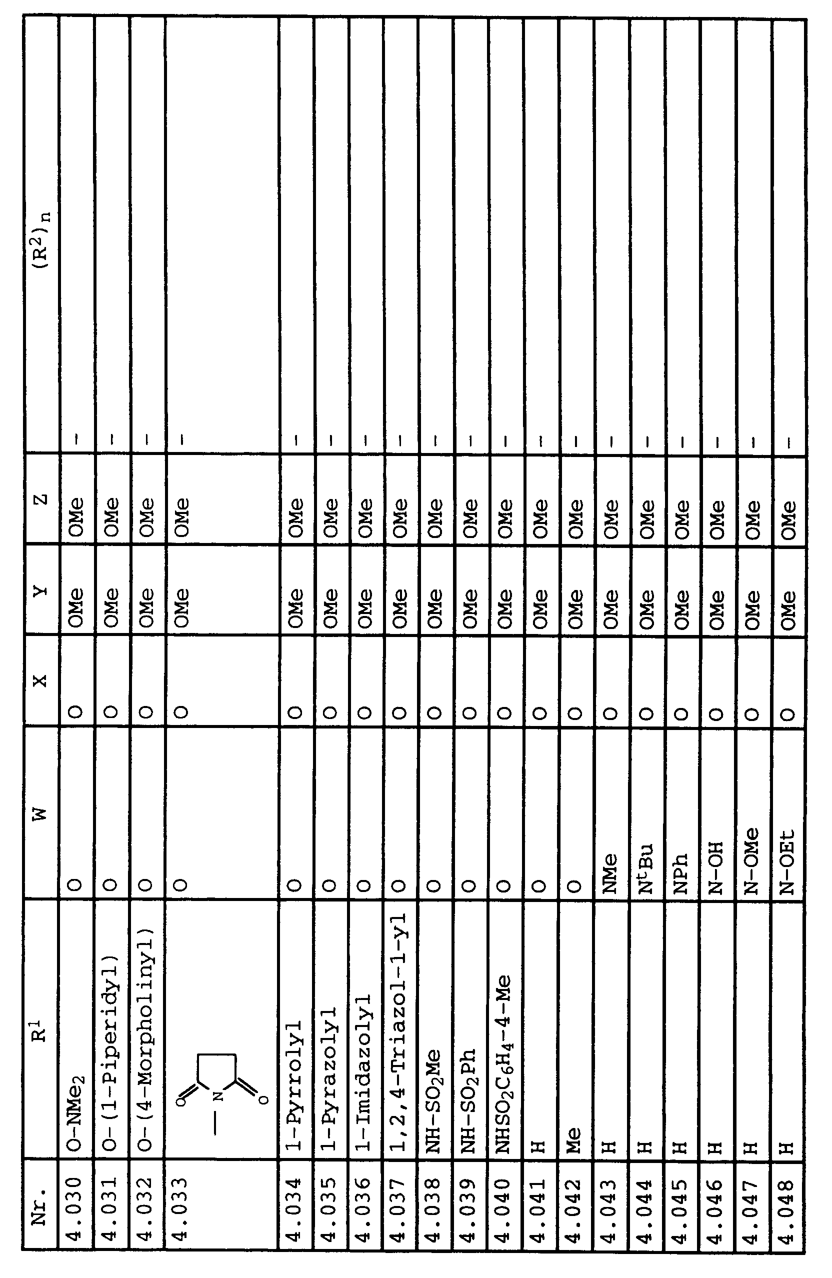

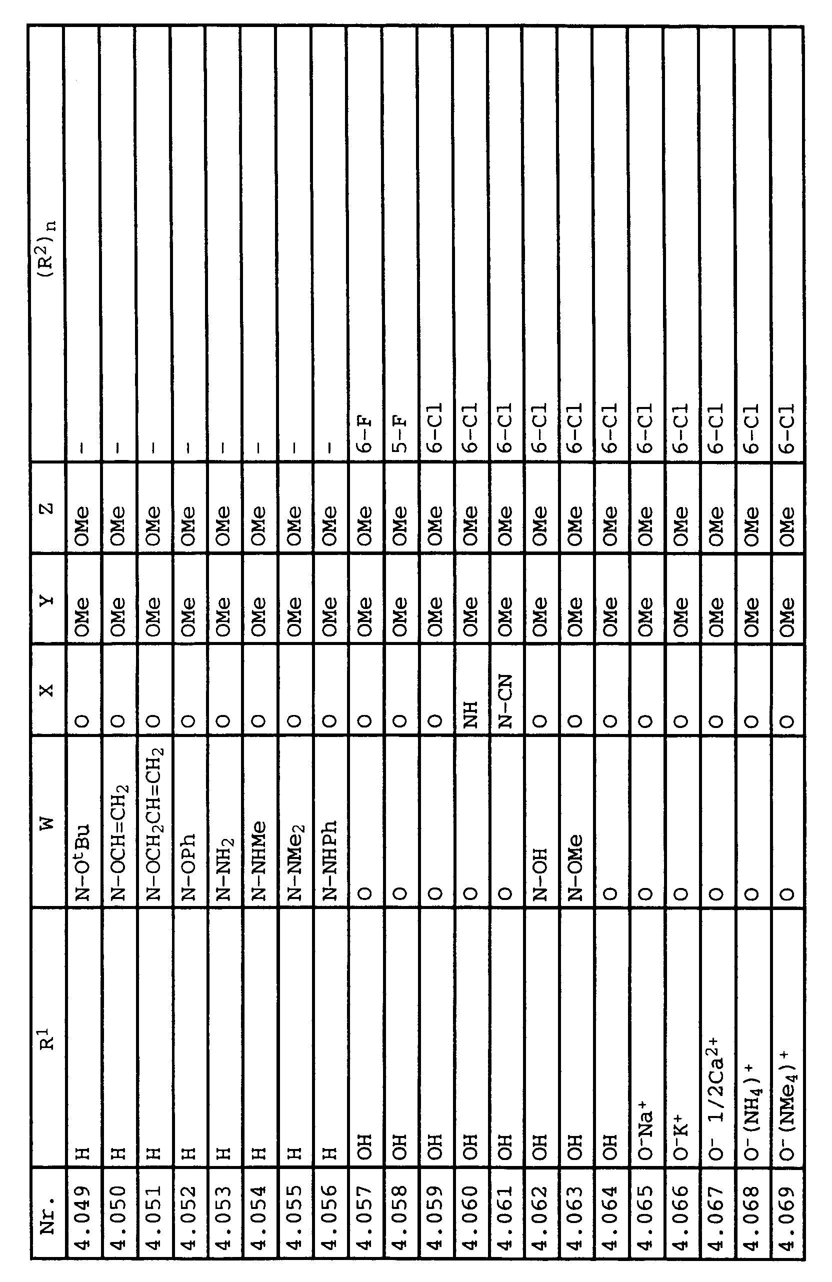

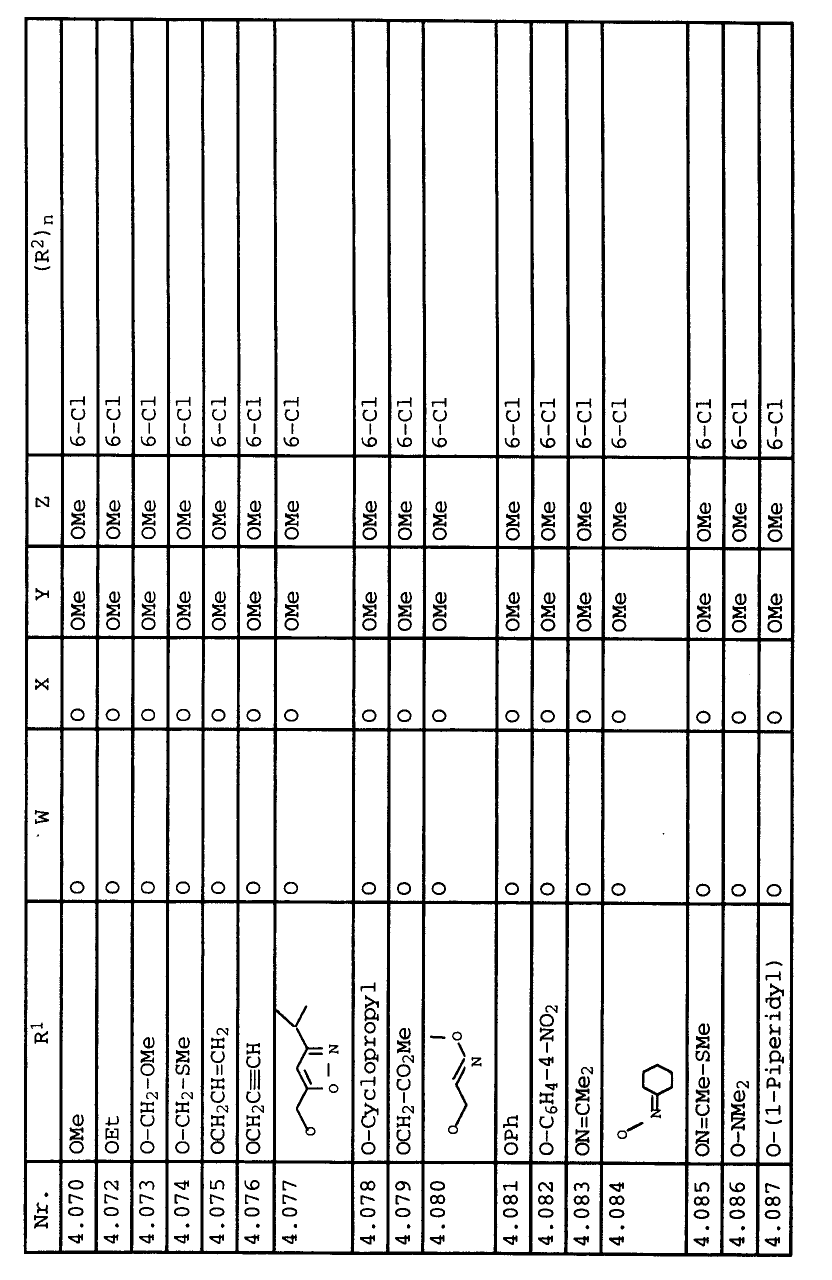

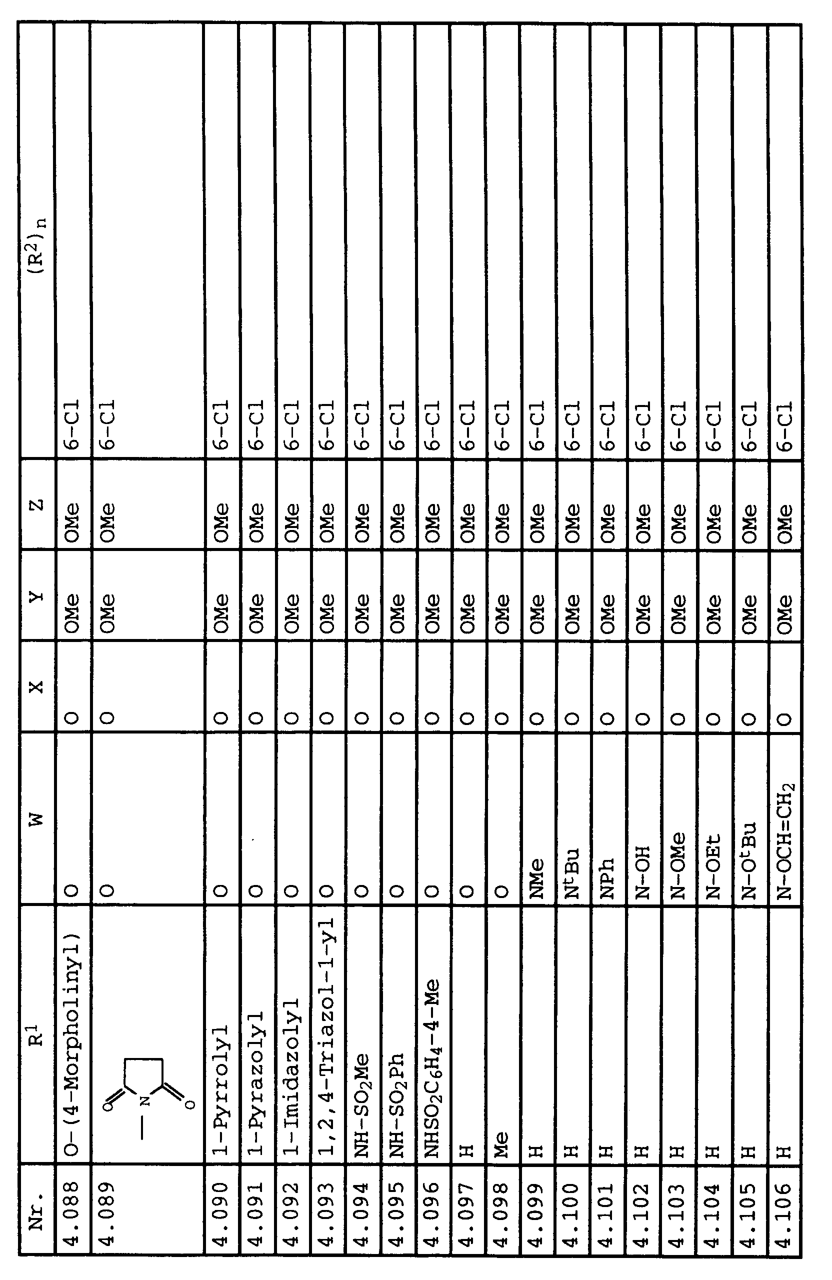

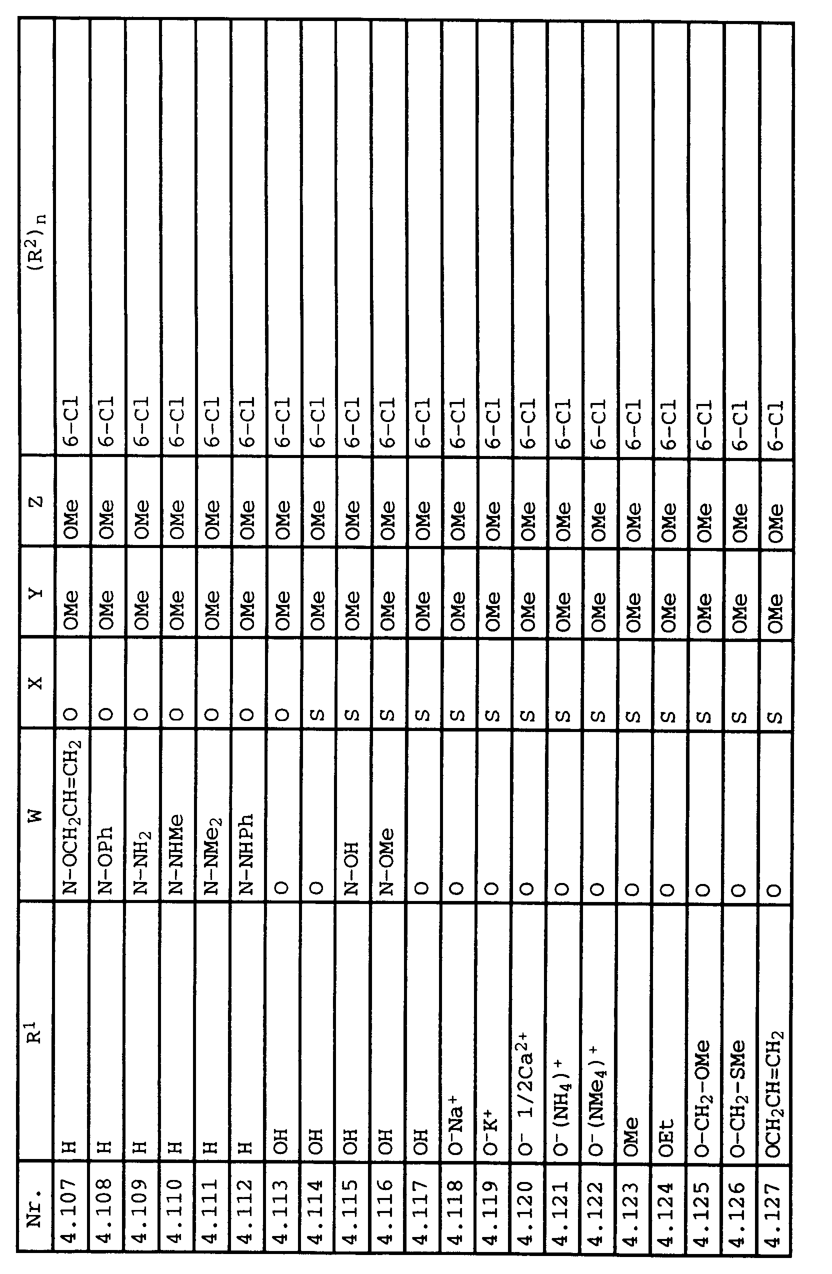

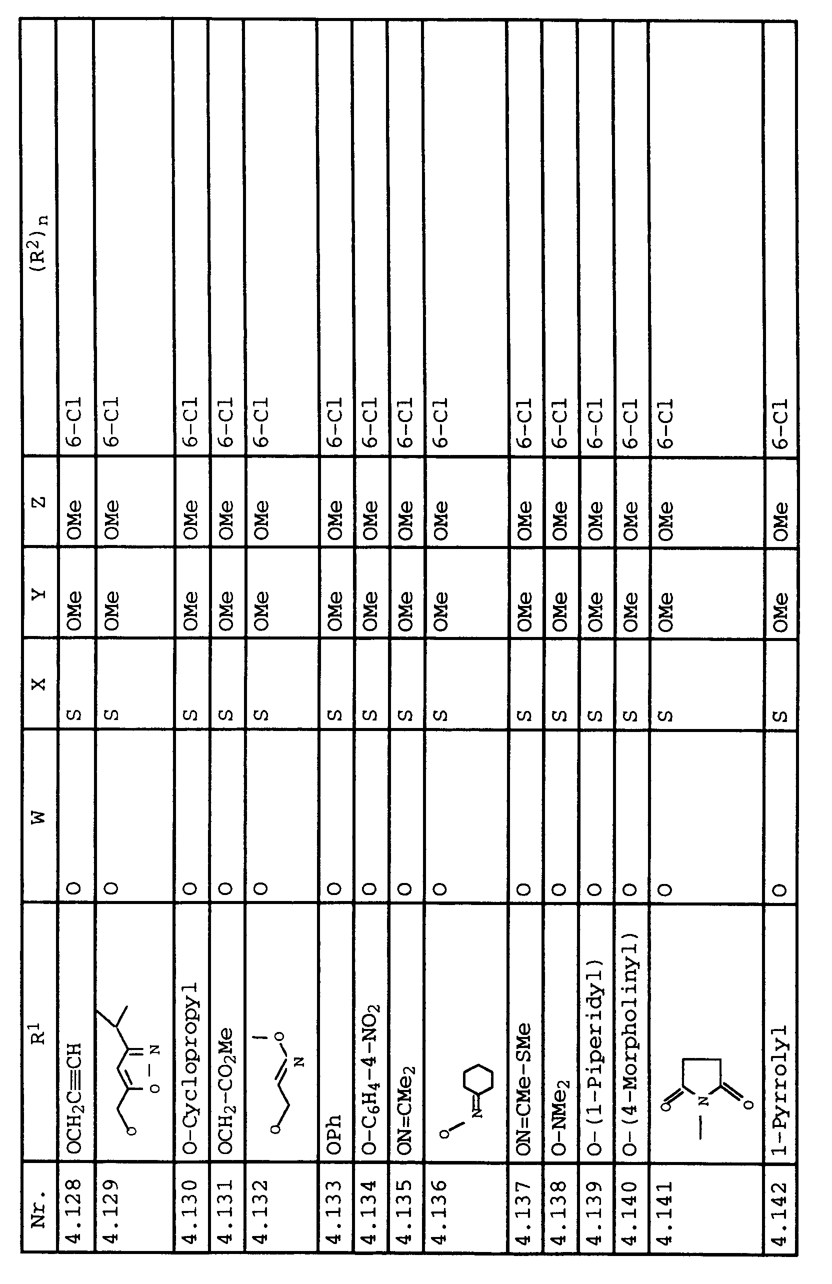

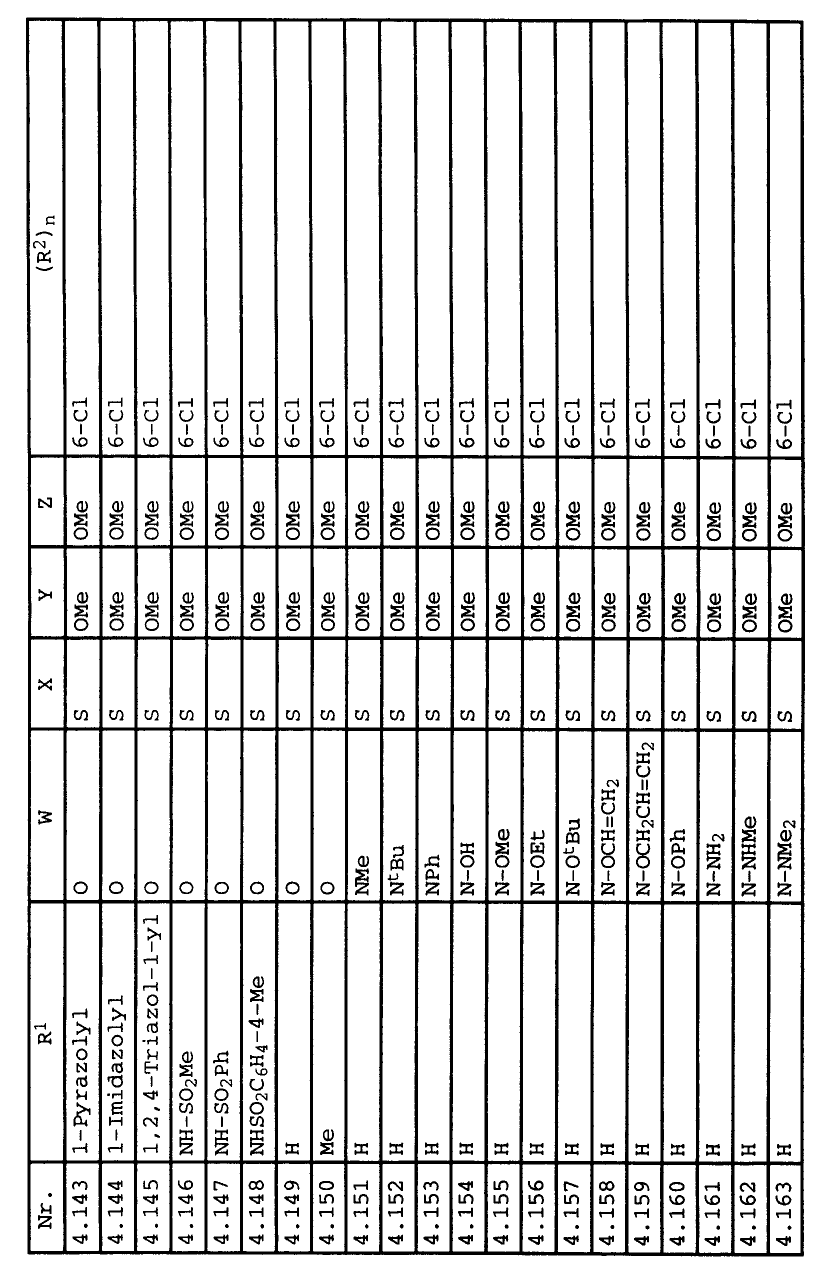

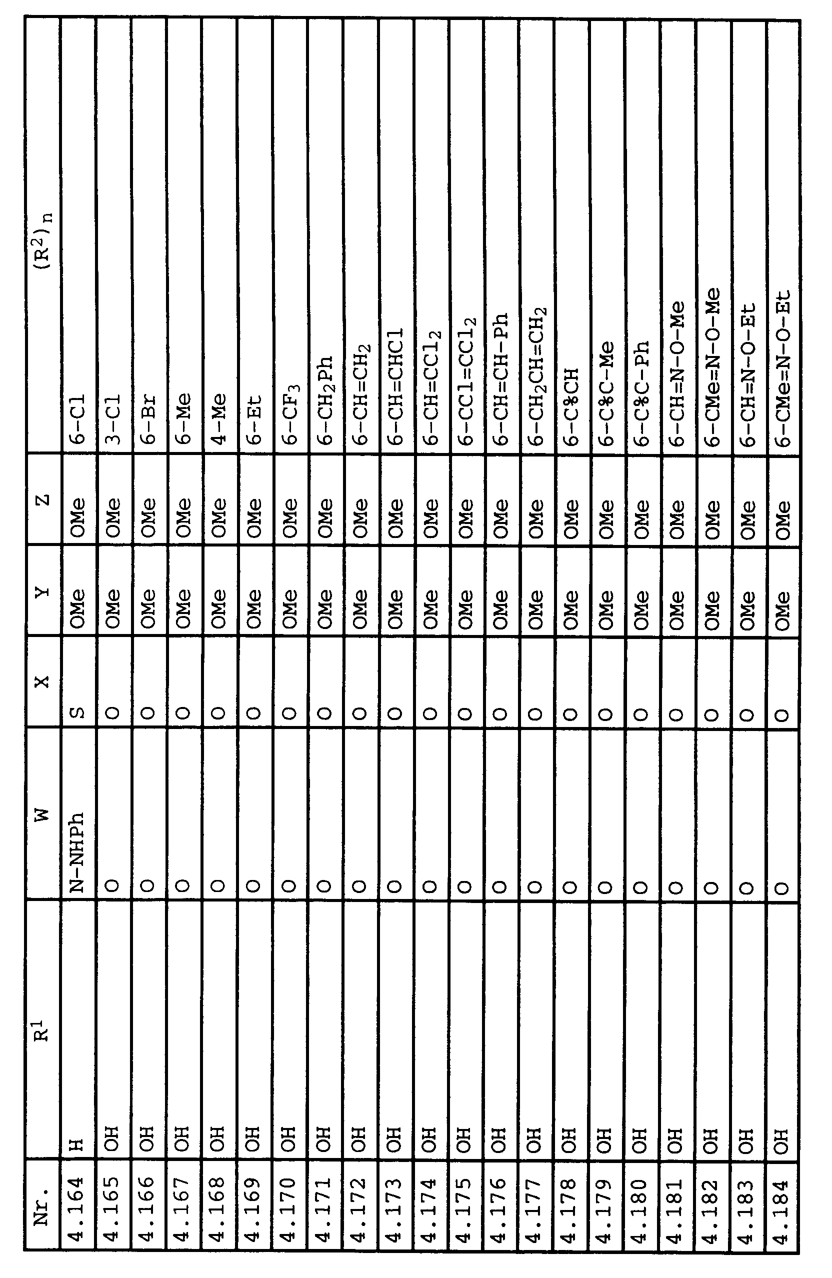

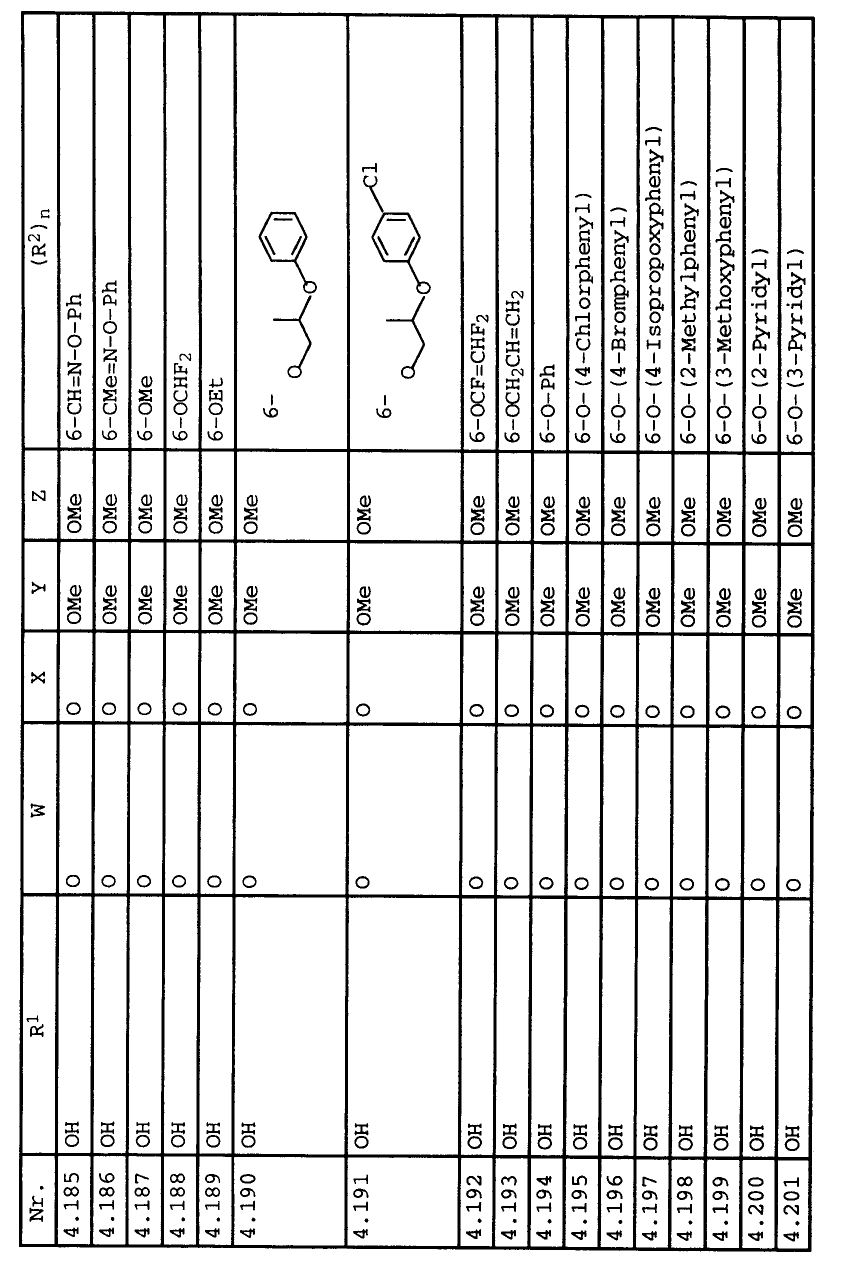

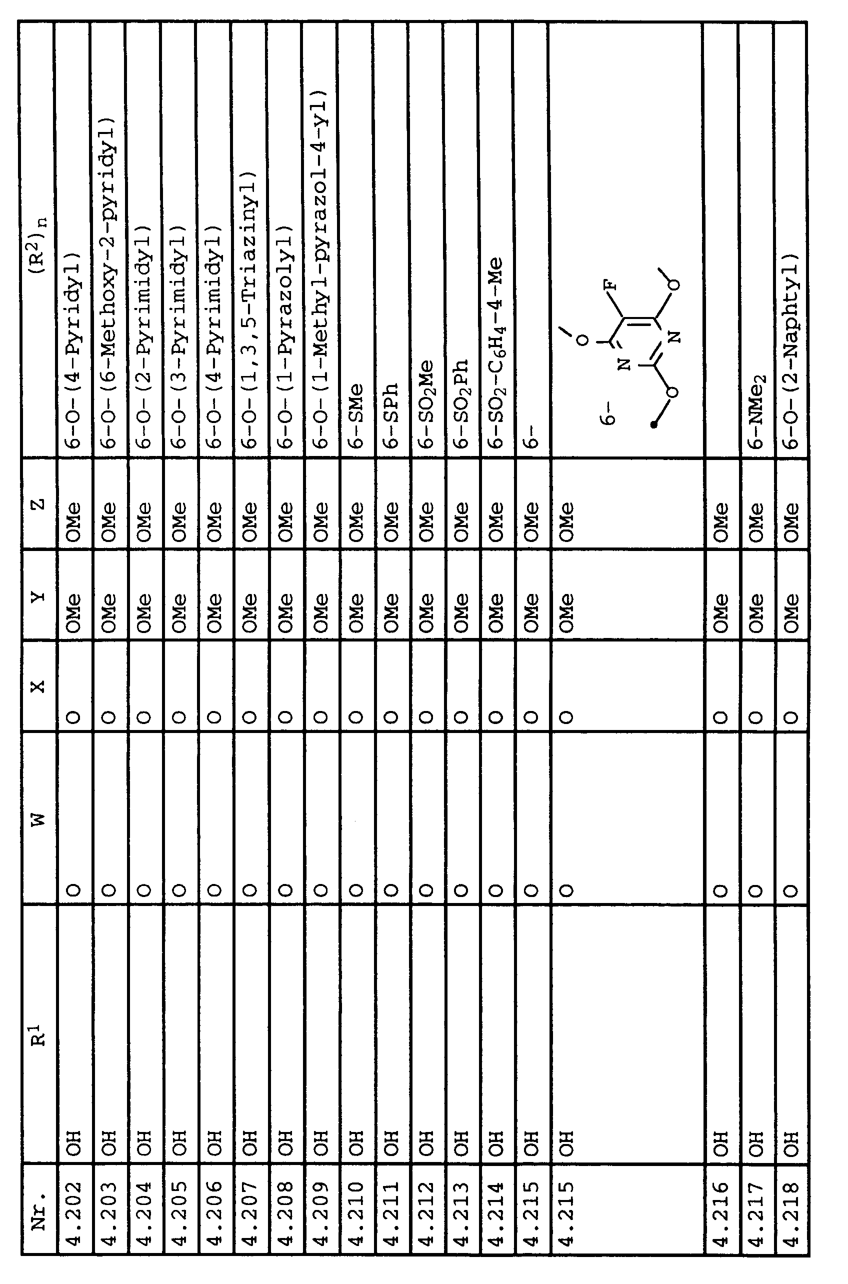

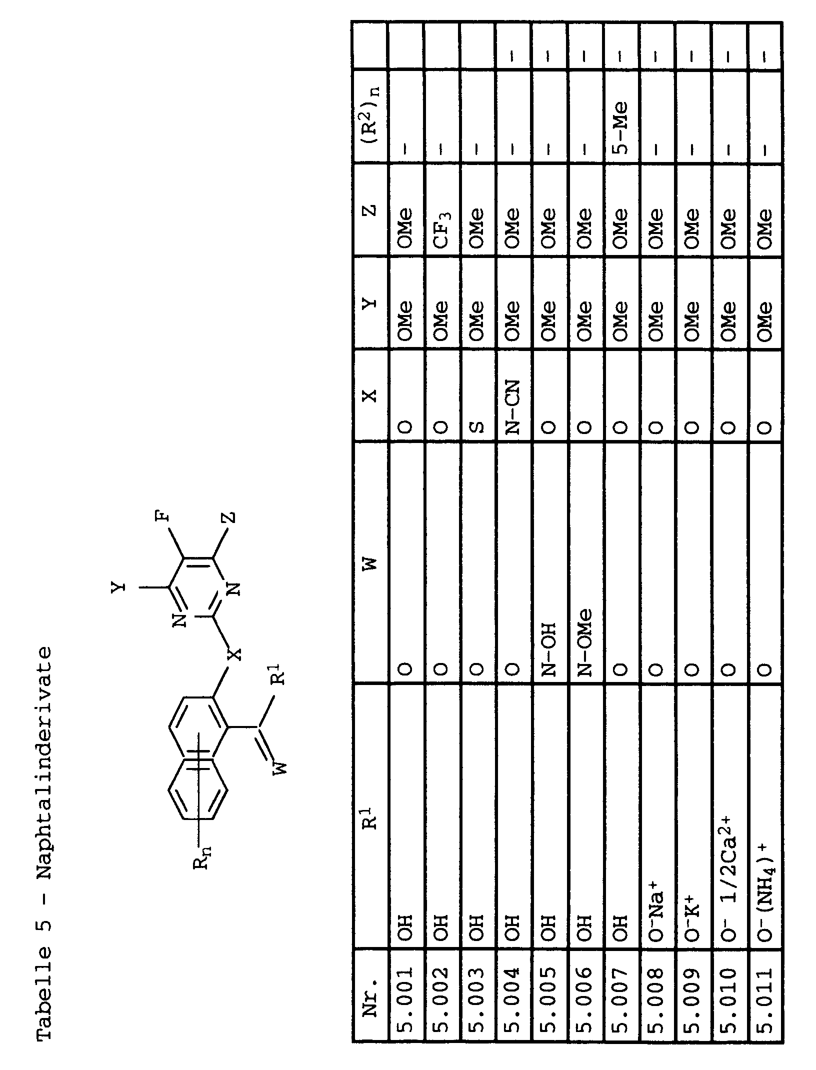

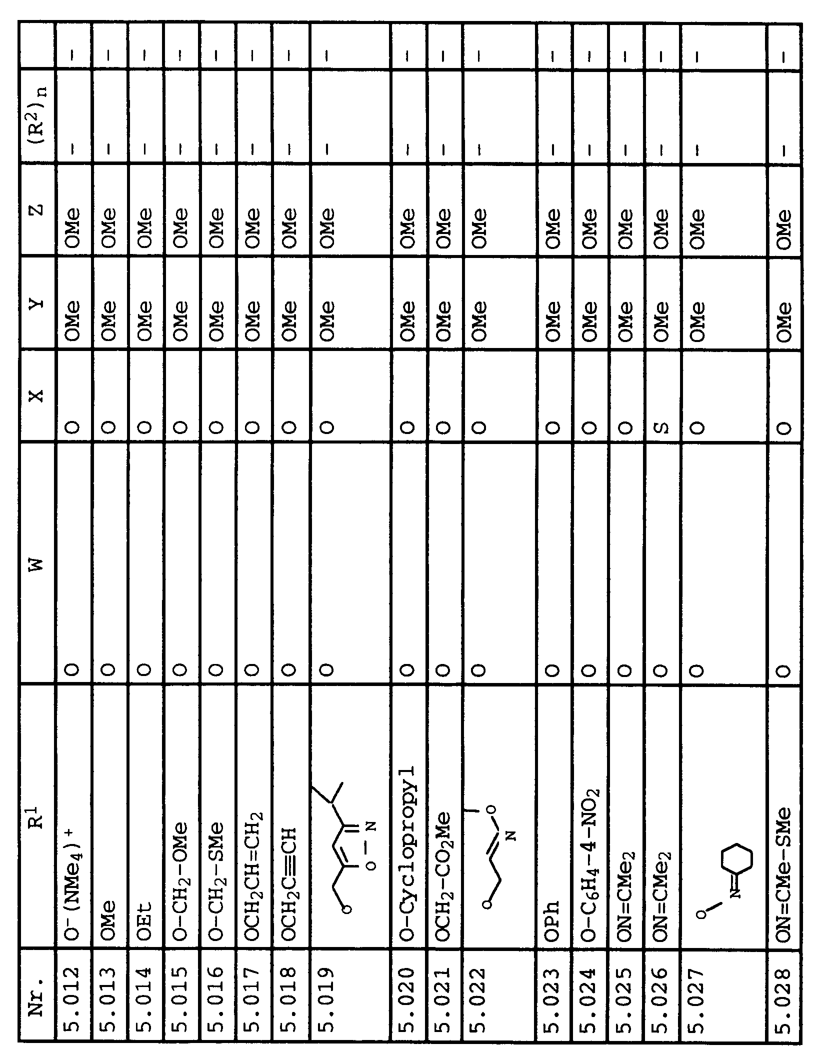

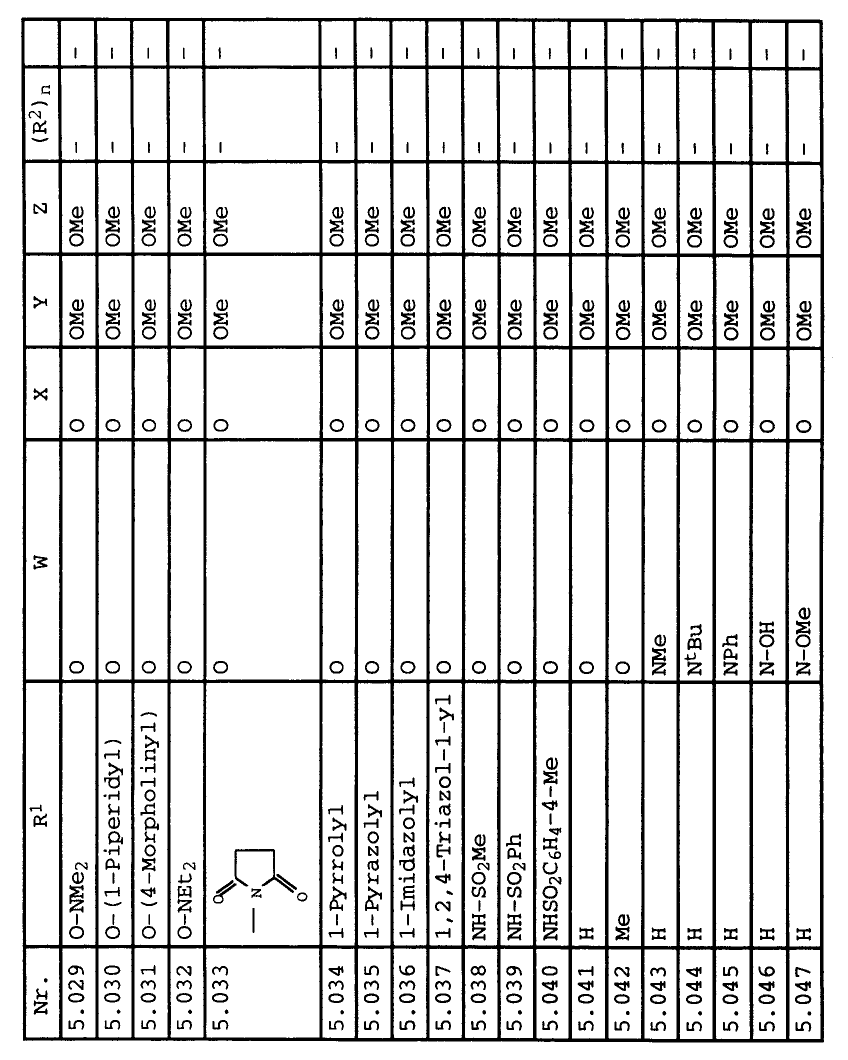

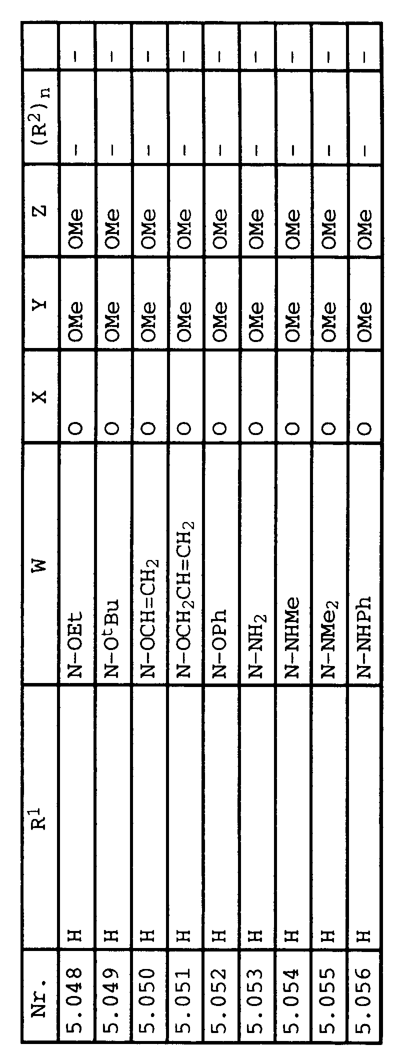

### Anwendungsbeispiele

### 5-Fluor-4,6-dihydroxy-2-methylsulfanyl-pyrimidin

44g(0.293mol) 2-Fluormalonsäuredimethylester wurden in 450ml Methanol gelöst und mit 106.5g(0.59mol) 30% NaOMe-Lösung versetzt. Anschließend fügte man 41.6g(0.15mol) Bis-(S-Methylisothioharnstoff)-Sulfat hinzu und rührte drei Tage bei RT. Der Ansatz wurde eingeengt, in Wasser gelöst und mit Salzsäure auf pH 1 gebracht. Der sich abscheidende Feststoff wurde abgesaugt und aus 1200ml Wasser umkristallisiert. Man erhielt 18g eines farblosen Feststoffs, mp. 230-238°C. Die Mutterlauge wurde auf 100ml eingeengt, wobei nochmals 4.6g Produkt (mp. 230°C) ausfielen. Gesamtausbeute: 22.6g(44%).

### 4,6-Dichlor-5-fluor-2-methylsulfanyl-pyrimidin

19.7g(0.112mol) 5-Fluor-4,6-dihydroxy-2-methylsulfanyl-pyrimidin wurden in 103g(0.67mol) Phosphoroxychlorid gelöst und mit 8.2g(0.068mol) N,N-Dimethylanilin versetzt. Man erhitzte unter Rückfluß, bis kein Ausgangsmaterial mehr vorhanden war (ca. 4h). Man ließ abkühlen, engte ein, versetzte den Rückstand mit 100ml Wasser, rührte auf und saugte den sich ausscheidenden Niederschlag ab. Man wusch mit Eiswasser und trocknete bei 40°C im Vakuum. Es verblieben 19.9g(83%) eines farblosen Feststoffs, mp. 54-56°C.

### 5-Fluor-4,6-dimethoxy-2-methylsulfanyl-pyrimidin

9g(42mmol) 4,6-Dichlor-5-fluor-2-methylsulfanyl-pyrimidin wurden in 32ml Methanol gelöst und mit 22.7g(126mmol) Natriummethylatlösung (30%) versetzt. Man erhitzte noch für 4h auf 50°C, wobei sich ein Feststoff abschied. Nach dem Abkühlen auf Raumtemperatur stellte man mit Eisessig auf pH 7 und rührte in 100ml Eiswasser ein. Der sich bildende Niederschlag wurde abgesaugt, mit Eiswasser nachgewaschen und bei Raumtemperatur an der Ölpumpe getrocknet. Man erhielt 8.1g(94%) eines farblosen Pulvers, mp. 78-79°C.

### 5-Fluor-4,6-dimethoxy-2-methylsulfonyl-pyrimidin

11.6g(57mmol) 5-Fluor-4,6-dimethoxy-2-methylsulfanyl-pyrimidin wurden in 80ml Eisessig vorgegeben, 0.86g Dinatriumwolframat zugefügt und auf 30°C erwärmt. Nun tropfte man 17.1ml(171mmol) Wasserstoffperoxidlösung (30%) hinzu und rührte über Nacht bei Raumtemperatur nach. Der Ansatz wurde in 400ml Eiswasser gegossen, 30min gerührt und dann abgesaugt und mit Eiswasser nachgewaschen. Nach dem Trocknen in Vakuumtrockenschrank bei 50°C verblieben 12.1g(90%) eines farblosen Feststoffs, mp. 130-133°C.

### 2-(5-Fluor-4,6-dimethoxy-pyrimidin-2-yloxy)-6-phenyl-benzoesäure (Verb. 1.001)

1.8 g(8.5mmol) 6-Phenylsalicylsäure wurden in 30 ml getrocknetem Dimethylsulfoxid gelöst und portionsweise mit 1.9g(17mmol) Kalium-tert.-butylat versetzt, wobei die Temperatur der Reaktionsmischung auf etwa 30°C stieg. Man kühlte auf Raumtemperatur, gab 2.0g(8.5mmol) 5-Fluor-4,6-dimethoxy-2-methylsulfonylpyrimidin zu und rührte etwa 4h bei 60°C. Die Reaktionsmischung wurde in die Mischung aus 100ml kaltem Wasser und 2ml Orhophosphorsäure gegossen und mit Methyl-tert.butylether extrahiert. Die organische Phase wurde mit etwas Wasser gewaschen, über Natriumsulfat getrocknet und eingeengt. Das zurückbleibende Öl wurde durch Chromatographie an Silica-Gel mit Hexan/Aceton gereinigt. Man erhielt 2.4g eines farblosen Pulvers, mp. 178-182°C.

Analog dem oben beschriebenen Verfahren wurden folgende Verbindungen hergestellt:
- 1.004: 6-(4-Chlorphenyl)-2-(5-fluor-4,6-dimethory-pyrimidin-2-yloxy)-benzoesäure, mp. 160-165°C
- 1.007: 6-(4-Bromphenyl)-2-(5-fluor-4,6-dimethoxy-pyrimidin-2-yl-oxy)-benzoesäure, mp. 160-168°C
- 2.1008: 6-(2-Methoxy-5-pyridyl)-2-(5-fluor-4,6-dimethoxy-pyrimidin-2-yloxy)-benzoesäure, mp. 178°C
- 2.1014: 6-(2-Ethoxy-6-pyridyl)-2-(5-fluor-4,6-dimethoxy-pyrimidin-2-yloxy)-benzoesäure, mp. 178-179°C.

## Patentansprüche

**1.** Fluorierte Pyrimidine der Formel I 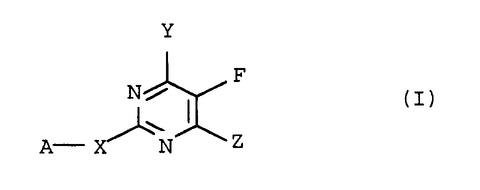 in welcher **A** für einen der folgenden Reste steht: 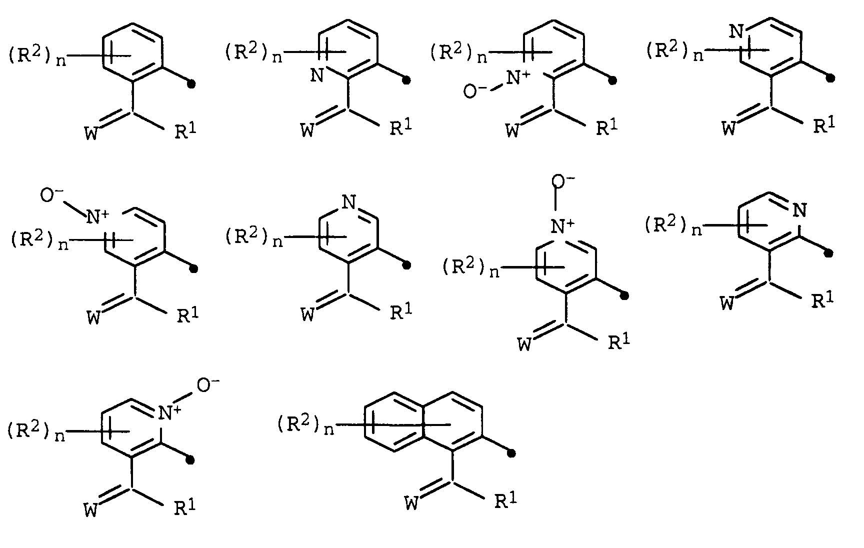 und in der die angegebenen Symbole folgende Bedeutung haben:
W =O, =N-R³, =N-O-R³, =N-NR⁴R⁵;
X -O-, -S-, und -NR⁶-;
Y,Z Halogen, Alkyl, Halogenalkyl, Alkoxy, Halogenalkoxy, Alkylthio, Alkylamino und/oder Dialkylamino;
R¹ Wasserstoff;
Alkyl, Alkylcarbonyl, Alkoxycarbonyl;
eine Succinyliminooxygruppe;
ein 5-gliedriger Heteroaromat, enthaltend ein bis drei Stickstoffatome, welcher ein bis vier Halogenatome und/oder ein bis zwei der folgenden Reste tragen kann: Alkyl, Halogenalkyl, Alkoxy, Halogenalkoxy und/oder Alkylthio;
ein Rest OR⁷;
ein Rest 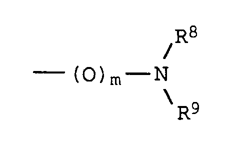 in dem R⁸ und R⁹ gleich oder unterschiedlich sein können;
oder ein Rest 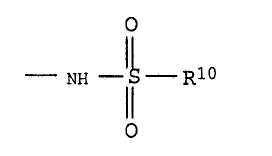
R² Wasserstoff;
Halogen;
Amino;
Alkyl, Alkenyl, Alkinyl, Alkoxy, Alkylthio, Alkylsulfonyl, Alkylamino, Dialkylamino, die einen der folgenden Substituenten tragen können, welche im Falle von Dialkylamino gleich oder unterschiedlich sein können: Alkoxy, Alkylthio, Dialkylamino, Thienyl, Furyl, einen fünfgliedrigen aromatischen Heterocyclus mit ein bis vier Stickstoffatomen oder ein bis drei Stickstoffatomen sowie zusätzlich einem Schwefel- oder Sauerstoffatom im Ring, wobei die Fünfringheterocyclen ein bis drei Halogenatome und/oder einen bis drei der folgenden Reste tragen können: Nitro, Cyano, Alkyl, Alkoxy, Alkylthio, Halogenalkyl; oder Phenyl, das unsubstituiert oder durch ein bis drei Halogenatome und/oder ein bis drei Methylgruppen substituiert ist; oder Phenoxy, das unsubstituiert oder durch ein bis drei Halogenatome und/oder ein bis drei Methylgruppen substituiert ist oder einen sechsgliedrigen aromatischen Heterocyclus mit ein bis drei Stickstoffatomen im Ring, welcher ein bis drei Halogenatome und/oder einen bis drei der folgenden Reste tragen kann: Nitro, Cyano, Alkyl, Alkoxy, Alkylthio, Halogenalkyl;
Phenyl, substituiertes Phenyl;
Phenoxy, substituiertes Phenoxy;
Phenylthio, substituiertes Phenylthio, Phenylsulfonyl, substituiertes Phenylsulfonyl;
einen 5-gliedrigen Heteroaromaten mit ein bis vier Stickstoffatomen oder ein bis drei Stickstoffatomen sowie zusätzlich einem Schwefel- oder Sauerstoffatom im Ring, welcher ein bis drei Halogenatome und/oder einen bis drei der folgenden Reste tragen kann: Nitro, Cyano, Alkyl, Alkoxy, Alkylthio, Halogenalkyl, Halogenalkoxy oder Phenyl, das unsubstituiert oder durch ein bis drei Halogenatome und/oder ein bis drei Methylgruppen substituiert ist;
einen benzokondensierten 5-gliedrigen Heteroaromaten, der ein bis drei Stickstoffatome oder ein Stickstoffatom und zusätzlich ein Sauerstoff- oder Schwefelatom enthalten und einen der folgenden Reste tragen kann: Halogen, Nitro, Cyano, Alkyl, Alkoxy, Alkylthio, Halogenalkyl, Halogenalkoxy oder Phenyl, das unsubstituiert oder durch ein bis drei Halogenatome und/oder ein bis drei Methylgruppen substituiert ist;
einen Thienyl- oder Furylrest, der ein bis drei Halogenatome und/oder einen bis drei der folgenden Reste tragen kann: Nitro, Cyano, Alkyl, Alkoxy, Alkylthio, Halogenalkyl, Halogenalkoxy oder Phenyl, das unsubstituiert oder durch ein bis drei Halogenatome und/oder ein bis drei Methylgruppen substituiert ist;
einen Benzthienyl- oder Benzofurylrest, der ein bis fünf Halogenatome und/oder einen bis drei der folgenden Reste tragen kann: Nitro, Cyano, Alkyl, Alkoxy, Alkylthio, Halogenalkyl, Halogenalkoxy oder Phenyl, das unsubstituiert oder durch ein bis drei Halogenatome und/oder ein bis drei Methylgruppen substituiert ist;
einen 6-gliedrigen Heteroaromaten mit ein bis vier Stickstoffatomen im Ring, welcher ein bis drei Halogenatome und/oder einen bis drei der folgenden Reste tragen kann: Nitro, Cyano, Alkyl, Alkoxy, Alkylthio, Halogenalkyl, Halogenalkoxy oder Phenyl, das unsubstituiert oder durch ein bis drei Halogenatome und/oder ein bis drei Methylgruppen substituiert ist;
einen Naphthyl-, Chinolinyl-, Isochinolinyl- oder Chinazolinylrest welcher jeweils bis zu drei Halogenatome und/oder bis zu drei der folgenden Reste tragen kann: Nitro, Cyano, Alkyl, Alkoxy, Alkylthio, Halogenalkyl, Halogenalkoxy oder Phenyl, das unsubstituiert oder durch ein bis drei Halogenatome und/oder ein bis drei Methylgruppen substituiert ist;
einen über ein Sauerstoffatom gebundenen 5-gliedrigen Heteroaromaten mit ein bis vier Stickstoffatomen oder ein bis drei Stickstoffatomen sowie zusätzlich einem Schwefel- oder Sauerstoffatom im Ring, welcher ein bis drei Halogenatome und/oder einen bis drei der folgenden Reste tragen kann: Nitro, Cyano, Alkyl, Alkoxy, Alkylthio, Halogenalkyl, Halogenalkoxy oder Phenyl, das unsubstituiert oder durch ein bis drei Halogenatome und/oder ein bis drei Methylgruppen substituiert ist;
einen über ein Sauerstoffatom gebundenen benzokondensierten 5-gliedrigen Heteroaromaten, der ein bis drei Stickstoffatome oder ein Stickstoffatom und zusätzlich ein Sauerstoff- oder Schwefelatom enthalten und einen der folgenden Reste tragen kann: Halogen, Nitro, Cyano, Alkyl, Alkoxy, Alkylthio, Halogenalkyl, Halogenalkoxy oder Phenyl, das unsubstituiert oder durch ein bis drei Halogenatome und/oder ein bis drei Methylgruppen substituiert ist;
einen über ein Sauerstoffatom gebundenen Thienyl- oder Furylrest, der ein bis drei Halogenatome und/oder einen bis drei der folgenden Reste tragen kann: Nitro, Cyano, Alkyl, Alkoxy, Alkylthio, Halogenalkyl, Halogenalkoxy oder Phenyl, das unsubstituiert oder durch ein bis drei Halogenatome und/oder ein bis drei Methylgruppen substituiert ist;
einen über ein Sauerstoffatom gebundenen Benzthienyl- oder Benzofurylrest, der ein bis fünf Halogenatome und/oder einen bis drei der folgenden Reste tragen kann: Nitro, Cyano, Alkyl, Alkoxy, Alkylthio, Halogenalkyl, Halogenalkoxy oder Phenyl, das unsubstituiert oder durch ein bis drei Halogenatome und/oder ein bis drei Methylgruppen substituiert ist;
einen über ein Sauerstoffatom gebundenen 6-gliedrigen Heteroaromaten mit ein bis vier Stickstoffatomen im Ring, welcher ein bis drei Halogenatome und/oder einen bis drei der folgenden Reste tragen kann: Nitro, Cyano, Alkyl, Alkoxy, Alkylthio, Halogenalkyl, Halogenalkoxy oder Phenyl, das unsubstituiert oder durch ein bis drei Halogenatome und/oder ein bis drei Methylgruppen substituiert ist;
einen über ein Sauerstoffatom gebundenen Naphthyl-, Chinolinyl-, Isochinolinyl- oder Chinazolinylrest welcher jeweils bis zu drei Halogenatome und/oder bis zu drei der folgenden Reste tragen kann: Nitro, Cyano, Alkyl, Alkoxy, Alkylthio, Halogenalkyl, Halogenalkoxy oder Phenyl, das unsubstituiert oder durch ein bis drei Halogenatome und/oder ein bis drei Methylgruppen substituiert ist;
einen Rest 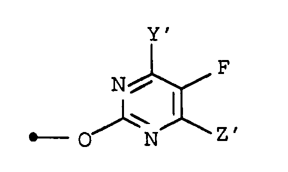 in dem X' und Z', die oben für X und Z angegebene Bedeutung haben und gleich oder unterschiedlich zu ihnen sein können;
eine Gruppierung 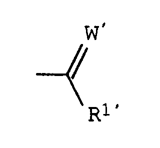 in der W' und R¹' die gleiche Bedeutung wie W bzw. R¹ haben, und gleich oder verschieden von diesen Einheiten sein können;
R³ Wasserstoff, Phenyl, substituiertes Phenyl oder Alkyl, Alkenyl, Alkinyl, die ein bis drei der folgenden Substituenten tragen können: Halogen, Alkyl, Alkoxy, Alkylcarbonyl, Alkoxycarbonyl, Carboxy, Alkylthio, Phenyl, substituiertes Phenyl;
R⁴ Wasserstoff, Alkyl, Phenyl;
R⁵ Wasserstoff, Phenyl, substituiertes Phenyl oder Alkyl, das ein bis drei der folgenden Substituenten tragen kann: Halogen, Alkoxy, Alkylcarbonyl, Alkoxcarbonyl, Carboxy, Alkylthio, Phenyl, substituiertes Phenyl;
R⁶ Wasserstoff, Alkyl, Formyl, Carbamoyl, Alkoxycarbonyl;
R⁷ Wasserstoff, ein Alkalimetallkation, das Äquivalent eines Erdalkalimetallkations oder ein organisches Ammoniumion;
eine Cycloalkylgruppe, welche ein bis drei Alkylreste tragen kann;
eine Alkylgruppe welche ein bis fünf Halogenatome und/oder einen oder zwei der folgenden Reste tragen kann: Alkoxy, Alkylthio, Cyano, Alkylcarbonyl, Alkoxycarbonyl, Cycloalkyl, ein Rest-O-N=CR¹¹R¹², in dem R¹¹ und R¹² gleich oder verschieden sein können, Phenyl, Phenoxy, oder Phenylcarbonyl, wobei die aromatischen Reste ihrerseits ein bis fünf Halogenatome und/oder ein bis drei der folgenden Reste tragen können: Alkyl, Halogenalkyl, Alkoxy, Halogenalkoxy und/oder Alkylthio;
eine Alkylgruppe, welche ein bis fünf Halogenatome tragen kann und einen der folgenden Reste trägt: ein 5-gliedriger Heteroaromat, enthaltend ein bis drei Stickstoffatome, welcher ein bis vier Halogenatome und/oder ein bis zwei der folgenden Reste tragen kann: Alkyl, Halogenalkyl, Alkoxy, Halogenalkoxy und/oder Alkylthio;
eine Alkylgruppe, welche in der 2-Position einen der folgenden Reste trägt: Alkoxyimino, Alkenyloxyimino, Halogenalkenyloxyimino oder Benzyloxyimino;
eine Alkenyl- oder eine Alkinylgruppe, wobei diese Gruppen ihrerseits ein bis fünf Halogenatome tragen können;
unsubstituiertes oder ein- bis dreifach durch Nitro, Alkyl oder Alkoxy substituiertes oder ein- bis fünffach durch Halogen substituiertes Phenyl;
ein Rest -N=CR¹³R¹⁴, in dem R¹³ und R¹⁴ gleich oder verschieden sein können;
ein über ein Stickstoffatom gebundener 5-gliedriger aromatischer Heterocyclus mit ein bis vier Stickstoffatomen im Ring oder ein über ein Stickstoffatom gebundener benzokondensierter 5-gliedriger aromatischer Heterocyclus mit ein bis drei Stickstoffatomen im Ring, die von Halogen, Alkyl, Halogenalkyl substituiert sein können;
R⁸,R⁹ Wasserstoff;
Alkyl, Alkenyl, Alkinyl, wobei diese Reste jeweils ein bis fünf Halogenatome und/oder ein bis zwei der folgenden Gruppen tragen können: Alkoxy, Alkylthio, Cyano, Alkylcarbonyl, Alkoxycarbonyl, bis-Dialkylamino, cyclo-Alkyl;
cyclo-Alkyl, das ein bis drei Alkylsubstituenten tragen kann;
Phenyl oder substituiertes Phenyl;
gemeinsam eine zu einem Ring geschlossene Alkylenkette oder gemeinsam eine zu einem Ring geschlossene Alkylenkette mit einem Heteroatom, das Sauerstoff, Schwefel oder Stickstoff sein kann, die jeweils ein bis drei Alkylsubstituenten tragen können;
oder eine Gruppe 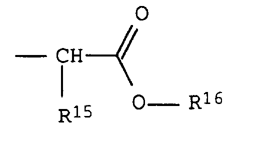
R¹⁰ Alkyl oder Phenyl, die ein bis vier der folgenden Substituenten tragen können: Halogen, Nitro, Cyano, Alkyl;
R¹¹,R¹² Alkyl, welches eine Alkoxy- oder Alkylthiogruppe tragen kann, gemeinsam eine Alkylenkette;
R¹³,R¹⁴ Alkyl, welches einen Phenylrest, eine Alkoxy- und/oder eine Alkylthiogruppe tragen kann, Cycloalkyl, Phenyl, gemeinsam eine Alkylenkette, welche ein bis fünf Alkylgruppen tragen kann und die durch eine Alkylenkette überbrückt sein kann;
R¹⁵ Wasserstoff oder Alkyl, das durch Hydroxy-, Amino-, Hydrogensulfid-, Alkylthio-, Carboxy-, Carbamoyl-, Guanidinyl-, Phenyl-, Hydroxyphenyl-, Imidazolyl- oder Indolyl-Radikale substituiert sein kann oder zusammen mit R⁹ über eine Alkylenkette zu einem Ring verbunden ist;
R¹⁶ Alkyl, Alkenyl oder Alkinyl;
m 0 oder 1;
n 0, 1, 2 oder 3;
wobei substituiertes Phenyl, substituiertes Phenoxy, substituiertes Phenylthio, substituiertes Phenylsulfonyl bedeutet, daß der Phenylring ein bis fünf Halogenatome und/oder einen bis drei der folgenden Reste tragen kann: Nitro, Cyano, Alkyl, Halogenalkyl, Alkoxy, Halogenalkoxy, Alkylthio, Alkylamino, Dialkylamino, Alkylcarbonyl, Alkoxycarbonyl, Phenyl, mit ein bis drei Halogenatomen oder ein bis drei Methylgruppen substituiertes Phenyl, Phenoxy, mit ein bis drei Halogenatomen oder ein bis drei Methylgruppen substituiertes Phenoxy oder an zwei benachbarten Atomen eine Einheit -O-(CH₂)ₒ-O-, wobei o die Werte 1 oder 2 annehmen kann.

**2.** Verfahren zur Herstellung der Verbindungen der Formel I gemäß Anspruch 1, dadurch gekennzeichnet, daß man ein entsprechendes Derivat der Formel
A-XH
worin A und X die oben angegebene Bedeutung haben, in an sich bekannter Weise mit einem Heterocyclus der Formel II 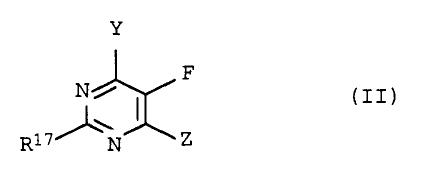 in der R¹⁷ für eine nucleofuge Abgangsgruppe steht, in Gegenwart einer anorganischen oder organischen Base umsetzt.

**3.** Verfahren zur Herstellung der Verbindungen der Formel I gemäß Anspruch 1, dadurch gekennzeichnet, daß man Verbindungen der Formel I in denen W Sauerstoff und R¹ eine Hydroxygruppe darstellt, zunächst in an sich bekannter Weise in das Halogenid oder eine andere aktivierte Form der Carbonsäure überführt und diese dann gegebenenfalls in Gegenwart einer anorganischen oder organischen Base oder in Gegenwart eines wasserabspaltenden Mittels mit einem Nucleophil umsetzt.

**4.** Zwischenprodukte der Formel II 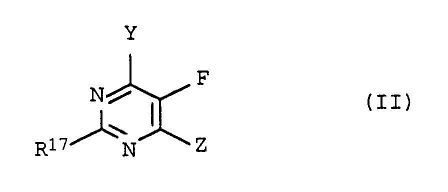 in denen X, Z und R¹⁷ die oben angegebene Bedeutung haben.

**5.** Herbizides Mittel, enthaltend eine Verbindung der Formel I gemäß Anspruch 1.

**6.** Herbizides Mittel gemäß Anspruch 5, enthaltend weitere wirksame Bestandteile.

**7.** Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Pyrimidins der Formel I gemäß Anspruch 1 behandelt.

**8.** Verwendung von Pyrimidinen der Formel I gemäß Anspruch 1 als Herbizide.

**9.** Mittel zur Beeinflussung des Pflanzenwachstums, enthaltend ein Pyrimidin der Formel I gemäß Anspruch 1.

**10.** Verfahren zur Regulierung des Pflanzenwachstums, dadurch gekennzeichnet, daß man eine regulativ wirksame Menge eines Pyrimidins, der allgemeinen Formel I gemäß Anspruch 1 auf die Samen, die Pflanzen und/oder deren Lebensraum einwirken läßt.

**12.** Fungizides und/oder nitrifikationshemmendes Mittel, enthaltend ein Pyrimidin der Formel I gemäß Anspruch 1.
